# EUROPEAN PATENT APPLICATION

(11) **EP 4 480 499 A1**
(43) Date of publication of application: **25.12.2024**
(21) Application number: 23755810.1
(22) Date of filing: 15.02.2023
(51) Int. Cl.: A61K 47/68, A61K 47/64, A61K 47/65, C07K 16/28, A61P 35/00

(54) **CONJUGATE OF ANTIBODY-ERIBULIN OR DERIVATIVE THEREOF, INTERMEDIATE THEREOF, PREPARATION METHOD THEREFOR, PHARMACEUTICAL COMPOSITION THEREOF AND USE THEREOF**

(30) Priority: 16.02.2022 CN 202210140796
(71) Applicant: Medilink Therapeutics (Suzhou) Co., Ltd., Suzhou, Jiangsu 215000 (CN)
(72) Inventor: XUE, Tongtong, Suzhou, Jiangsu 215000 (CN); XIAO, Liang, Suzhou, Jiangsu 215000 (CN); SONG, Shuai, Suzhou, Jiangsu 215000 (CN); SHI, Xinzhen, Suzhou, Jiangsu 215000 (CN); LI, Jundong, Suzhou, Jiangsu 215000 (CN); CAI, Jiaqiang, Suzhou, Jiangsu 215000 (CN)
(74) Representative: Greaves Brewster LLP
(86) International application number: PCT/CN2023/076238
(87) International publication number: WO 2023/155808

(57) **Abstract**

A conjugate of antibody-eribulin or a derivative thereof, an intermediate thereof, a preparation method therefor, a pharmaceutical composition thereof and use thereof. The conjugate of the antibody-eribulin or the derivative thereof provided herein as shown in general formula I is shown below. The conjugate of general formula I has high anti-tumor activity and high stability in circulation, and has excellent tumor tissue targeting property and therapeutic indexes.

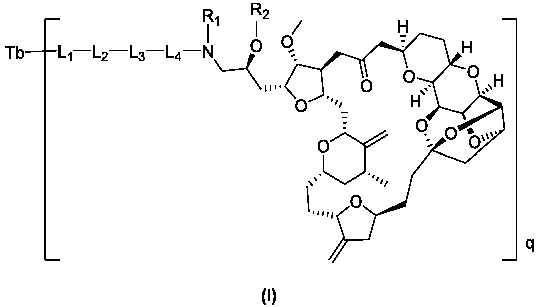

## Description

The present application claims the right of the priority of Chinese patent application 2022101407965 filed on February 16, 2022. The contents of the above Chinese patent application are incorporated herein by reference in its entirety.

### TECHNICAL FIELD

The present disclosure relates to a conjugate of antibody-eribulin or a derivative thereof, an intermediate thereof, a preparation method therefor, a pharmaceutical composition thereof, and a use thereof.

### BACKGROUND

Halaven (or eribulin) is a microtubule dynamics inhibitor belonging to the halichondrin class with a distinct binding profile. In addition to the mechanism of action of inhibiting the growth of microtubule dynamics, non-clinical studies have shown that Halaven has unique actions on the tumor microenvironment, such as increasing vascular perfusion and permeability in tumor cores, promoting epithelial status, and decreasing the ability of breast cancer cells to migrate. Halaven has currently been approved for the treatment of breast cancer in more than 70 countries and regions in Europe, America, and Asia. A phase I clinical study reported by Synold *et al.* enrolled a total of 40 patients, among which 38 were evaluable. Eribulin mesylate was administered by intravenous infusion on day 1, day 8, and day 15 with 28 days as one cycle of chemotherapy, and a maximum tolerated dose (MTD) of 1.4 mg/m² was determined. The most common grade 3-4 adverse events in the treatment with eribulin mesylate were neutropenia (64%). Antibody-drug conjugate (ADC) is a conjugate of antibody and small molecule drug, which combines the tumor targeting effect of antibodies with the activity of bioactive molecules. ADC has become a biological missile with promising advantage of efficacy and safety. The antibody guides the ADC to bind to target cells, followed by cellular internalization, and the small molecule drug is released inside the cells by enzymatic hydrolysis under the action of specific enzymes to treat diseases. Therefore, it can be expected that an ADC consisting of eribulin and a tumor-targeting antibody can maintain the therapeutic effect of eribulin while eliminating or reducing the toxic side effects caused by eribulin acting on non-diseased tissues, thus improving therapeutic efficacy.

Maleimide is widely used to form ADC molecules by coupling with thiols of antibodies through a Michael addition reaction due to its high selectivity, high reactivity, and good stability. 9 of the 15 marketed ADC drugs are coupled via maleimide. It has been reported in many literatures that the thiosuccinimide bond is unstable in the presence of thiol-containing substances (e.g., in plasma) and can be broken through a reverse Michael reaction or exchanged for endogenous thiols such as albumin (HAS) and glutathione (GSH), resulting in unsatisfactory pharmacodynamics, pharmacokinetics, and safety.

### CONTENT OF THE PRESENT INVENTION

The present disclosure aims to provide a conjugate of antibody-eribulin or a derivative thereof, an intermediate thereof, a preparation method therefor, a pharmaceutical composition thereof, and a use thereof. The conjugate of antibody-eribulin or the derivative thereof of the present disclosure has high anti-tumor activity, can kill tumor cells more effectively, and has better *in vivo* anti-tumor effect than eribulin alone. The high stability of ADC in the circulation can reduce the shedding of non-targeted bioactive molecules in non-target cells and increase the effective intracellular release of bioactive molecules, allowing the bioactive molecules to have a higher concentration ratio between tumor and blood with a higher therapeutic index. By adding basic nitrogen atoms to the linker, the exposure of the entire conjugate of antibody-eribulin or the derivative thereof in a relatively acidic tumor environment is increased, resulting in better tumor tissue targeting and an increase in the concentration ratio of bioactive molecules between tumor and blood with a higher therapeutic index.

Thus, in a first aspect of the present disclosure, the present disclosure provides a conjugate of antibody-eribulin or a derivative thereof of formula I, 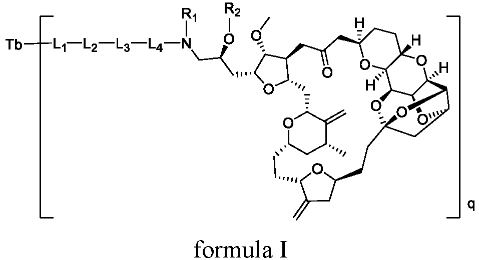
or a stereoisomer of the conjugate, a prodrug thereof, a pharmaceutically acceptable salt thereof, or a pharmaceutically acceptable solvate thereof,
wherein
Tb is an antibody or an antigen-binding fragment thereof that binds to a target, or a targeting moiety;
q is a drug-to-Tb coupling ratio;
R¹ and R² are each independently selected from hydrogen (H), deuterium (D), and C₁₋₆ alkyl; or, R¹ and R² together with the nitrogen atom and the oxygen atom to which they are attached form a 5- to 6-membered heterocyclic ring; the 5- to 6-membered heterocyclic ring is optionally substituted by one or more substituents of C₁₋₆ alkyl, C₁₋₆ alkoxy, C₃₋₆ cycloalkyl, and C₃₋₆ heterocyclyl;
L₁ is selected from 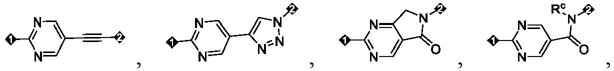 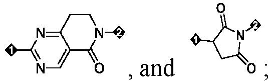 position 1 is attached to Tb via an S atom and position 2 is attached to L₂;
R^{c} is selected from hydrogen (H) and C₁₋₆ alkyl;
L₂ is selected from 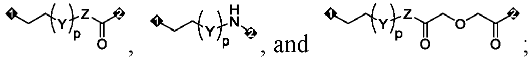 position 1 is attached to L₁ and position 2 is attached to L₃;
p is selected from any integer between 0 and 10;
Y is selected from -CH₂- and -OCH₂CH₂-;
Z is selected from -CR³R⁴- and -NR⁵-;
R³ and R⁴ are each independently selected from hydrogen (H), C₁₋₄ alkyl, C₂₋₄ alkenyl, C₂₋₄ alkynyl, C₃₋₆ cycloalkyl, and C₃₋₆ heterocyclyl; or, R³ and R⁴ together with the carbon atom to which they are attached form a 3- to 6-membered carbocyclic ring or a 3- to 6-membered heterocyclic ring;
R⁵ is selected from hydrogen (H), C₁₋₄ alkyl, C₂₋₄ alkenyl, C₂₋₄ alkynyl, C₃₋₆ cycloalkyl, and C₃₋₆ heterocyclyl;
L₃ is selected from a chemical bond, 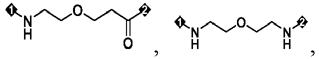 an amino acid residue, or a short peptide consisting of 2 to 10 amino acid residues; the amino acid residue is selected from a natural amino acid residue, an unnatural amino acid residue, or an amino acid residue represented by AA¹ or a stereoisomer thereof;
the amino acid residue represented by AA¹ has a structure as follows, 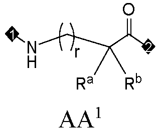
wherein
R^{a} and R^{b} are each independently selected from hydrogen (H) and 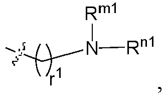 and R^{a} and R^{b} are not both hydrogen (H);
or, R^{a} and R^{b} together with the carbon atom to which they are both attached form a 4-to 10-membered heterocyclic ring; the 4- to 10-membered heterocyclic ring is optionally substituted by one or more R⁰;
r and r¹ are each independently selected from any integer between 0 and 20;
R^{m1} and Rⁿ¹ are each independently selected from hydrogen (H), C₁₋₆ alkyl, and C₃₋₆ cycloalkyl;
or, R^{m1} and Rⁿ¹ together with the nitrogen atom to which they are both attached form a 4- to 10-membered heterocyclic ring; the 4- to 10-membered heterocyclic ring is optionally substituted by one or more R^{0'};
R⁰ and R^{0'} are each independently selected from C₁₋₆ alkyl, C₃₋₆ cycloalkyl, -NR^{m2}Rⁿ², and optionally C₁₋₆ alkyl-substituted 4- to 10-membered heterocyclyl;
R^{m2} and Rⁿ² are each independently selected from hydrogen (H) and C₁₋₆ alkyl;
L₄ is absent or present; when L₄ is present, L₄ is selected from 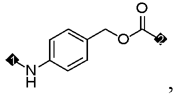 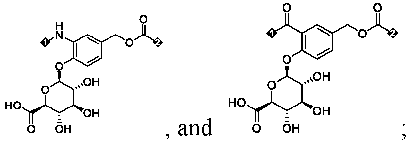 position 1 is attached to L₃ and position 2 is attached to the nitrogen atom of eribulin.

In some embodiments, provided is the conjugate of antibody-eribulin or the derivative thereof of formula I, or a stereoisomer of the conjugate, a prodrug thereof, a pharmaceutically acceptable salt thereof, or a pharmaceutically acceptable solvate thereof,
wherein
Tb is an antibody or an antigen-binding fragment thereof that binds to a target, or a targeting moiety;
q is a drug-to-Tb coupling ratio;
R¹ and R² are each independently selected from hydrogen (H), deuterium (D), and C₁₋₆ alkyl; or, R¹ and R² together with the nitrogen atom and the oxygen atom to which they are attached form a 5- to 6-membered heterocyclic ring; the 5- to 6-membered heterocyclic ring is optionally substituted by one or more substituents of C₁₋₆ alkyl, C₁₋₆ alkoxy, C₃₋₆ cycloalkyl, and C₃₋₆ heterocyclyl;
L₁ is selected from 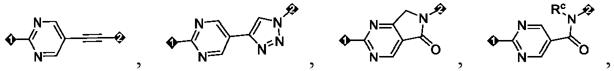 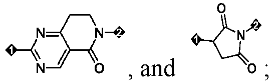 position 1 is attached to Tb via an S atom and position 2 is attached to L₂;
R^{c} is selected from hydrogen (H) and C₁₋₆ alkyl;
L₂ is selected from 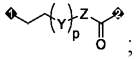 position 1 is attached to L₁ and position 2 is attached to L₃;
p is selected from any integer between 0 and 10;
Y is selected from CH₂ and OCH₂CH₂;
Z is selected from CR³R⁴ and NR⁵;
R³ and R⁴ are each independently selected from H, C₁₋₄ alkyl, C₂₋₄ alkenyl, C₂₋₄ alkynyl, C₃₋₆ cycloalkyl, and C₃₋₆ heterocyclyl; or, R³ and R⁴ together with the carbon atom to which they are attached form a 3- to 6-membered carbocyclic ring or a 3- to 6-membered heterocyclic ring;
R⁵ is selected from H, C₁₋₄ alkyl, C₂₋₄ alkenyl, C₂₋₄ alkynyl, C₃₋₆ cycloalkyl, and C₃₋₆ heterocyclyl;
L₃ is selected from an amino acid residue or a short peptide consisting of 2 to 10 amino acid residues; the amino acid residue is selected from a natural amino acid residue, an unnatural amino acid residue, or an amino acid residue represented by AA¹ or a stereoisomer thereof;
the amino acid residue represented by AA¹ has a structure as follows, 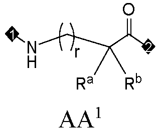
wherein
R^{a} and R^{b} are each independently selected from Hand 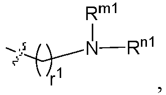 and R^{a} and R^{b} are not both H;
or, R^{a} and R^{b} together with the carbon atom to which they are both attached form a 4-to 10-membered heterocyclic ring; the 4- to 10-membered heterocyclic ring is optionally substituted by one or more R⁰;
r and r¹ are each independently selected from any integer between 0 and 20;
R^{m1} and Rⁿ¹ are each independently selected from H, C₁₋₆ alkyl, and C₃₋₆ cycloalkyl;
or, R^{m1} and Rⁿ¹ together with the nitrogen atom to which they are both attached form a 4- to 10-membered heterocyclic ring; the 4- to 10-membered heterocyclic ring is optionally substituted by one or more R^{0'};
R⁰ and R^{0'} are each independently selected from C₁₋₆ alkyl, C₃₋₆ cycloalkyl, -NR^{m2}Rⁿ², and optionally C₁₋₆ alkyl-substituted 4- to 10-membered heterocyclyl;
R^{m2} and Rⁿ² are each independently selected from H and C₁₋₆ alkyl;
L₄ is absent or present; when L₄ is present, L₄ is selected from 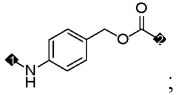 position 1 is attached to L₃ and position 2 is attached to the nitrogen atom of eribulin.
In some embodiments, when L₁ is 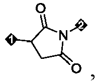 position 1 is attached to Tb via an S atom, and position 2 is attached to L₂:
L₃ is not a chemical bond, Val-Cit, Val-Lys, (Gly)₂-Phe-Gly, or Ala-Ala-Asn; or,
L₄ is present and is not 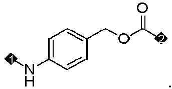
In some embodiments, when L₁ is 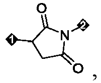 position 1 is attached to Tb via an S atom, position 2 is attached to L₂, and L₃ is Val-Cit, Val-Lys, (Gly)₂-Phe-Gly, or Ala-Ala-Asn: L₄ is selected from absent, 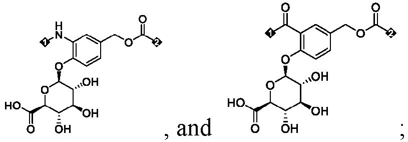 or,
when L₃ is a chemical bond: L₄ is present.

In some embodiments, q is selected from any numerical value between 0.1 and 16.0; in preferred embodiments, q is selected from any integer between 0.1 and 10.0.

In some embodiments, q is selected from any numerical value between 2 and 8.

In some embodiments, q is selected from 1, 2, 3, 4, 5, 6, 7, and 8.

In some embodiments, R¹ and R² are hydrogen (H); or, R¹ and R² together with the nitrogen atom and the oxygen atom to which they are attached form 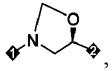 and position 1 is attached to L₄ or L₃.

In some embodiments, L₁ is selected from 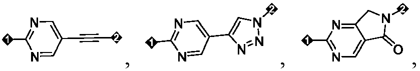 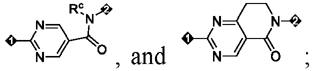 position 1 is attached to Tb via an S atom and position 2 is attached to L₂.

In some embodiments, L₁ is selected from 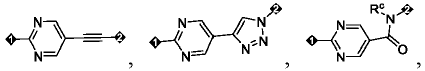 and 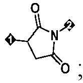 position 1 is attached to Tb via an S atom and position 2 is attached to L₂.

In some embodiments, L₁ is selected from 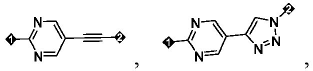 and 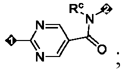 position 1 is attached to Tb via an S atom and position 2 is attached to L₂.

In some embodiments, L₁ is 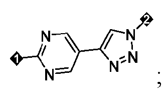 position 1 is attached to Tb via an S atom and position 2 is attached to L₂.

In some embodiments, R^{c} is hydrogen (H).

In some embodiments, p is selected from 0, 1, 2, 3, 4, 5, 6, 7, and 8.

In some embodiments, Z is -CH₂-, -C(CH₃)₂-, or -NH-.

In some embodiments, Z is -C(CH₃)₂-.

In some embodiments, R³ and R⁴ are each independently selected from hydrogen (H) and methyl.

In some embodiments, R⁵ is hydrogen (H) or methyl.

In some embodiments, L₂ is selected from 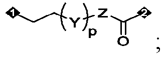 position 1 is attached to L₁ and position 2 is attached to L₃.

In some embodiments, L₂ is selected from 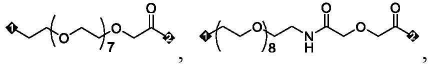 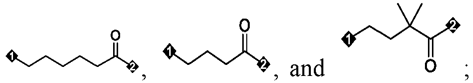 position 1 is attached to L₁ and position 2 is attached to L₃.

In some embodiments, L₂ is 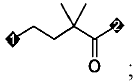 position 1 is attached to L₁ and position 2 is attached to L₃.

In some embodiments, L₃ is selected from amino acid residues Val, D-Val, Cit, Phe, Lys, Lys(Ac), Leu, Gly, Ala, Asn, Asp, Arg, and AA¹, or a short peptide consisting of 2 to 10 amino acid residues selected from Val, Cit, Phe, Lys, D-Val, Leu, Gly, Ala, Asn, Asp, and AA¹.

In some embodiments, the L₃ is a dipeptide, tripeptide, tetrapeptide, pentapeptide, hexapeptide, heptapeptide, octapeptide, nonapeptide, or decapeptide comprising at least one (e.g., 1, 2, or 3) amino acid residue represented by AA¹ or a stereoisomer thereof.

In some embodiments, the L₃ is a dipeptide, tripeptide, or tetrapeptide comprising at least one amino acid residue represented by AA¹ or a stereoisomer thereof.

In some embodiments, L₃ is selected from Val, Cit, Phe, Lys, D-Val, Leu, Gly, Ala, Asn, AA¹, Val-Cit, Cit-Val, Cit-Ala, Val-Ala, Lys-Val, Val-Lys(Ac), Phe-Lys, Phe-Lys(Ac), Ala-Ala, Val-AA¹, Ala-AA¹, Gly-AA¹, AA¹-Gly, Ala-Ala-Ala, Ala-Ala-Asn, Ala-Ala-Asp, Val-AA¹-Gly, Ala-AA¹-Gly, Gly-AA¹-Gly, Lys-(Ala)₂-Asn, Lys-(Ala)₂-Asp, Gly-Phe-Gly, (Gly)₂-Phe-Gly, D-Val-Leu-Lys, Gly-Gly-Arg, Ala-Ala-Asn, Gly-Gly-Phe, Val-Lys-Gly, Val-Lys-(Gly)₂, Val-Lys, Lys-Ala-Asn, and (Gly)₂-Phe-AA¹.

In some embodiments, L₃ is selected from Val-AA¹-Gly, Val-AA¹, Val-Cit, and (Gly)₂-Phe-AA¹.

In some embodiments, L₃ is selected from Val-AA¹ and Val-Cit.

In some embodiments, L₃ is selected from a chemical bond, 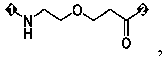 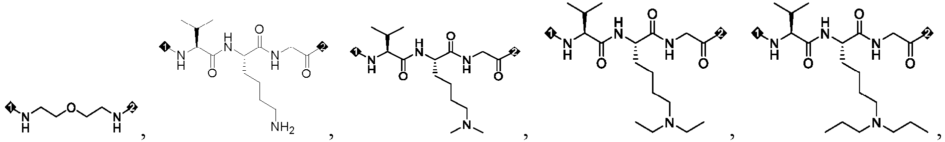 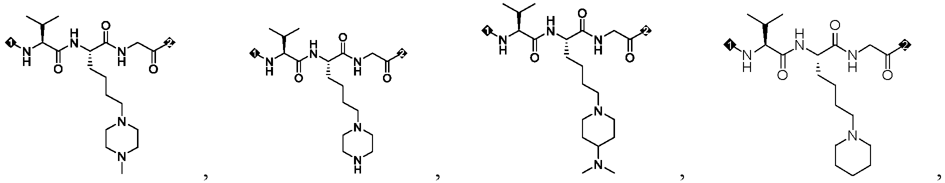 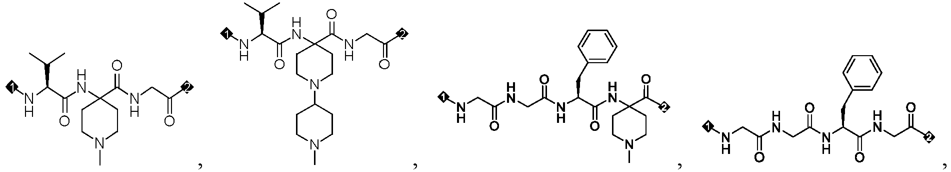 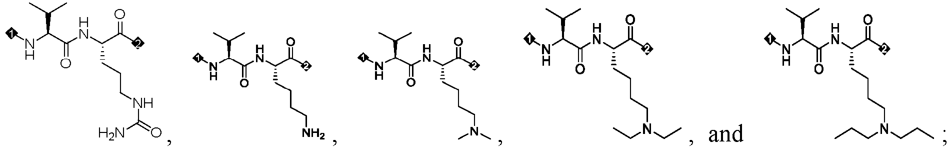 position 1 is attached to L₂ and position 2 is attached to L₄ or the nitrogen atom of eribulin.

In some embodiments, L₃ is selected from 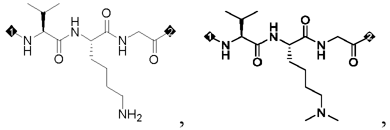 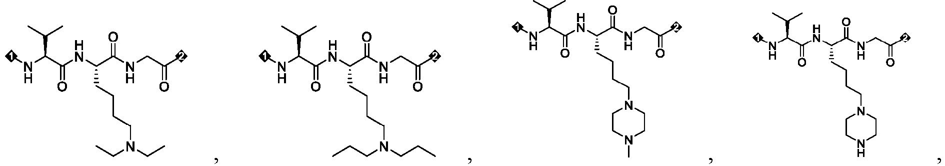 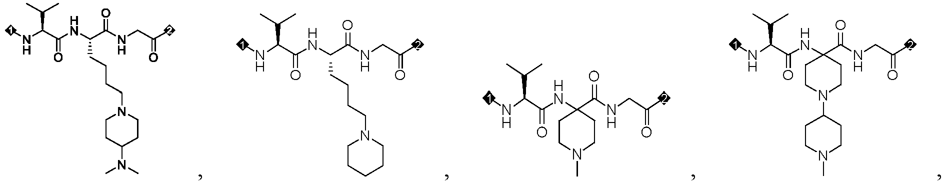 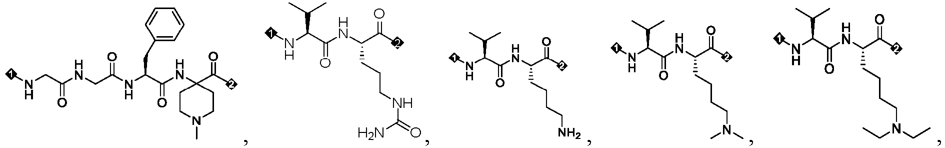 and 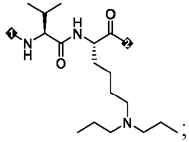 position 1 is attached to L₂ and position 2 is attached to L₄ or the nitrogen atom of eribulin.

In some embodiments, L₃ is selected from 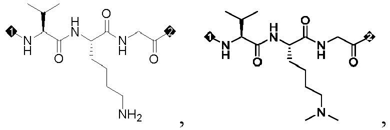 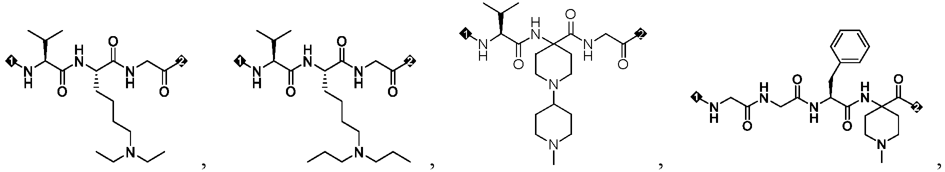 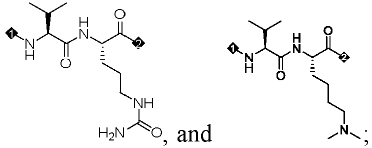 position 1 is attached to L₂ and position 2 is attached to L₄ or the nitrogen atom of eribulin.

In some embodiments, L₃ is selected from 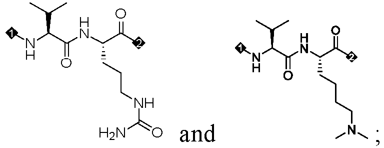 position 1 is attached to L₂ and position 2 is attached to L₄ or the nitrogen atom of eribulin.

In some embodiments, one of R^{a} and R^{b} is hydrogen (H), and the other is

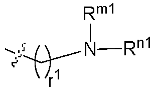

In some embodiments, R^{a} and R^{b} together with the carbon atom to which they are both attached form a 5- to 6-membered heterocyclic ring substituted by R⁰.

In some embodiments, R^{a} and R^{b} together with the carbon atom to which they are both attached form a piperidine ring or a piperazine ring, and the piperidine ring and the piperazine ring are substituted by R⁰.

In some embodiments, R^{a} and R^{b} together with the carbon atom to which they are both attached form a piperidine ring substituted by R⁰.

In some embodiments, R^{a} and R^{b} together with the carbon atom to which they are both attached form 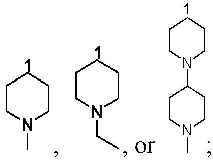 the carbon atom marked with number 1 is the carbon atom to which R^{a} and R^{b} are both attached.

In some embodiments, r and r¹ are each independently selected from 0, 1, 2, 3, 4, and 5.

In some embodiments, r and r¹ are each independently selected from 0 and 4.

In some embodiments, r is 0 and r¹ is 4.

In some embodiments, R^{m1} and Rⁿ¹ are each independently selected from hydrogen (H) and C₁₋₆ alkyl; preferably C₁₋₆ alkyl.

In some embodiments, R^{m1} and Rⁿ¹ are each independently selected from hydrogen (H), methyl, ethyl, *n*-propyl, and *n*-butyl; preferably methyl, ethyl, *n*-propyl, and *n*-butyl.

In some embodiments, R^{m1} and Rⁿ¹ together with the nitrogen atom to which they are both attached form a 5- to 6-membered heterocyclic ring optionally substituted by R^{0'}.

In some embodiments, R^{m1} and Rⁿ¹ together with the nitrogen atom to which they are both attached form a piperidine ring or a piperazine ring, and the piperidine ring and the piperazine ring are optionally substituted by R^{0'}.

In some embodiments, R^{m1} and Rⁿ¹ together with the nitrogen atom to which they are both attached form 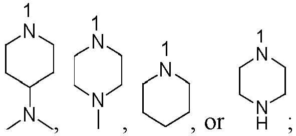 the nitrogen atom marked with number 1 is the nitrogen atom to which R^{m1} and Rⁿ¹ are both attached.

In some embodiments, R⁰ and R^{0'} are each independently selected from C₁₋₆ alkyl, - NR^{m2}Rⁿ², and optionally C₁₋₆ alkyl-substituted 5- to 6-membered heterocyclyl.

In some embodiments, R⁰ is selected from C₁₋₆ alkyl and C₁₋₆ alkyl-substituted 5- to 6-membered heterocyclyl; the 5- to 6-membered heterocyclyl is selected from piperidinyl and piperazinyl.

In some embodiments, R⁰ is selected from methyl, ethyl, and methyl-substituted 5- to 6-membered heterocyclyl; the 5- to 6-membered heterocyclyl is piperidinyl.

In some embodiments, R⁰ is selected from methyl and methyl-substituted 5- to 6-membered heterocyclyl; the 5- to 6-membered heterocyclyl is piperidinyl.

In some embodiments, R⁰ is selected from methyl, ethyl, and

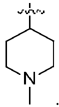

In some embodiments, R⁰ is selected from methyl and

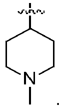

In some embodiments, R^{0'} is selected from C₁₋₆ alkyl and -NR^{m2}Rⁿ².

In some embodiments, R^{0'} is selected from methyl and -NR^{m2}Rⁿ².

In some embodiments, R^{m2} and Rⁿ² are methyl.

In some embodiments, the amino acid residue represented by AA¹ is selected from 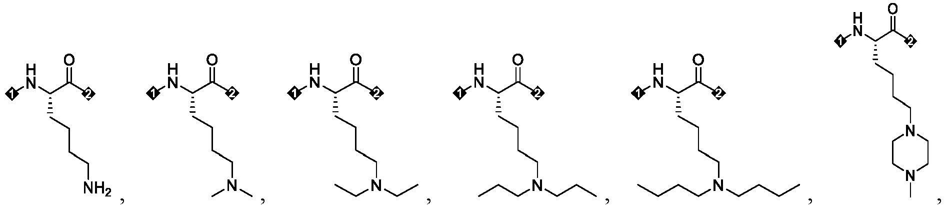 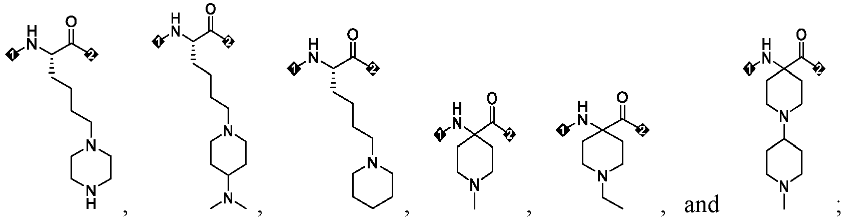 such as

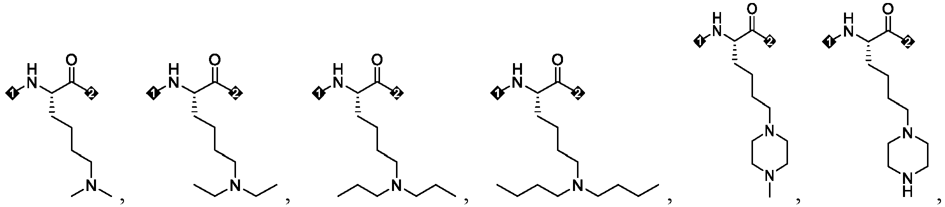

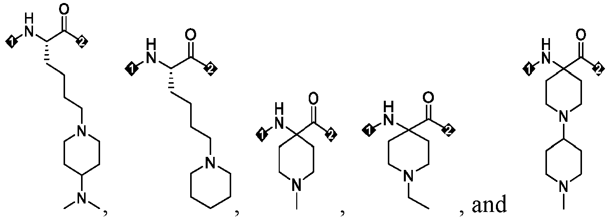

In some embodiments, the amino acid residue represented by AA¹ is selected from 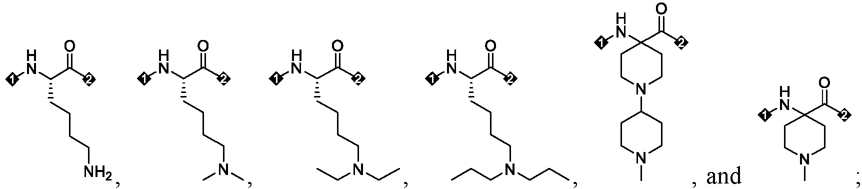 such as

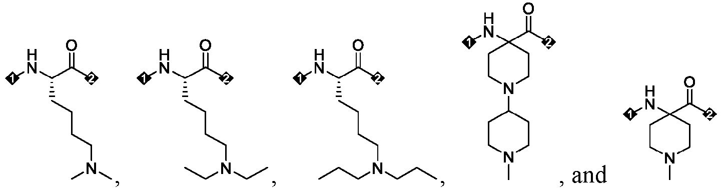

In some embodiments, the amino acid residue represented by AA¹ is

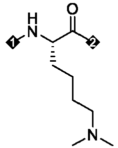

In some embodiments, L₄ is absent or present; when L₄ is present, L₄ is selected from 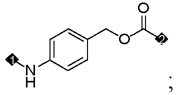 position 1 is attached to L₃ and position 2 is attached to the nitrogen atom of eribulin.

In some embodiments, L₄ is 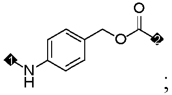 position 1 is attached to L₃ and position 2 is attached to the nitrogen atom of eribulin.

In some embodiments, the linker 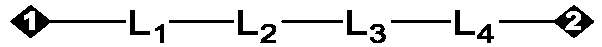 is selected from a linker of the following formulas:

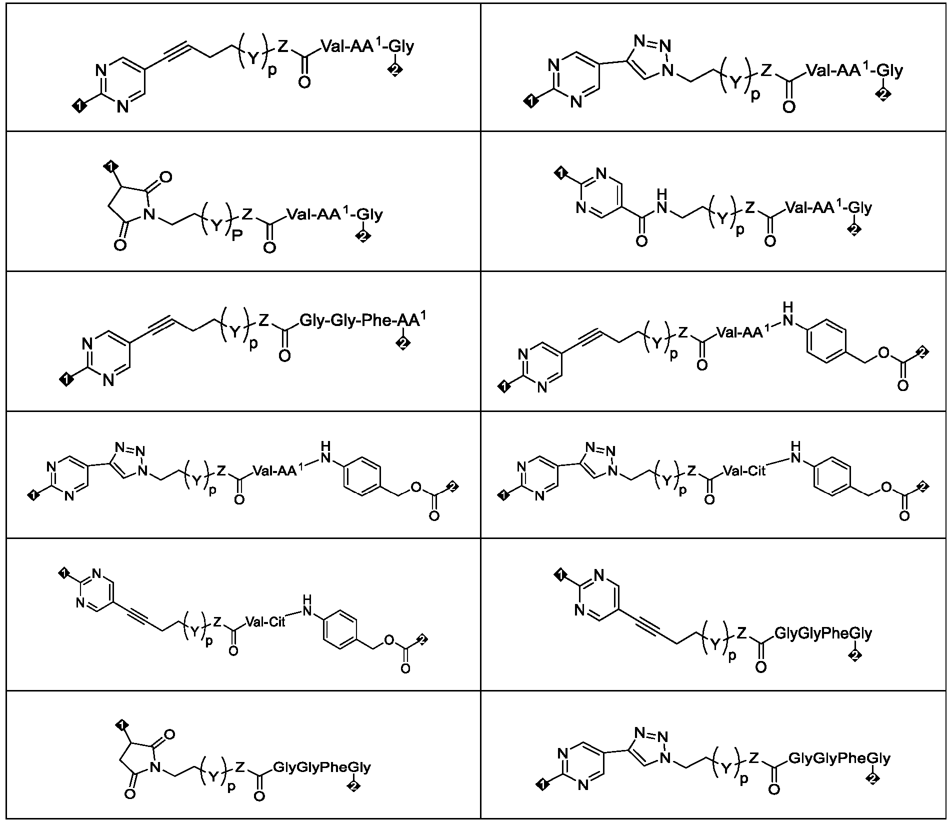

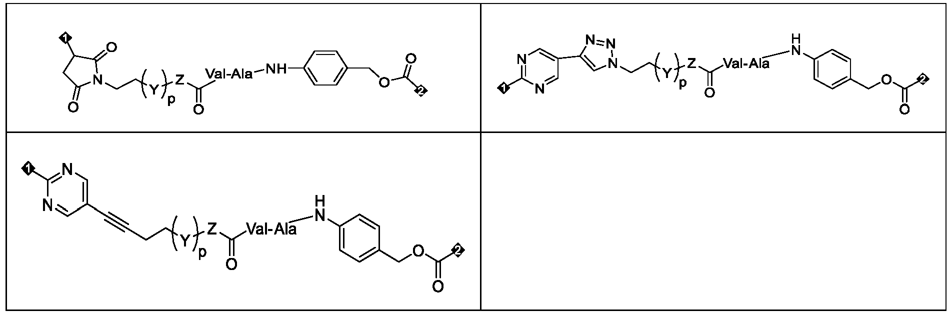

wherein Y, Z, p, and AA¹ are as defined in any one of the embodiments of the present disclosure; position 1 is attached to Tb via an S atom and position 2 is attached to the nitrogen atom of eribulin.

In some embodiments, the linker 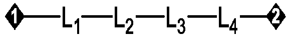 is selected from a linker of the following formulas:

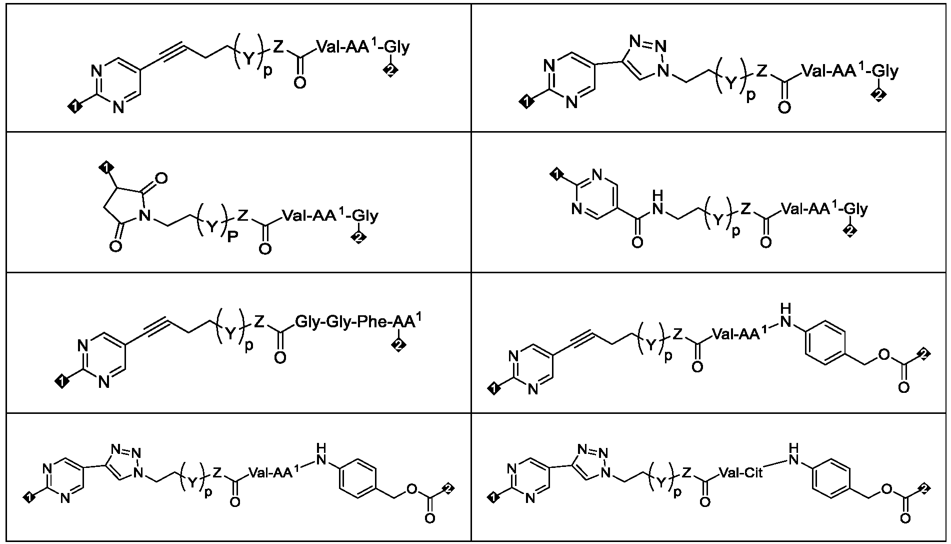

wherein Y, Z, p, and AA¹ are as defined in any one of the embodiments of the present disclosure; position 1 is attached to Tb via an S atom and position 2 is attached to the nitrogen atom of eribulin.

In some embodiments, the linker 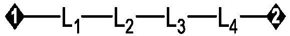 is selected from a linker of the following formulas:

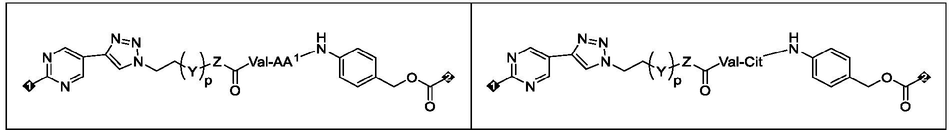

wherein Y, Z, p, and AA¹ are as defined in any one of the embodiments of the present disclosure; position 1 is attached to Tb via an S atom and position 2 is attached to the nitrogen atom of eribulin.

In some embodiments, the linker 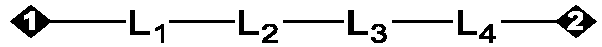 is selected from:

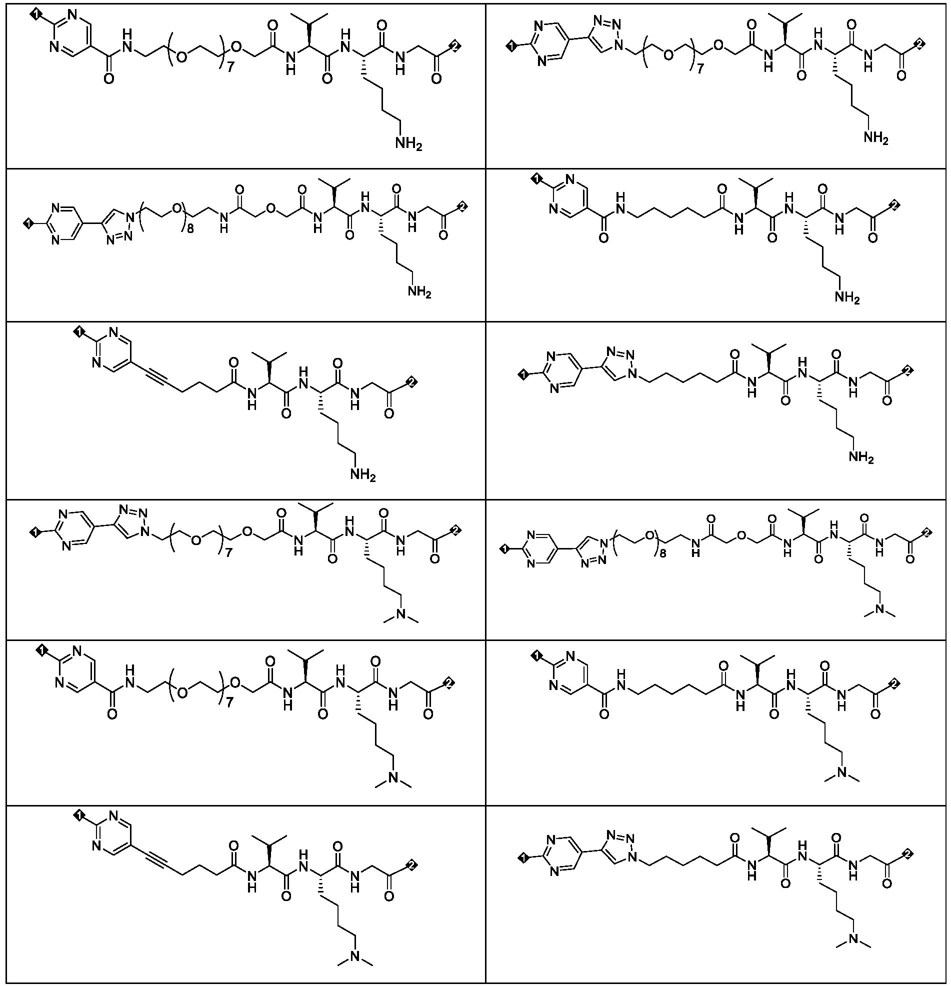

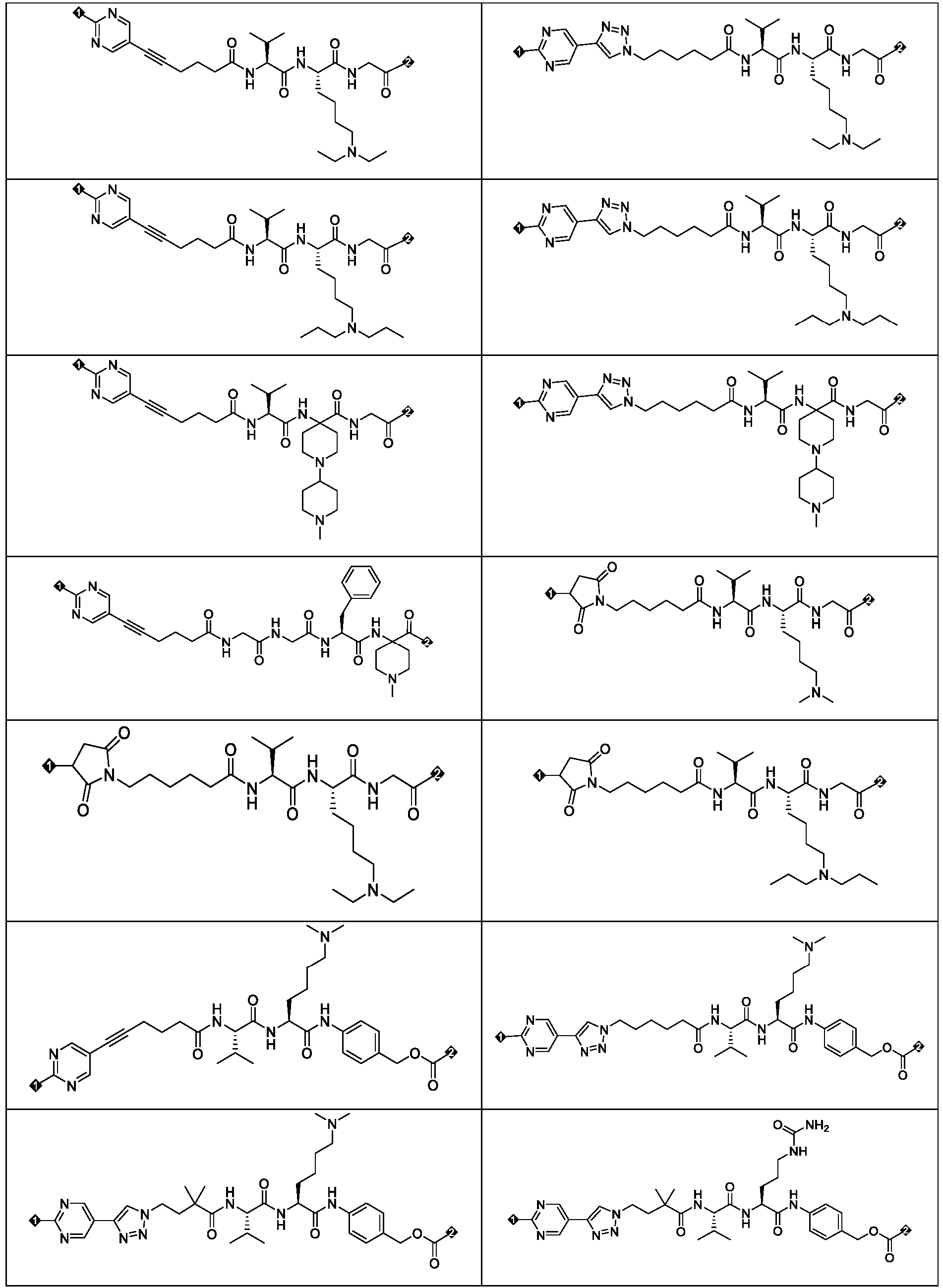

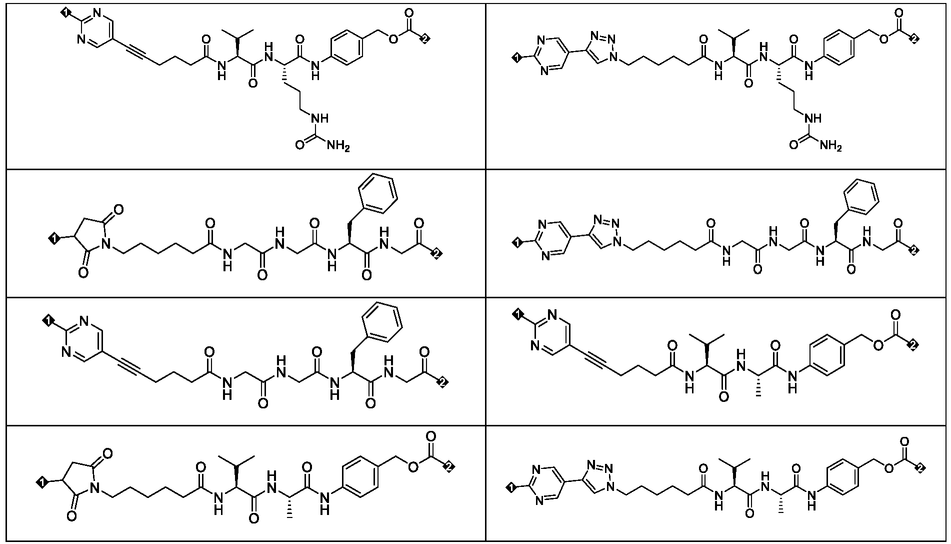

position 1 is attached to Tb via an S atom and position 2 is attached to the nitrogen atom of eribulin.

In some embodiments, the linker 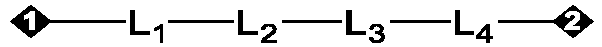 is selected from:

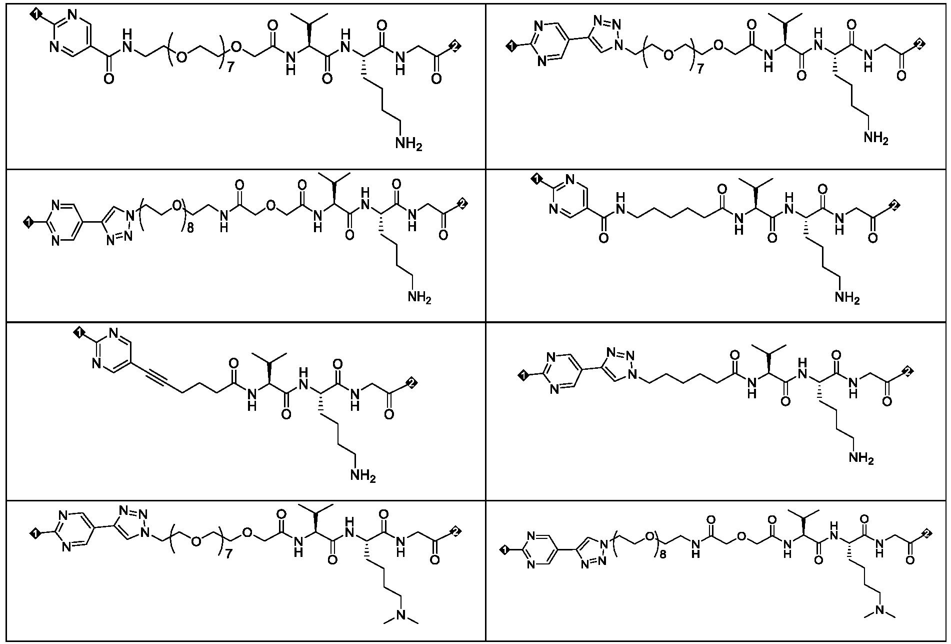

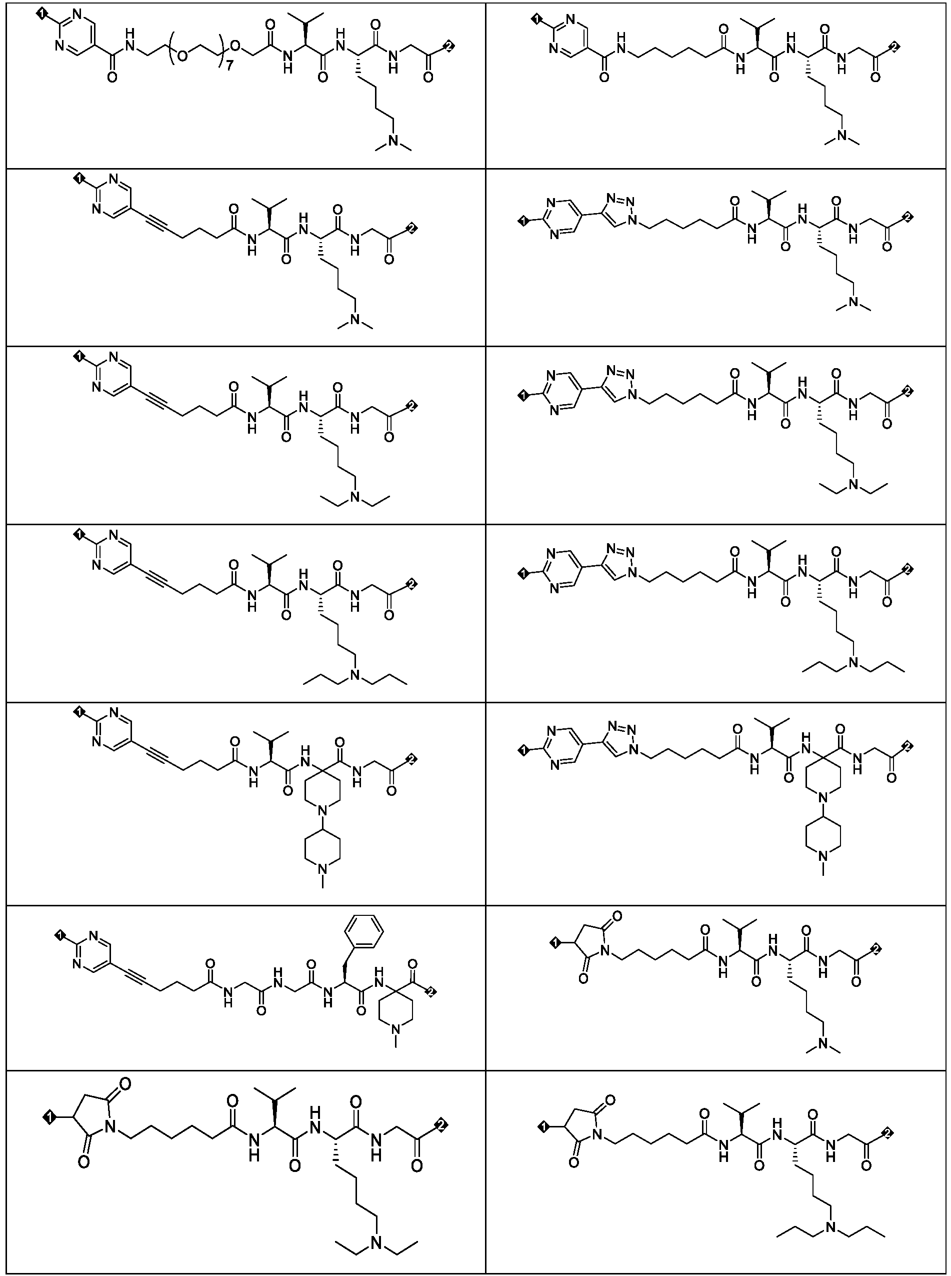

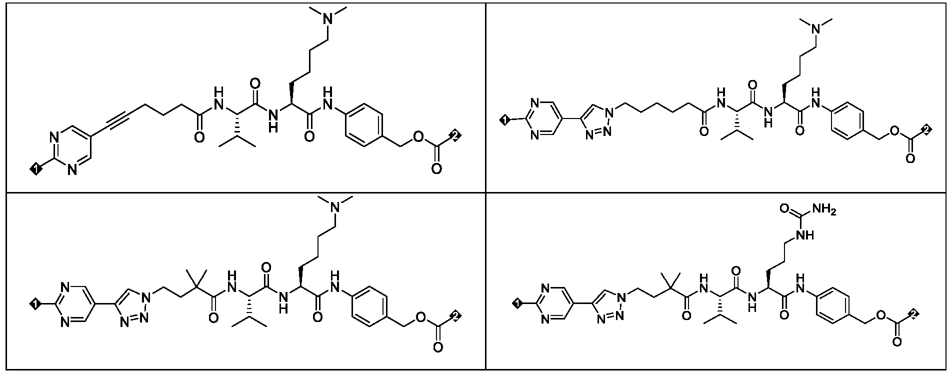

position 1 is attached to Tb via an S atom and position 2 is attached to the nitrogen atom of eribulin.

In some embodiments, the linker 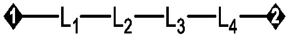 is selected from:

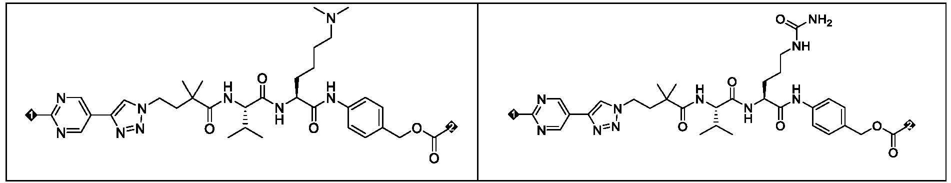

position 1 is attached to Tb via an S atom and position 2 is attached to the nitrogen atom of eribulin.

In some embodiments, the conjugate of antibody-eribulin or the derivative thereof is preferably a compound of the following formulas:

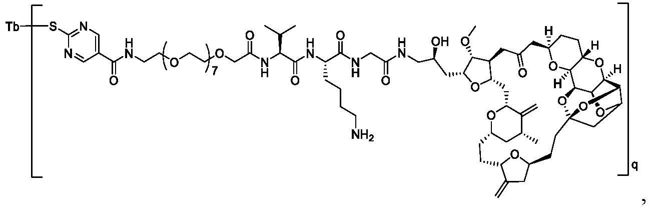

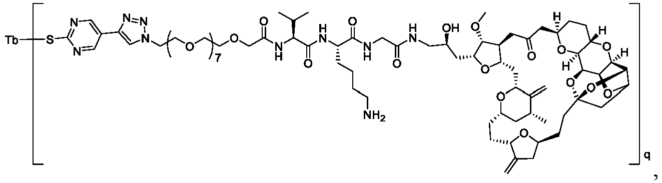

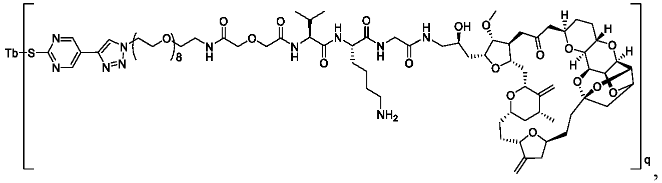

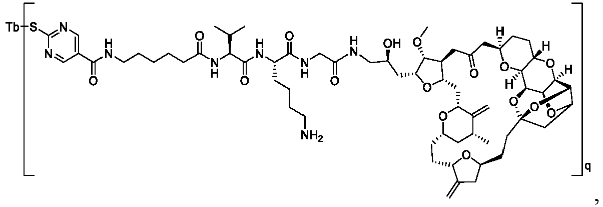

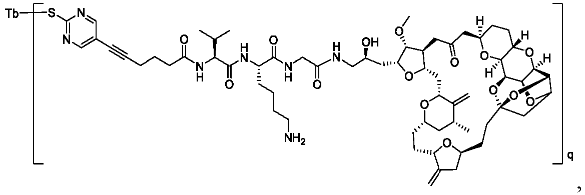

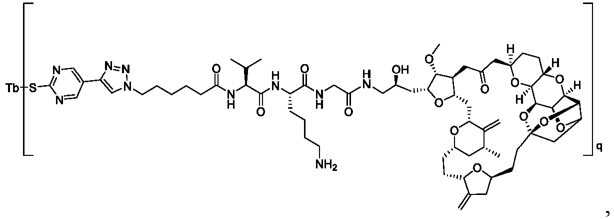

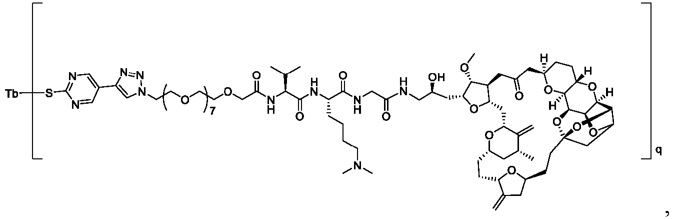

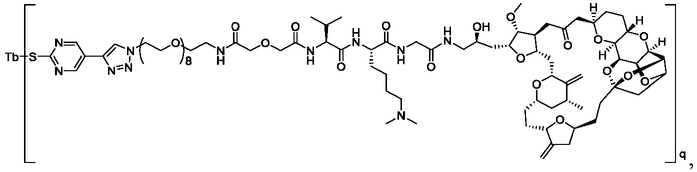

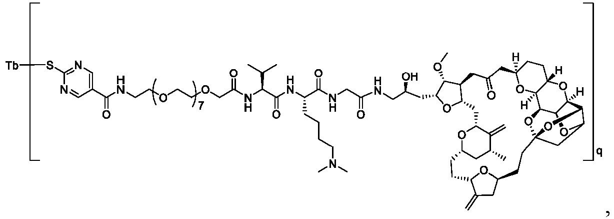

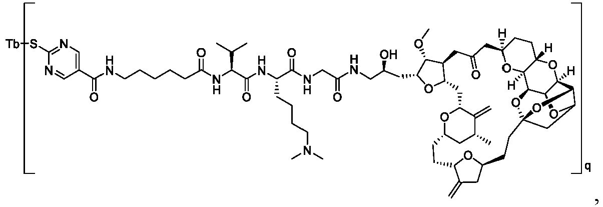

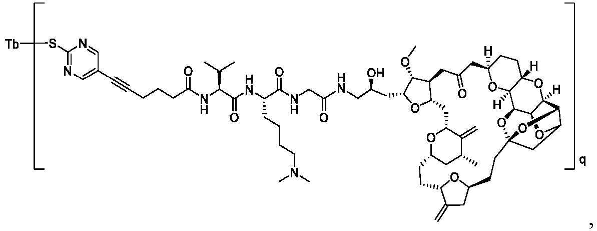

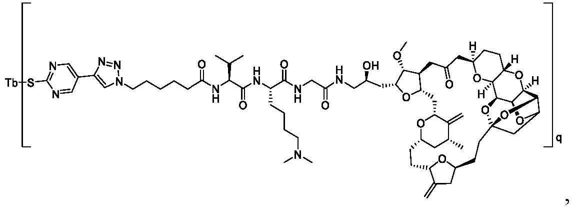

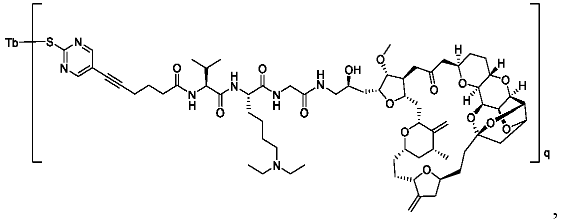

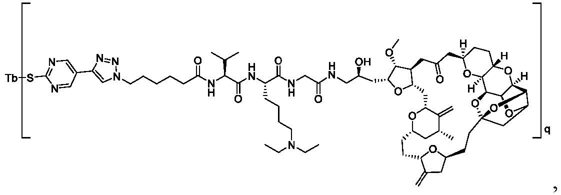

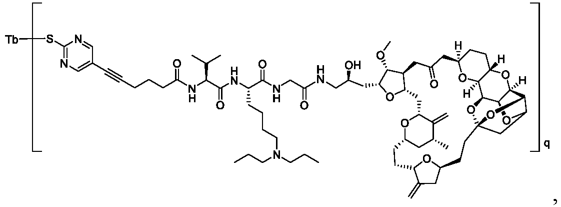

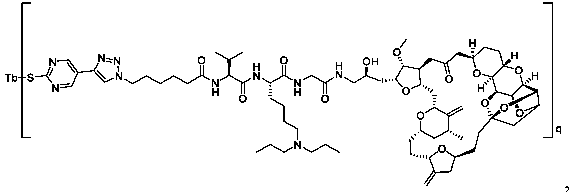

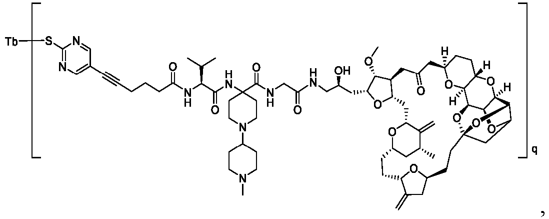

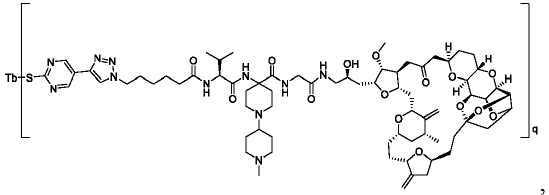

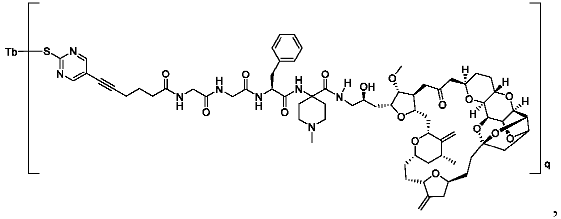

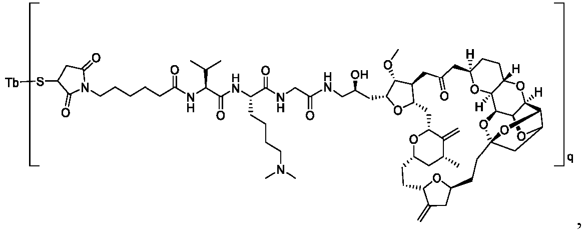

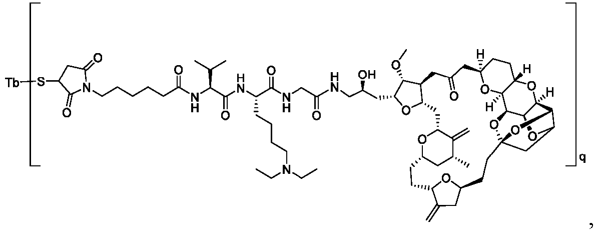

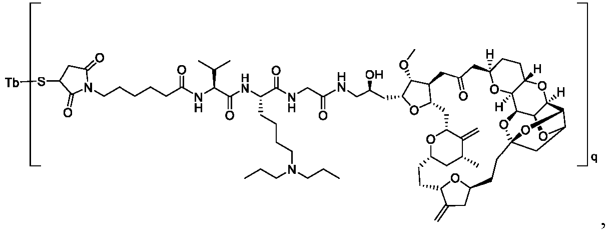

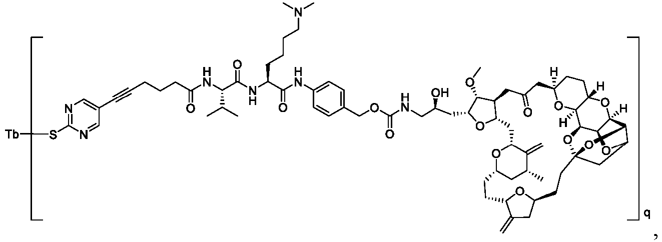

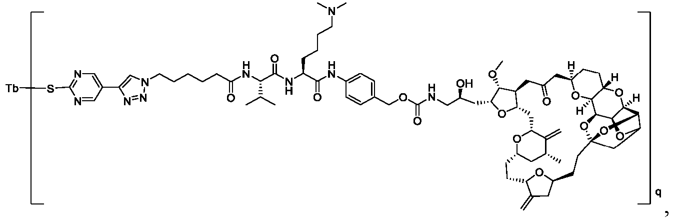

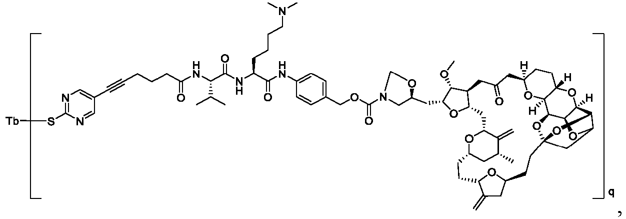

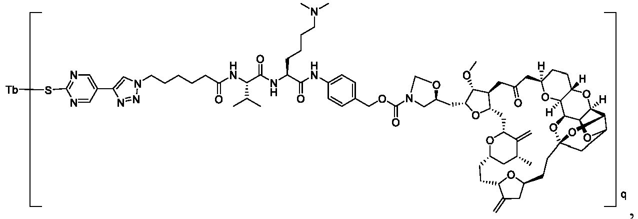

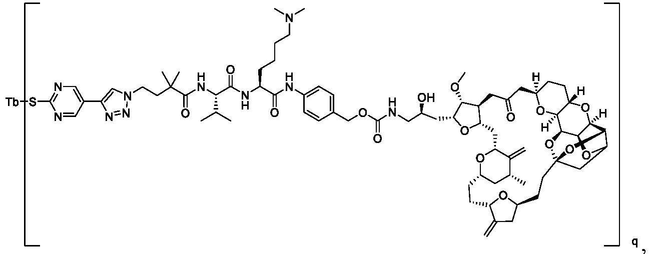

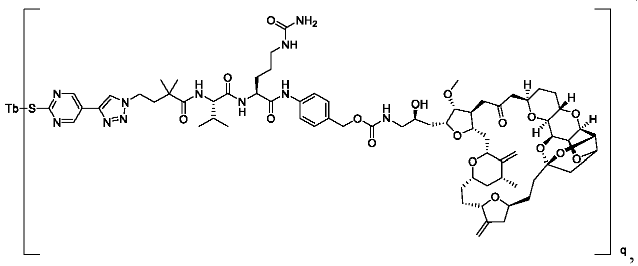

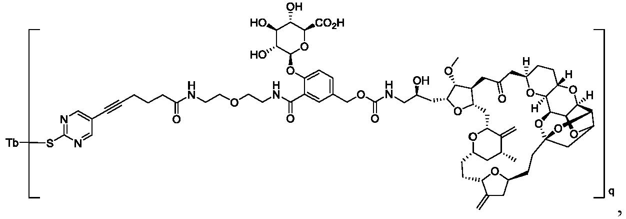

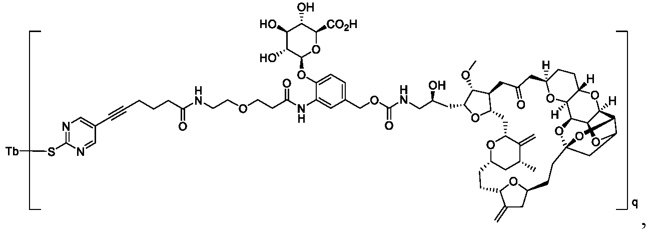

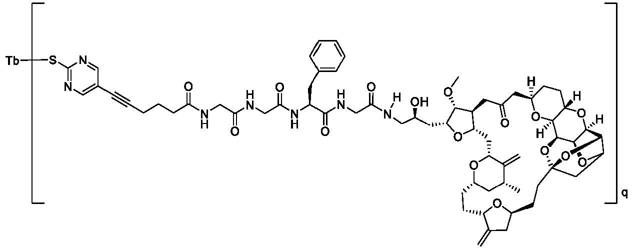

In addition, it should also be noted and it can be understood by those skilled in the art that L is attached to a sulfhydryl group contained in Tb (*e.g*., antibody) after a disulfide bond is broken (e.g., reduction of the disulfide bond by the reducing agent TCEP can break the disulfide bond to generate the sulfhydryl group -SH), that is, -S- between L and Tb is not an additional external sulfur atom. For example, -S- is not an additional external sulfur atom, but is formed by linking the sulfhydryl group contained in Tb to L after the disulfide bond is broken.

In some embodiments, Tb is an antibody or an antigen-binding fragment thereof.

In some embodiments, the antibody or the antigen-binding fragment thereof comprise Fab, Fab', F(ab')2, Fd, Fv, dAb, complementarity determining region fragment, single-chain antibody (*e.g*., scFv), non-human antibody, humanized antibody, chimeric antibody, fully human antibody, probody, bispecific antibody, or multi-specific antibody.

In some embodiments, Tb is a monoclonal antibody or an antigen-binding fragment thereof.

In some embodiments, Tb is an antibody or an antigen-binding fragment thereof which has endocytic activity.

In some embodiments, Tb is an antibody or an antigen-binding fragment thereof which has the activity of binding to a free antigen in tumor tissues and/or a tumor cell surface antigen.

In some preferred embodiments, Tb is selected from an antibody or an antigen-binding fragment thereof that binds to the following targets: epidermal growth factor, integrin α5β6, integrin α4β7, 0772P, 5T4, ACTA2, ADGRE1, AGS-16, AIF1, AKR1C1, AKR1C2, ANGPTL4, ApoE, ASLG659, BAFF-R, BMPR1B, BNIP3, Brevican, C1QA, C1QB, CA6, pCAD, P-cadherin, CADM1, CCL5, CCR5, CDl lb, CD1 lc, CD19, CD20, CD21, CD22, CD30, CD33, CD37, CD40, CD45 (PTPRC), CD49D (ITGA4), CD56, CD66e, CD70, CD72, CD74, CD79a, CD80, CD138, CD223, CDCP1, CDH11, Claudin18.2, COL6A3, COL7A1, CRIPTO, CSF1R, CTGF, CTSD, CTSS, CXCL10, CXCL11, CXCR5, DDIT4, DLL3, DLL4, DR5, E16, EFNA4, B7H3, EGFR, EGFRvIII, EGLN, EGLN3, EMR2, Endothelin receptor, ENPP3, EpCAM, EphB2R, ETBR, FGF2, FGFR2, FcRH1, FcRH2, GEDA, GPNMB, GZMB, Her2, Her3, HLA-DOB, HMOX1, IFNG, IFI6, IGF-1R, IGFBP3, IL10RA1, IL20Rα, IL-6, IRTA2, KISS1R, KRT33A, LOX, LRRC15, LUM, LY6E, LY64, LY86, LYPD3, MCPT8, MDP, Mesothelin, c-Met, MFI2, MMP10, MMP14, MMP16, MPF, MSG783, MS4A7, MUC 1, MUC16, NaPi2b, NaPi3b, NCA, Nectin 4, NOG, P2X5, PD-L1, PDK1, PDGFRA, PFKFB3, PGF, PGK1, PIK3AP1, PIK3CD, PLOD2, PSCA, PSCAhlg, PSMA, RNF43, ROR1, Sema5b, SERPINEl, SLC39A6, SLITRK6, SLTRK6, STAT1, STC2, STEAP1, STEAP2, TCF4, TENB2, TGF, TGFBR1, TGFB2, Tissue factor, TNFRSF21, TNFSF9, Trop-2, TrpM4, Tyro7, UPK1B, VEGFA, and WNT5A.

In some embodiments, Tb is an anti-B7H3 antibody or an antigen-binding fragment thereof, an anti-Trop-2 antibody or an antigen-binding fragment thereof, an anti-Her2 antibody or an antigen-binding fragment thereof, an anti-Her3 antibody or an antigen-binding fragment thereof, or an anti-EGFR antibody or an antigen-binding fragment thereof.

In some embodiments, Tb is an anti-Her2 antibody or an antigen-binding fragment thereof. In some embodiments, the anti-Her2 antibody is anbenitamab, coprelotamab, disitamab, gancotamab, margetuximab, pertuzumab, timigutuzumab, zanidatamab, trastuzumab, pertuzumab, or an antigen-binding fragment thereof.

In some embodiments, Tb is an anti-Trop-2 antibody or an antigen-binding fragment thereof. In some embodiments, the anti-Trop-2 antibody is datopotamab, sacituzumab, or an antigen-binding fragment thereof.

In some embodiments, Tb is an anti-EGFR antibody or an antigen-binding fragment thereof. In some embodiments, the anti-EGFR antibody is demupitamab, depatuxizumab, futuximab, imgatuzumab, laprituximab, losatuxizumab, matuzumab, modotuximab, necitumumab, nimotuzumab, panitumumab, pimurutamab, serclutamab, tomuzotuximab, zalutumumab, cetuximab, or an antigen-binding fragment thereof.

In some embodiments, Tb is an anti-B7H3 antibody or an antigen-binding fragment thereof. In some embodiments, the anti-B7H3 antibody is enoblituzumab, mirzotamab, omburtamab, antibody 1D1-01, antibody 2E3-02, or an antigen-binding fragment thereof.

In some embodiments, Tb is an anti-B7H3 antibody or an antigen-binding fragment thereof.

In some embodiments, the anti-B7H3 antibody or the antigen-binding fragment thereof comprises a complementarity determining region (CDR) as follows, wherein the CDR is defined by the Kabat numbering system: HCDR1 with the sequence of SEQ ID NO: 9, HCDR2 with the sequence of SEQ ID NO: 10, and HCDR3 with the sequence of SEQ ID NO: 11; and/or, LCDR1 with the sequence of SEQ ID NO: 12, LCDR2 with the sequence of SEQ ID NO: 13, and LCDR3 with the sequence of SEQ ID NO: 14.

In some embodiments, the anti-B7H3 antibody or the antigen-binding fragment thereof comprises a complementarity determining region (CDR) as follows, wherein the CDR is defined by the IMGT numbering system:

HCDR1 with the sequence of SEQ ID NO: 15, HCDR2 with the sequence of SEQ ID NO: 16, and HCDR3 with the sequence of SEQ ID NO: 17; and/or, LCDR1 with the sequence of SEQ ID NO: 18, LCDR2 with the sequence of GAS, and LCDR3 with the sequence of SEQ ID NO: 19.

In some embodiments, Tb is an anti-B7H3 antibody or an antigen-binding fragment thereof, wherein the anti-B7H3 antibody or the antigen-binding fragment thereof comprises: a VH set forth in SEQ ID NO: 1 or 5 and/or a VL set forth in SEQ ID NO: 2 or 6. The anti-B7H3 antibody or the antigen-binding fragment thereof further comprises: (a) a heavy chain constant region (CH) of a human immunoglobulin or a variant thereof; and/or (b) a light chain constant region (CL) of a human immunoglobulin or a variant thereof.

In some embodiments, Tb is an anti-B7H3 antibody or an antigen-binding fragment thereof, wherein the anti-B7H3 antibody or the antigen-binding fragment thereof comprises: a VH set forth in SEQ ID NO: 5 and a CH set forth in SEQ ID NO: 20 or a variant thereof, and/or, a VL set forth in SEQ ID NO: 6 and a CL set forth in SEQ ID NO: 21 or a variant thereof.

In some embodiments, Tb is an anti-B7H3 antibody or an antigen-binding fragment thereof, wherein the anti-B7H3 antibody or the antigen-binding fragment thereof comprises: a VH set forth in SEQ ID NO: 1 and a CH set forth in SEQ ID NO: 20 or a variant thereof, and/or, a VL set forth in SEQ ID NO: 2 and a CL set forth in SEQ ID NO: 21 or a variant thereof.

In some preferred embodiments, the B7H3 antibody or the antigen-binding fragment thereof comprises: a heavy chain set forth in SEQ ID NO: 7 and/or a light chain set forth in SEQ ID NO: 8.

In some preferred embodiments, the B7H3 antibody or the antigen-binding fragment thereof comprises: a heavy chain set forth in SEQ ID NO: 3 and/or a light chain set forth in SEQ ID NO: 4.

In some embodiments, the conjugate of antibody-eribulin or the derivative thereof is preferably a compound of the following formula, 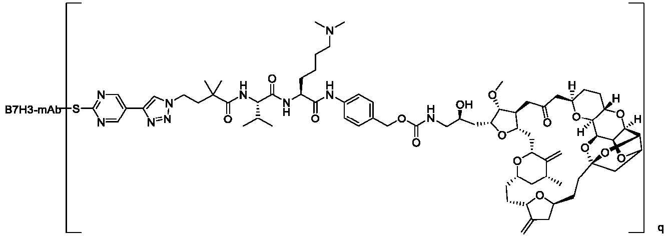 wherein B7H3-mAb is antibody 2E3-02, and q is selected from any integer between 0.1 and 10.0; preferably, q is selected from 1, 2, 3, 4, 5, 6, 7, and 8.

In some embodiments, the conjugate of antibody-eribulin or the derivative thereof is preferably a compound of the following formula, 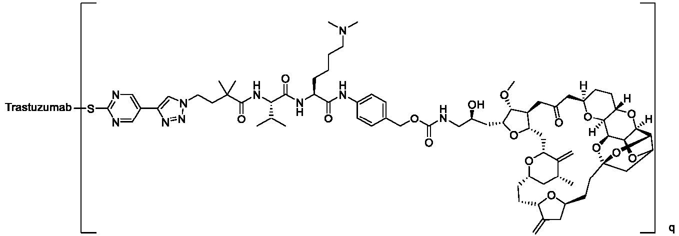 wherein q is selected from any integer between 0.1 and 10.0; preferably, q is selected from 1, 2, 3, 4, 5, 6, 7, and 8.

In some embodiments, the conjugate of antibody-eribulin or the derivative thereof is preferably a compound of the following formula, 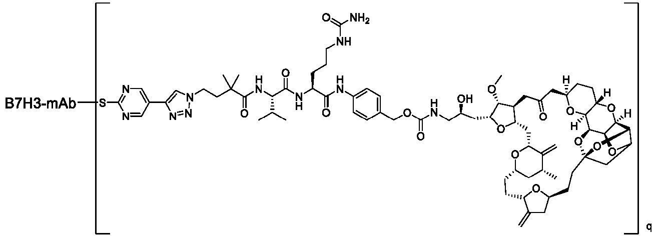 wherein B7H3-mAb is antibody 2E3-02, and q is selected from any integer between 0.1 and 10.0; preferably, q is selected from 1, 2, 3, 4, 5, 6, 7, and 8.

In some embodiments, the conjugate of antibody-eribulin or the derivative thereof is preferably a compound of the following formula, 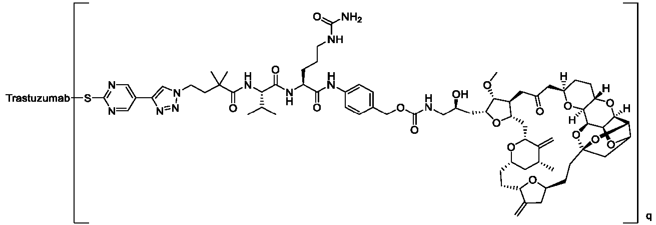 wherein the antibody is trastuzumab, and q is selected from any integer between 0.1 and 10.0; preferably, q is selected from 1, 2, 3, 4, 5, 6, 7, and 8.

The present disclosure also provides a drug-linker conjugate of general formula II, 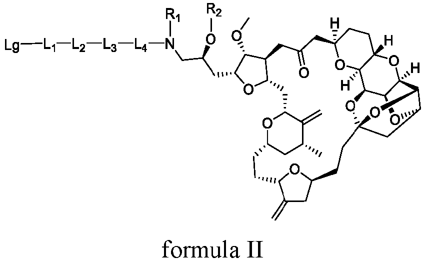
or a stereoisomer of the drug-linker conjugate, a prodrug thereof, a pharmaceutically acceptable salt thereof, or a pharmaceutically acceptable solvate thereof,
wherein
when L₁ is 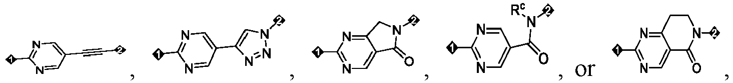 position 1 is attached to Lg and position 2 is attached to L₂;
Lg is a leaving group when reacting with Tb; L₂, L₃, L₄, R^{c}, R¹, and R² are as defined in any one of the embodiments of the present disclosure;
when L₁ is 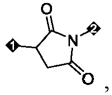 Lg-L₁ is 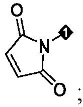 L₂, L₃, L₄, R^{c}, R¹, and R² are as defined in any one of the embodiments of the present disclosure.

In some embodiments, Lg is selected from halogen, sulfonyl, a tertiary amine salt group (Me₃N⁺, Et₃N⁺), diazonium, -OMs, MeSO₂-, and CF₃SO₃-.

In some embodiments, Lg is selected from F, Cl, and MeSO₂-.

In some embodiments, Lg is selected from F and MeSO₂-.

In some embodiments, the drug-linker conjugate of general formula II is preferably any one of the following compounds:

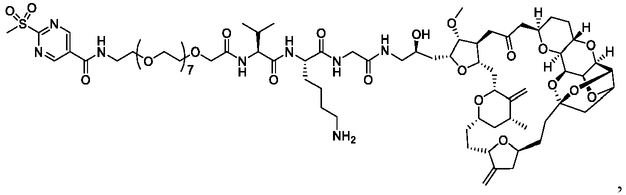

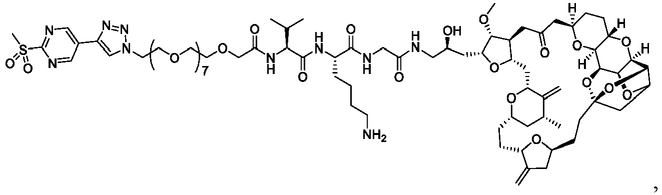

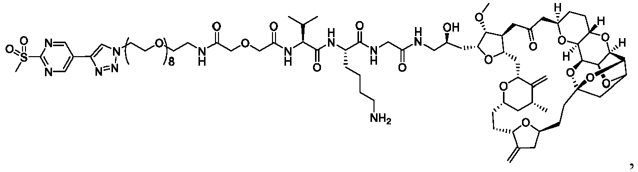

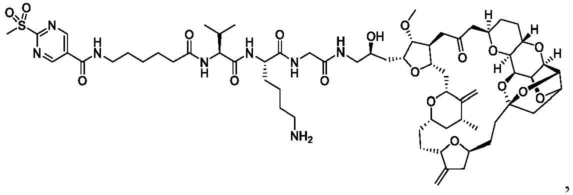

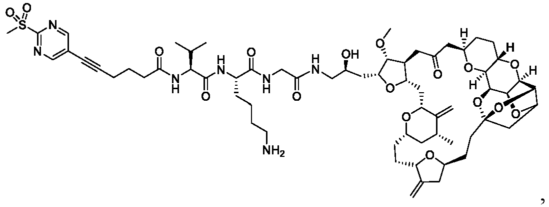

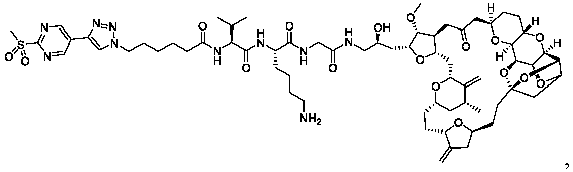

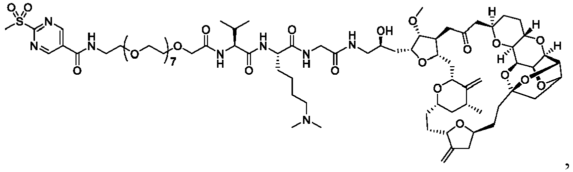

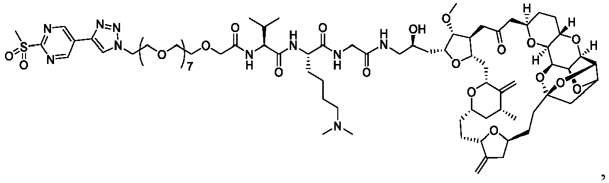

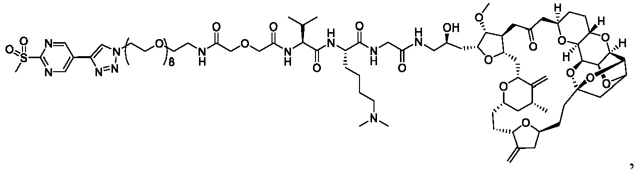

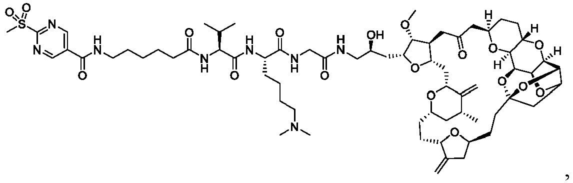

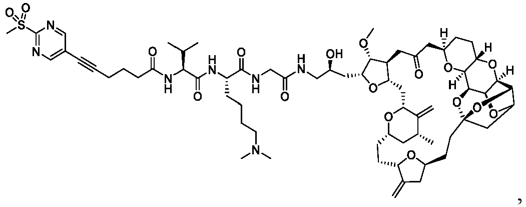

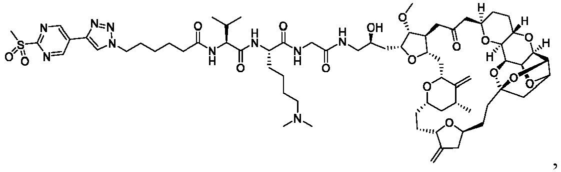

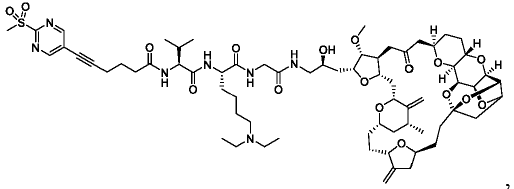

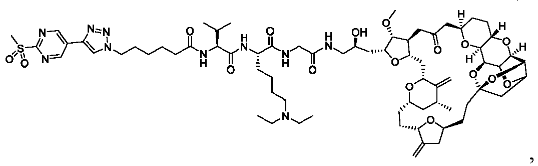

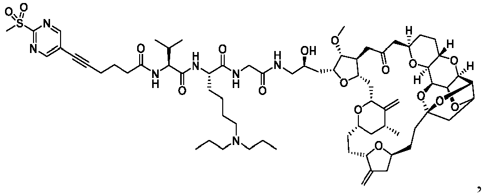

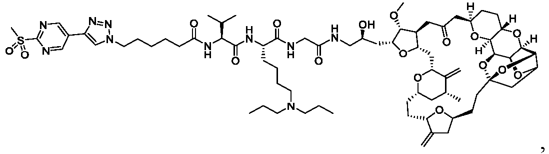

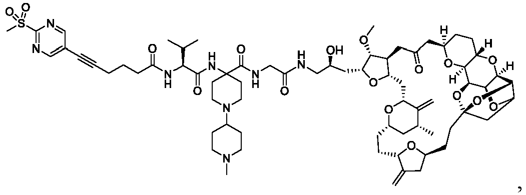

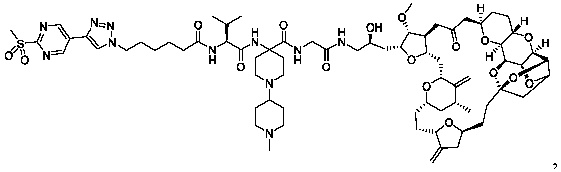

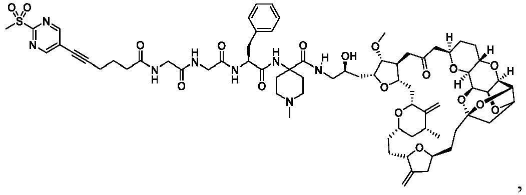

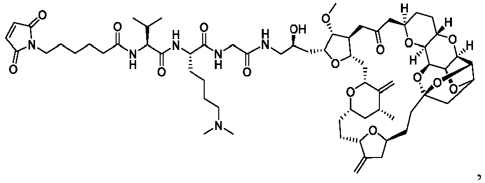

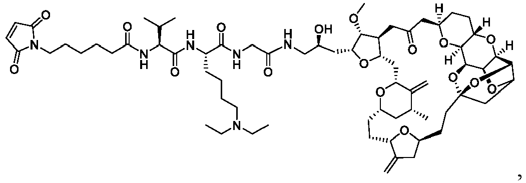

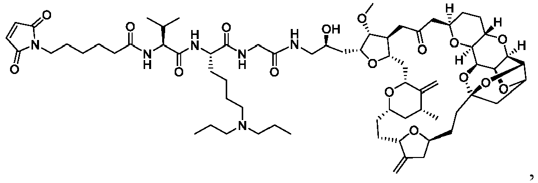

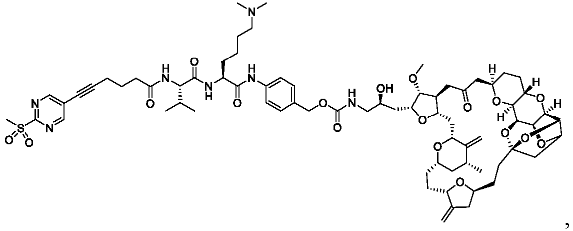

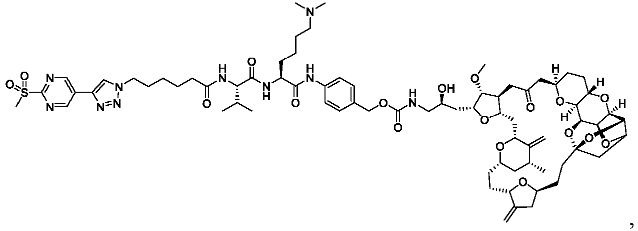

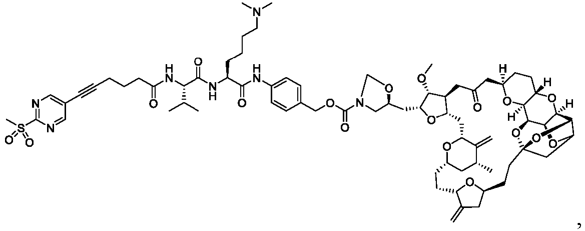

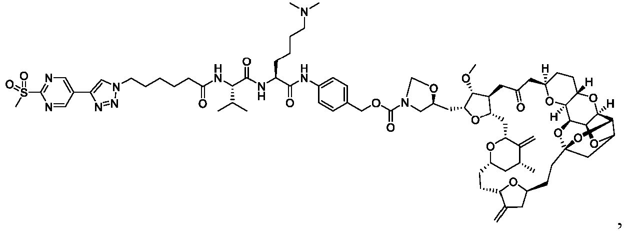

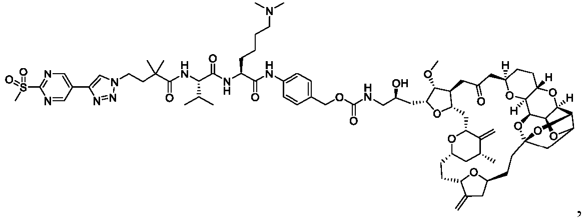

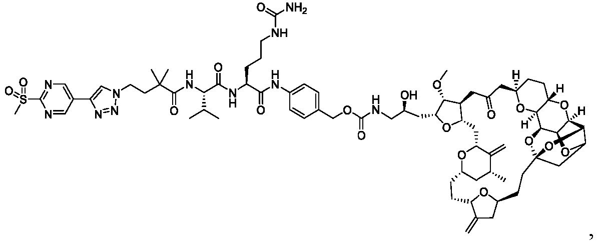

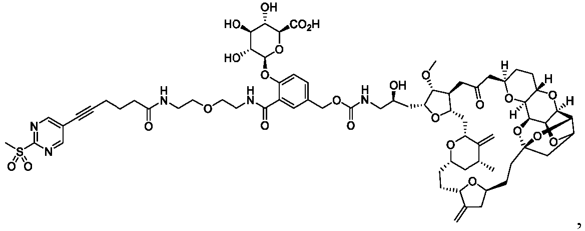

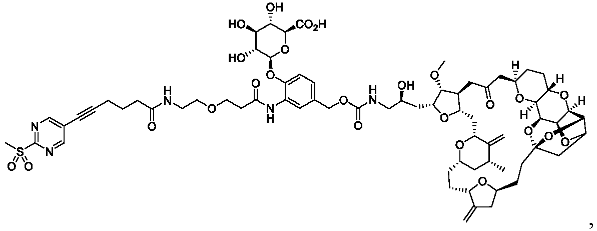

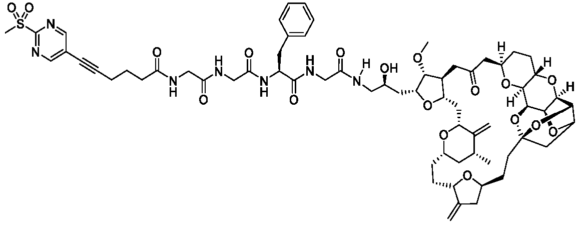

In some embodiments, the drug-linker conjugate is preferably a compound of the following formulas:

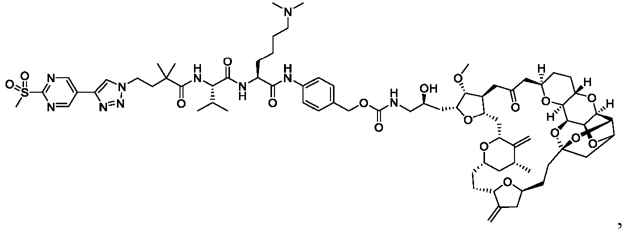

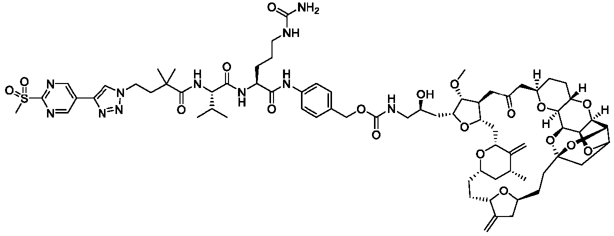

The present disclosure also provides a linker of general formula III:

Lg-L₁-L₂-L₃-L₄-Lg1 III

Lg, L₁, L₂, L₃, and L₄ are as defined in any one of the embodiments of the present disclosure; Lg1 is a leaving group when reacting with eribulin or a derivative thereof.

In some embodiments, Lg1 is preferably -OH, 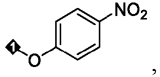 or halogen.

The present disclosure provides a method for preparing the conjugate of antibody-eribulin or the derivative thereof, the stereoisomer thereof, the pharmaceutically acceptable salt thereof, or the pharmaceutically acceptable solvate thereof, comprising: performing a coupling reaction between a sulfhydryl group on Tb and the drug-linker conjugate of general formula II 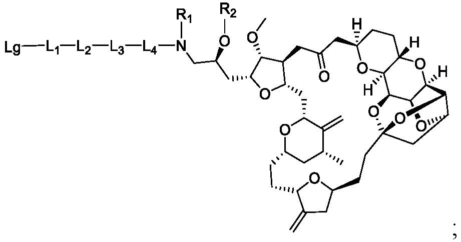 wherein Tb, q, Lg, L₁, L₂, L₃, L₄, R₁, and R₂ are as defined in any one of the embodiments of the present disclosure.

In some embodiments, the method comprises the step of performing a coupling reaction between a sulfhydryl group on Tb and the drug-linker conjugate of general formula II 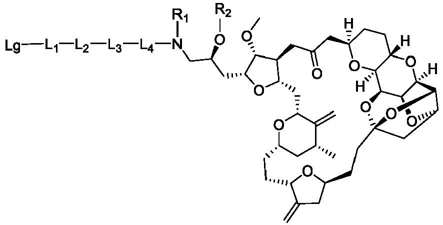 in a suitable solvent and under suitable conditions to form a C-S bond.

In some embodiments, the molar ratio of the sulfhydryl group on Tb to the drug-linker conjugate may be 1:(1-20), such as 1:(2-20), 1:(4-20), 1:(6-20), 1:(8-20), 1:(10-20), 1:(12-20), 1:(14-20), 1:(16-20), or 1:(18-20).

In some embodiments, the coupling reaction is preferably carried out in water and/or an organic solvent. The organic solvent is preferably one or more of *N,N-*dimethylformamide, dimethyl sulfoxide, N-methylpyrrolidone, a nitrile solvent (e.g., acetonitrile), and an alcohol solvent (e.g., methanol, ethanol).

In some embodiments, the post-treatment operation of the method may be a conventional post-treatment method for coupling reactions in the art, preferably purifying the coupling product by chromatography. The chromatography is typically one or more of ion exchange chromatography, hydrophobic chromatography, reversed-phase chromatography, or affinity chromatography.

The present disclosure provides a method for preparing the drug-linker conjugate of formula II, which is selected from any one of the following methods:
method I comprises: performing a reaction between the linker of general formula III and eribulin or the derivative thereof as shown below; 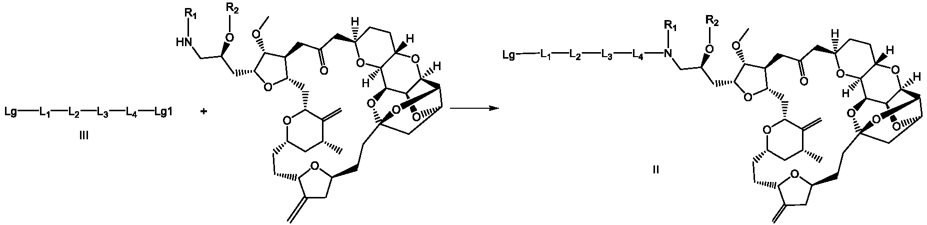
Lg, L₁, L₂, L₃, L₄, and Lg1 are as defined in any one of the embodiments of the present disclosure; wherein eribulin or the derivative thereof contains a group that reacts with Lg1;
method II comprises: performing a coupling reaction between a compound of general formula III' and a compound of general formula III" as shown below; 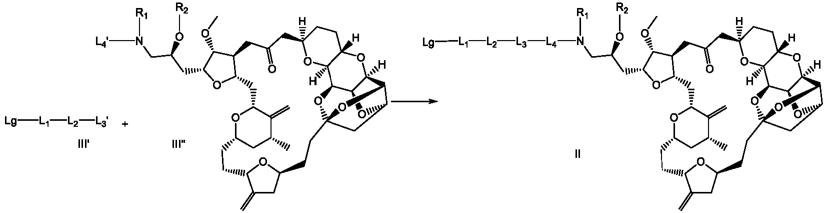
Lg, L₁, L₂, L₃, L₄, R₁, and R₂ are as defined in any one of the embodiments of the present disclosure; L₃-L₄ is obtained by the reaction of L₃' and L₄'.

As a preferred embodiment, the method for preparing the drug-linker conjugate of formula II of the present disclosure comprises the following steps: activating fragment A and performing a coupling reaction with fragment C as shown below to obtain coupling product D, which is the drug-linker conjugate of formula II;

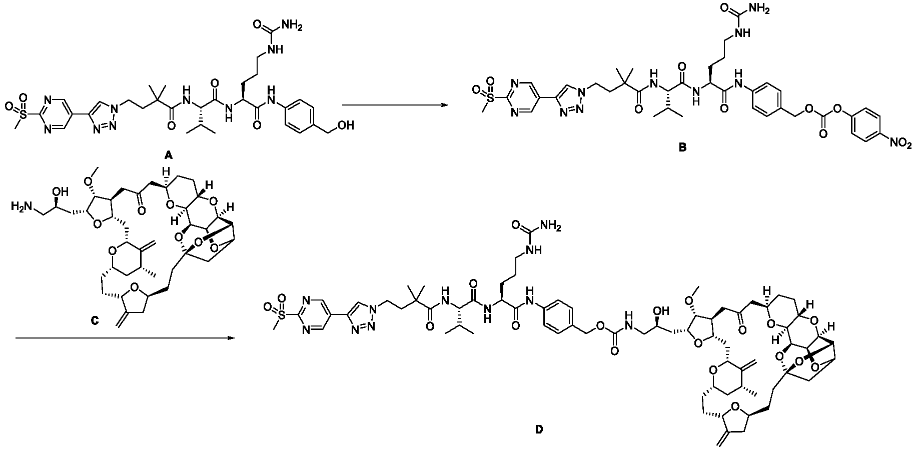

### Conjugate population of antibody-eribulin or derivative thereof

The present disclosure provides a conjugate population of antibody-eribulin or the derivative thereof, comprising the conjugate of antibody-eribulin or the derivative thereof, the stereoisomer thereof, the pharmaceutically acceptable salt thereof, or the pharmaceutically acceptable solvate thereof; the conjugate of antibody-eribulin or the derivative thereof in the mixture has two or more q values.
In some examples, the drug-to-antibody ratio (DAR) in the conjugate population of antibody-eribulin or the derivative thereof is selected from an integer or decimal between 1 and 10;
in some examples, the drug-to-antibody ratio (DAR) in the conjugate population of antibody-eribulin or the derivative thereof is selected from an integer or decimal between 1 and 8;
in some examples, the drug-to-antibody ratio (DAR) in the conjugate population of antibody-eribulin or the derivative thereof is selected from an integer or decimal between 2 and 8;
in some examples, the drug-to-antibody ratio (DAR) in the conjugate population of antibody-eribulin or the derivative thereof is selected from an integer or decimal of 2 ± 0.5, 4 ± 0.5, 6 ± 0.5, and 8 ± 0.5;
in some preferred examples, the drug-to-antibody ratio (DAR) in the conjugate population of antibody-eribulin or the derivative thereof is selected from 2, 2.5, 3, 3.5, 4, 4.2, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, and 9; such as 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, and 9.

In one example, the ADC conjugate population of antibody-eribulin or the derivative thereof comprises an ADC having a DAR distribution of 1 to 8, such as 1.5, 2, 4, 6, and 8 *(i.e.,* 1.5, 2, 4, 6, and 8 payload species). It should be noted that degradation products can be generated, such that the conjugate population of antibody-eribulin or the derivative thereof may also contain a DAR of 1, 3, 5, and 7. Furthermore, the ADC in the conjugate population of antibody-eribulin or the derivative thereof may also have a DAR greater than 8. The ADC conjugate population of antibody-eribulin or the derivative thereof is produced by reduction of interchain disulfide followed by coupling.

In one example, the ADC conjugate population of antibody-eribulin or the derivative thereof comprises both of the following: an ADC having a DAR of 4 or less *(i.e.,* the payload species are 4 or less) and an ADC having a DAR of 6 or more *(i.e.,* the payload species are 6 or more).

The present disclosure provides a pharmaceutical composition comprising the conjugate of antibody-eribulin or the derivative thereof, the stereoisomer thereof, the pharmaceutically acceptable salt thereof, or the pharmaceutically acceptable solvate thereof, or the drug-linker conjugate of formula II, the stereoisomer thereof, the pharmaceutically acceptable salt thereof, or the pharmaceutically acceptable solvate thereof, or the conjugate population of antibody-eribulin or the derivative thereof, and optionally one or more pharmaceutically acceptable adjuvants. The conjugate of antibody-eribulin or the derivative thereof, the stereoisomer thereof, the pharmaceutically acceptable salt thereof, or the pharmaceutically acceptable solvate thereof, or the drug-linker conjugate of formula II, the stereoisomer thereof, the pharmaceutically acceptable salt thereof, or the pharmaceutically acceptable solvate thereof, or the conjugate population of antibody-eribulin or the derivative thereof may be used in a therapeutically effective amount.

The present disclosure provides a pharmaceutical composition, and when the conjugate of antibody-eribulin or the derivative thereof comprised in the pharmaceutical composition has only one q value, the ratio of the conjugate of eribulin or the derivative thereof to antibody (DAR) is consistent with the q value. In some embodiments, the ratio of the conjugate of eribulin or the derivative thereof to antibody (DAR) is selected from 1 to 8. In some preferred embodiments, the ratio of the conjugate of eribulin or the derivative thereof to antibody (DAR) is selected from 1, 2, 3, 4, 5, 6, 7, and 8.

The present disclosure provides a use of the conjugate of antibody-eribulin or the derivative thereof, the stereoisomer thereof, the prodrug thereof, the pharmaceutically acceptable salt thereof, or the pharmaceutically acceptable solvate thereof, or the drug-linker conjugate of formula II, the stereoisomer thereof, the prodrug thereof, the pharmaceutically acceptable salt thereof, or the pharmaceutically acceptable solvate thereof, or the conjugate population of antibody-eribulin or the derivative thereof, or the pharmaceutical composition in the manufacture of a medicament for the treatment and/or prevention of a disease *(e.g.,* a cancer disease) associated with abnormal cell activity. The conjugate of antibody-eribulin or the derivative thereof, the stereoisomer thereof, the prodrug thereof, the pharmaceutically acceptable salt thereof, or the pharmaceutically acceptable solvate thereof, or the drug-linker conjugate of formula II, the stereoisomer thereof, the prodrug thereof, the pharmaceutically acceptable salt thereof, or the pharmaceutically acceptable solvate thereof, or the conjugate population of antibody-eribulin or the derivative thereof, or the pharmaceutical composition may be used in a therapeutically effective amount.

The present disclosure provides a method for preventing and/or treating a disease (e.g., a cancer disease) associated with abnormal cell activity, comprising: administering to an individual in need a prophylactically and/or therapeutically effective amount of the conjugate of antibody-eribulin or the derivative thereof, the stereoisomer thereof, the prodrug thereof, the pharmaceutically acceptable salt thereof, or the pharmaceutically acceptable solvate thereof, or the drug-linker conjugate of formula II, the stereoisomer thereof, the prodrug thereof, the pharmaceutically acceptable salt thereof, or the pharmaceutically acceptable solvate thereof, or the conjugate population of antibody-eribulin or the derivative thereof, or the pharmaceutical composition.

In some embodiments, the disease associated with abnormal cell activity may be a hematological and/or solid tumor, for example selected from esophageal cancer (*e.g*., esophageal adenocarcinoma and esophageal squamous cell carcinoma), brain tumor, lung cancer (*e.g*., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, urothelial cancer, solid tumor, non-Hodgkin lymphoma, central nervous system tumor (*e.g*., neuroglioma, glioblastoma multiforme, glioma, or sarcoma), prostate cancer, or thyroid cancer.

In some embodiments, the disease is preferably a cancer disease associated with B7H3.

In some embodiments, the disease is preferably a cancer disease associated with HER2.

In some embodiments, the disease is preferably a cancer disease associated with TROP2.

In some embodiments, the disease is preferably a cancer disease associated with EGFR.

In some embodiments, the disease is preferably a solid tumor.

In the present disclosure, unless otherwise stated, the scientific and technical terms used herein have the meanings that are generally understood by those skilled in the art. Moreover, the cell culture, molecular genetics, nucleic acid chemistry, and immunology laboratory procedures used herein are all routine procedures widely used in the corresponding fields. Meanwhile, for a better understanding of the present disclosure, definitions and explanations of relevant terms are provided below.

As used herein, examples of the term "pharmaceutically acceptable salt" are organic acid addition salts formed from organic acids that form pharmaceutically acceptable anions, including, but not limited to, formate, acetate, propionate, benzoate, maleate, fumarate, succinate, tartrate, citrate, ascorbate, α-ketoglutarate, α-glycerophosphate, alkyl sulfonate, or arylsulfonate; preferably, the alkylsulfonate is methylsulfonate or ethylsulfonate; the arylsulfonate is benzenesulfonate or *p*-toluenesulfonate. Suitable inorganic salts may also be formed, including, but not limited to, hydrochloride, hydrobromide, hydroiodate, nitrate, bicarbonate, carbonate, sulfate, phosphate, *etc.*

Pharmaceutically acceptable salts can be obtained using standard procedures well known in the art, for example, by reacting a sufficient amount of a basic compound with a suitable acid providing a pharmaceutically acceptable anion.

In the present disclosure, the pharmaceutically acceptable adjuvant refers to the excipient and additive used in the manufacture of medicaments and the formulation of prescriptions, and refers to the substances contained in a pharmaceutical preparation whose safety has been reasonably evaluated, besides the active ingredients. In addition to shaping, acting as a carrier, and improving stability, the pharmaceutically acceptable adjuvant also has important functions such as solubilization, hydrotropy, sustained and controlled release, and is an important ingredient that may affect the quality, safety, and efficacy of drugs. According to its source, the pharmaceutically acceptable adjuvant can be divided into natural product, semi-synthetic product, and full synthetic product. According to its function and use, the pharmaceutically acceptable adjuvant can be divided into: solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adherents, antioxidants, chelating agents, penetration enhancers, pH adjusters, buffers, plasticizers, surfactants, foaming agents, defoaming agents, thickeners, inclusion agents, humectants, absorbents, diluents, flocculants and deflocculants, filter aids, release blockers, *etc.* According to its route of administration, the pharmaceutically acceptable adjuvant can be divided into oral administration, injection, mucosal administration, transdermal or topical administration, nasal or oral inhalation administration, ocular administration, *etc.* The same pharmaceutically acceptable adjuvant can be used in pharmaceutical preparations with different routes of administration, and has different effects and uses.

The pharmaceutical composition can be prepared into various suitable dosage forms according to the route of administration. For example, tablets, capsules, granules, oral solutions, oral suspensions, oral emulsions, powders, tinctures, syrups, injections, suppositories, ointments, creams, pastes, ophthalmic preparations, pills, implants, aerosols, powder aerosols, sprays, *etc.* Herein, the pharmaceutical composition or suitable dosage form may contain 0.01 mg to 1000 mg of the compound of the present disclosure or a pharmaceutically acceptable salt or conjugate thereof, suitably 0.1 mg to 800 mg, preferably 0.5 mg to 500 mg, preferably 0.5 mg to 350 mg, particularly preferably 1 mg to 250 mg.

The pharmaceutical composition can be administered in the form of injection, including injection solution, sterile powder for injection, and concentrated solution for injection. Herein, useful carriers and solvents include water, Ringer's solution, and isotonic sodium chloride solution. In addition, sterile non-volatile oils can also be used as a solvent or suspending medium, such as monoglycerides or diglycerides.

The term "treatment" as used herein generally refers to obtaining a desired pharmacological and/or physiological effect. The effect may be prophylactic in terms of complete or partial prevention of a disease or its symptoms; and/or therapeutic in terms of partial or complete stabilization or cure of a disease and/or side effects due to the disease. "Treatment" as used herein encompasses any treatment of a disease in a patient, including: (a) prevention of a disease or symptom in a patient who is susceptible to the disease or symptom but has not yet been diagnosed with the disease; (b) suppression of symptoms of the disease, *i.e*., preventing its development; or (c) alleviation of symptoms of the disease, *i.e*., causing regression of the disease or symptom.

In the present disclosure, the term "individual" includes humans or non-human animals. Exemplary human individuals include human individuals (referred to as patients) with a disease (*e.g*., a disease described herein) or normal individuals. In the present disclosure, the term "non-human animal" includes all vertebrates, such as non-mammals (*e.g*., birds, amphibians, reptiles) and mammals, such as non-human primates, livestocks, and/or domesticated animals (*e.g*., sheep, dogs, cats, cows, pigs, *etc.).*

In the present disclosure, the term "effective dose" refers to the amount of a compound that, when administered, alleviates one or more symptoms of the condition being treated to some extent.

In the present disclosure, the term "conjugate of antibody-eribulin or a derivative thereof" refers to a substance in which eribulin or a derivative thereof is linked to a targeting moiety. In some embodiments of the present disclosure, eribulin or a derivative thereof is linked to the targeting moiety by a linker. The linker can be broken under a specific environment or action, thereby separating the bioactive molecule from the targeting moiety. In some embodiments of the present disclosure, the linker comprises a cleavable or non-cleavable unit, such as a peptide or a disulfide bond. In some embodiments of the present disclosure, the bioactive molecule is directly linked to the targeting moiety by a covalent bond that can be broken under a specific environment or action, thereby separating the bioactive molecule from the targeting moiety.

In the present disclosure, the term "linker" refers to a fragment that links a bioactive molecule (drug molecule) to a targeting moiety.

In the present disclosure, the term "targeting moiety" refers to a moiety in a conjugate that is capable of specifically binding to a target (or portion of a target) on the cell surface. Through the interaction of the targeting moiety with the target, the conjugate can be delivered to a specific cell population.

In the present disclosure, when the targeting moiety in a conjugate is an antibody, the conjugate may be referred to as a "drug-antibody conjugate".

In the present disclosure, an antibody or antigen-binding fragment thereof includes a derivatized antibody or antigen-binding fragment thereof, such as an antibody or antigen-binding fragment thereof having a sulfhydryl group, wherein the derivatization allows the antibody to have a group or ability to react with a drug-linker conjugate. The sulfhydryl group -SH can be derivatized by breaking the disulfide bond (e.g., by reduction with the reducing agent TCEP).

In the present disclosure, the term "antibody" is used in its broadest interpretation to include intact monoclonal antibodies, polyclonal antibodies, and multi-specific antibodies (e.g., bispecific antibodies) formed from at least two intact antibodies, so long as they have the desired biological activity. In the present disclosure, "antibody" and "immunoglobulin" are used interchangeably. "Antibody molecule" or "antibody" as used herein refers to immunoglobulin molecules and immunologically active portions of immunoglobulin molecules, *i.e*., molecules containing an antigen-binding site that immune-specifically binds to an antigen. Thus, the term antibody broadly encompasses not only an intact antibody molecule, but also fragments of the antibody as well as variants (including derivatives) of the antibody and antibody fragments. When "antibody molecule" or "antibody" is used in the same context as antigen-binding fragment, "antibody molecule" or "antibody" refers to an intact antibody molecule or a full-length antibody. The term antibody molecule as described in the present specification includes, for example, but is not limited to, single-chain Fv (scFv), Fab fragments, Fab' fragments, F(ab')2, disulfide-linked Fv (sdFv), Fv, and intact antibodies or full-length antibodies. The term "single-chain Fv" or "scFv" refers to a polypeptide comprising the VL domain of an antibody linked to the VH domain of the antibody. For example, antibodies that immune-specifically bind to B7H3 can cross-react with other antigens. Preferably, antibodies that immune-specifically bind to B7H3 does not cross-react with other antigens. Antibodies that immune-specifically bind to B7H3 can be identified, for example, by immunoassays or other methods known to those skilled in the art. "Intact" antibody or "full-length" antibody refers to a protein comprising two heavy chains (H) and two light chains (L) linked to each other by disulfide bonds, and the protein comprises: (1) for the heavy chain, a heavy chain variable region (abbreviated herein as "VH") and a heavy chain constant region containing three domains CH1, CH2, and CH3; and (2) for the light chain, a light chain variable region (abbreviated herein as "VL") and a light chain constant region containing one domain CL. The antibodies of the present disclosure include, but are not limited to, monoclonal, multi-specific, humanized or chimeric antibodies, single-chain antibodies, Fab fragments, F(ab') fragments, anti-idiotypic (anti-Id) antibodies (including, for example, anti-Id antibodies of antibodies of the present disclosure), and epitope-binding fragments of any of the above antibodies. The immunoglobulin molecules of the present disclosure may be of any type (*e.g*., IgG, IgE, IgM, IgD, IgA and IgY), any class *(e.g.,* IgG1, IgG2, IgG3, IgG4, IgA1, and IgA2), or any subclass of immunoglobulin. Preferably, the antibodies of the present disclosure comprise or consist of a VH domain, a VH CDR (often represented herein by HCDR), a VL domain, or a VL CDR (often represented herein by LCDR) having any one of the amino acid sequences or fragments or variants thereof described in the table of sequence and specific information thereof.

In the present disclosure, the term "monoclonal antibody" refers to an antibody derived from a population of substantially homogeneous antibodies, i.e., the antibodies making up the population are identical except for a small number of natural mutations that may be present. Monoclonal antibodies have high specificity against one determinant (epitope) of an antigen, while polyclonal antibodies comprise different antibodies against different determinants (epitopes). In addition to specificity, the advantage of monoclonal antibodies is that they can be synthesized without contamination from other antibodies. The modifier "monoclonal" as used herein means that the antibody is characterized as being derived from a substantially homogeneous population of antibodies and should not be construed as needing to be prepared by a special method.

In some embodiments of the present disclosure, monoclonal antibodies further include, in particular, chimeric antibodies, *i.e*., a portion of the heavy and/or light chain is identical or homologous to a type, a class, or a subclass of antibodies, and the remainder is identical or homologous to another type, another class, or another subclass of antibodies, so long as they have the desired biological activity (see, for example, US 4,816,567; and Morrison et al., 1984, PNAS, 81: 6851-6855). Chimeric antibodies that can be used in the present disclosure include primatized antibodies comprising variable region antigen-binding sequences and human constant region sequences from non-human primates (*e.g*., ancient monkeys, chimpanzees, *etc*.).

The term "antibody fragment" refers to a portion of an antibody, preferably the antigen-binding or variable region. Examples of antibody fragments include Fab, Fab', F(ab')2, Fd, Fv, dAb, complementarity determining region fragments, diabodies, linear antibodies, and single-chain antibody molecules.

The term "bispecific antibody", also known as "bifunctional antibody conjugate", refers to a conjugate formed by a first antibody (fragment) and a second antibody (fragment) through a coupling arm. The conjugate retains the activity of each antibody and thus has bifunctional and bispecific properties.

The term "multi-specific antibody" includes, for example, a trispecific antibody which is an antibody with three different antigen binding specificities, and a tetraspecific antibody which is an antibody with four different antigen binding specificities.

The term "intact antibody" refers to an antibody comprising an antigen-binding variable region, a light chain constant region (CL), and a heavy chain constant region (CH1, CH2, and CH3). The constant region may be a natural sequence (*e.g*., a human natural constant region sequence) or an amino acid sequence variant thereof. The intact antibody is preferably an intact antibody with one or more effector functions.

The term "probody" is a modified antibody comprising an antibody or antibody fragment that can specifically bind to a target thereof and can be coupled with a masking moiety, wherein the masking moiety means that the cleavage constant for the binding capacity of the antibody or antibody fragment to the target thereof is at least 100-fold, 1000-fold, or 10000-fold greater than that for the binding capacity of the antibody or antibody fragment not coupled with a masking moiety to the target thereof.

In the present disclosure, a "humanized" form of a non-human (*e.g*., mouse) antibody refers to a chimeric antibody that contains minimal non-human immunoglobulin sequences. Most humanized antibodies are human recipient immunoglobulins whose hypervariable region residues have been replaced with non-human (*e.g*., mouse, rat, rabbit, or non-human primate) hypervariable region residues (donor antibodies) with the desired specificity, affinity, and functions. In some embodiments, framework region (FR) residues of human immunoglobulins are also replaced with non-human residues. Furthermore, the humanized antibody may further comprise residues not present in the recipient antibody or donor antibody. These modifications are intended to further optimize the performance of the antibody. The humanized antibody generally comprise at least one variable region, typically two variable regions, in which all or almost all of the hypervariable loops correspond to those of non-human immunoglobulins, while the FRs are full or nearly full sequences of human immunoglobulins. The humanized antibody may further comprise at least a portion of an immunoglobulin constant region (Fc, typically a human immunoglobulin Fc). For details see, for example, Jones et al., 1986, Nature, 321: 522-525; Riechmann et al., 1988, Nature, 332: 323-329; and Presta, 1992, Curr Op Struct Bwl 2: 593-596.

Intact antibodies can be divided into different "classes" based on the amino acid sequence of the heavy chain constant region. The five main classes are IgA, IgD, IgE, IgG, and IgM, several of which can also be divided into different "subclasses" (isotypes), such as IgG1, IgG2, IgG3, IgG4, IgA1, and IgA2. The heavy chain constant regions of different classes of antibodies are called α, β, ε, γ, and µ, respectively. The subunit structures and three-dimensional configurations of different classes of immunoglobulins are well known in the art.

In the present disclosure, amino acid substitutions in the antibody are mostly L-amino acid substitutions, but are not limited thereto. In some embodiments, one or more D-amino acids may be included in the antibody peptide chain. Peptides containing D-amino acids are more stable and less susceptible to degradation in the oral cavity, gut, or plasma than peptides containing only L-amino acids.

The monoclonal antibodies used in the present disclosure can be produced by a number of methods. For example, the monoclonal antibodies for use in the present disclosure can be obtained by hybridoma methods using cells from a number of species (including mouse, hamster, rat, and human) (see, for example, Kohler et al., 1975, Nature, 256: 495), or prepared by recombinant DNA techniques (see, for example, US 4,816,567), or isolated from phage antibody libraries (see, for example, Clackson et al., 1991, Nature, 352: 624-628; and Marks et al., 1991, Journal of Molecular Biology, 222: 581-597).

In some embodiments, the Tb is a targeting moiety, such as ligands, proteins, polypeptides, non-protein reagents (e.g., sugars, RNA, or DNA), and antibody analogs.

In the present disclosure, the term "targeting moiety" refers to a moiety in a conjugate that is capable of specifically binding to a target (or portion of a target) on the cell surface. Through the interaction of the targeting moiety with the target, the conjugate can be delivered to a specific cell population.

In the present disclosure, when the Tb in a conjugate is a targeting moiety, the conjugate may also be referred to as a "conjugate of antibody-eribulin or a derivative thereof".

In some embodiments of the present disclosure, the targeting moiety is trastuzumab or pertuzumab. Trastuzumab is an anti-Her2 monoclonal antibody whose amino acid sequence is known to those skilled in the art and whose schematic sequence can be seen in, for example, CN103319599, wherein the Lys at the terminal is easily deleted, but such a deletion does not affect the biological activity (see Dick, L. W. et al., Biotechnol. Bioeng., 100: 1132-1143).

In some embodiments of the present disclosure, the targeting moiety is an anti-Trop-2 antibody which is RS7 (*i.e*., sacituzumab of the present disclosure) as described in U.S. Patent No. 7,517,964; and hRS7 (*i.e*., sacituzumab of the present disclosure) as described in US2012/0237518. The anti-Trop-2 antibody that can be used in the present disclosure can also be obtained by screening through vector design and construction, as well as construction of an antibody library displaying antibodies as disclosed in CN103476941A, or by screening a G-MAB^{®} library of Sorrento Therapeutics, Inc.

In the present disclosure, ErbB2 and Her2/neu are used interchangeably, both of which represent human Her2 proteins of natural sequences (Genebank Accession No.: X03363, see, for example, Semba et al., 1985, PNAS, 82: 6497-6501; and Yamamoto et al., 1986, Nature, 319: 230-234) and functional derivatives thereof, such as amino acid sequence variants. ErbB2 represents a gene encoding human Her2 and neu represents a gene encoding rat p185neu. In some embodiments, the compound or conjugate of the present disclosure can inhibit or kill cells expressing ErbB2 receptors, such as breast cancer cells, ovarian cancer cells, gastric cancer cells, endometrial cancer cells, salivary gland cancer cells, lung cancer cells, kidney cancer cells, colon cancer cells, thyroid cancer cells, pancreatic cancer cells, bladder cancer cells, or liver cancer cells.

In the present disclosure, Trop-2 or TROP2 refers to human trophoblast cell-surface antigen 2, also known as TACSTD2, M1S1, GA733-1, EGP-1, which is a cell surface receptor expressed by many human tumor (e.g., breast cancer, colorectal cancer, lung cancer, pancreatic cancer, ovarian cancer, prostate cancer, and cervical cancer) cells. In some embodiments, the compound or conjugate of the present disclosure can inhibit or kill cells expressing TROP2 receptors, such as breast cancer cells, colorectal cancer cells, lung cancer cells, pancreatic cancer cells, ovarian cancer cells, prostate cancer cells, or cervical cancer cells.

In some embodiments of the present disclosure, the targeting moiety is an anti-B7H3 monoclonal antibody or an antigen-binding fragment thereof. In some embodiments, the anti-B7H3 antibody includes all anti-B7H3 antibodies in the prior art, for example, see CN112521512, WO2021027674, WO2021021543, WO2021006619, CN111662384, CN111454357, WO2020151384, WO2020140094, WO2020103100, WO2020102779, WO2020063673, WO2020047257, WO2020041626, CN110684790, CN110642948, WO2019225787, WO2019226017, US20190338030, CN110305213, WO2018209346, WO2018177393, US9150656, WO2016106004, WO2016044383, WO2016033225, WO2015181267, US20120294796, WO2011109400, CN101104639, WO2004093894, WO2002010187, and WO2001018021.

In some embodiments of the present disclosure, the anti-B7H3 antibody is selected from the antibody numbered M30-H1-L4 in CN103687945B and the antibody numbered mAb-C-DUBAin CN109069633A. In some embodiments of the present disclosure, the anti-B7H3 antibody is selected from 1D1 or 2E3.

As used herein, unless otherwise expressly stated, the expressions "each ... independently selected from" and "... each independently selected from" used throughout this specification are interchangeable, and should be understood in a broad sense, which can mean that in different groups, the specific options expressed for the same or different symbols do not affect each other, and which can also mean that in the same group, the specific options expressed for the same or different symbols do not affect each other.

In the structure 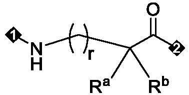 of the amino acid residue represented by AA¹, if r is 0, those skilled in the art can understand that the structure of the amino acid residue represented by AA¹ will change to

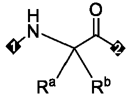

In the structure 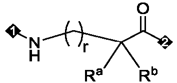 of the amino acid residue represented by AA¹, if R^{a} and R^{b} together with the carbon atom to which they are both attached form a 4- to 10-membered heterocyclic ring, the 4- to 10-membered heterocyclic ring is optionally substituted by one or more R⁰, wherein the term "the 4- to 10-membered heterocyclic ring is optionally substituted by one or more R⁰" means that the 4- to 10-membered heterocyclic ring may be either unsubstituted or substituted by one or more R⁰, and in the more R⁰, the definition of each R⁰ may be the same or different. Other similar definitions can be understood with reference to the foregoing.

In various parts of this specification, substituents of the compounds in the present disclosure are disclosed according to group types or ranges. Specifically, the present disclosure includes each individual sub-combination of respective members within these group types or ranges. For example, the term "C₁₋₆ alkyl" particularly refers to methyl, ethyl, C₃ alkyl, C₄ alkyl, C₅ alkyl, and C₆ alkyl as independently disclosed.

As used herein, the term "C₁₋₆ alkyl" refers to a linear or branched alkyl group containing 1 to 6 carbon atoms, including, for example, "C₁₋₃ alkyl" or "C₁₋₄ alkyl", methyl, ethyl, *etc.* Specific examples include, but are not limited to: methyl, ethyl, n-propyl, isopropyl, n-butyl, isobutyl, *sec*-butyl, *tert*-butyl, pentyl, and hexyl.

As used herein, the term "C₁₋₄ alkyl" refers to a linear or branched alkyl group containing 1 to 4 carbon atoms, including, for example, "C₁₋₃ alkyl", methyl, ethyl, *etc.* Specific examples include, but are not limited to: methyl, ethyl, *n*-propyl, isopropyl, *n*-butyl, isobutyl, sec-butyl, and *tert*-butyl.

As used herein, the term "C₂₋₆ alkenyl" refers to a linear, branched, or cyclic alkenyl group containing at least one double bond and 2 to 6 carbon atoms, including, for example, "C₂₋₄ alkenyl", *etc.* Examples include, but are not limited to: vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl, *etc.*

As used herein, the term "C₂₋₆ alkynyl" refers to a linear or branched alkynyl group containing at least one triple bond and 2 to 6 carbon atoms, including, for example, "C₂₋₄ alkynyl", *etc.* Examples include, but are not limited to: ethynyl, propynyl, 2-butynyl, 2-pentynyl, 3-pentynyl, 4-methyl-2-pentynyl, 2-hexynyl, 3-hexynyl, 5-methyl-2-hexynyl, *etc.*

As used herein, the term "halogen" includes fluorine, chlorine, bromine, and iodine.

As used herein, the term "3- to 6-membered cycloalkyl" or "C₃₋₆ cycloalkyl" refers to a saturated cyclic alkyl group containing 3 to 6 carbon atoms, including cyclopropane (*i.e*., cyclopropyl), cyclobutane (*i.e*., cyclobutyl), cyclopentane (*i.e*., cyclopentyl), and cyclohexyl.

As used herein, the term "3- to 7-membered carbocycloalkyl" or "C₃₋₇ cycloalkyl" refers to a saturated cyclic alkyl group containing 3 to 7 carbon atoms, including cyclopropyl, cyclobutyl, cyclopentyl, cyclohexyl, and cycloheptyl.

As used herein, the term "3- to 8-membered carbocyclic ring" refers to a saturated, unsaturated, or partially unsaturated carbocyclic ring containing 3 to 8 carbon atoms. Specific examples include, but are not limited to, cyclopropane (*i.e*., cyclopropyl), cyclobutane (*i.e*., cyclobutyl), cyclopentane (*i.e*., cyclopentyl), and cyclohexane (*i.e*., cyclohexyl).

As used herein, the term "C₁₋₆ alkoxy" refers to an alkyl group as defined above which is attached to the parent molecular moiety through an oxygen atom. Specific examples include, but are not limited to, methoxy, ethoxy, propoxy, isopropoxy, n-propoxy, isopropoxy, n-butoxy, isobutoxy, *tert*-butoxy, pentoxy, hexyloxy, *etc.*

As used herein, the term "C₁₋₄ alkoxy" refers to an alkyl group as defined above which is attached to the parent molecular moiety through an oxygen atom. Specific examples include, but are not limited to, methoxy, ethoxy, propoxy, isopropoxy, *n*-propoxy, isopropoxy, *n*-butoxy, isobutoxy, *tert*-butoxy, *etc.*

As used herein, the term "4- to 10-membered heterocyclyl" refers to a cyclic group containing 4 to 10 ring atoms (at least one of which is a heteroatom, such as a nitrogen atom, an oxygen atom, or a sulfur atom). The term "4- to 6-membered heterocyclyl" refers to a cyclic group containing 4 to 6 ring atoms (at least one of which is a heteroatom, such as a nitrogen atom, an oxygen atom, or a sulfur atom). Optionally, ring atoms (*e.g*., carbon atoms, nitrogen atoms, or sulfur atoms) in the cyclic structure may be oxidized. "4- to 8-membered heterocyclyl" includes, for example, "4- to 8-membered nitrogen-containing heterocyclyl", "4-to 8-membered oxygen-containing heterocyclyl", "4- to 7-membered heterocyclyl", "4- to 7-membered oxygen-containing heterocyclyl", "4- to 7-membered heterocyclyl", "4- to 6-membered heterocyclyl", "5- to 7-membered heterocyclyl", "5- to 6-membered heterocyclyl", "5- to 6-membered nitrogen-containing heterocyclyl", including, but not limited to, oxetanyl, pyrrolidinyl, tetrahydrofuryl, piperidinyl, piperazinyl, tetrahydropyranyl, homopiperazinyl, *etc.*

As used herein, the term "4- to 10-membered heterocyclic ring" refers to a ring containing 4 to 10 ring atoms (at least one of which is a heteroatom, such as a nitrogen atom, an oxygen atom, or a sulfur atom). The term "5- to 6-membered heterocyclic ring" refers to a ring containing 5 to 6 ring atoms (at least one of which is a heteroatom, such as a nitrogen atom, an oxygen atom, or a sulfur atom), including, but not limited to, pyrrolidine, tetrahydrofuran, piperidine, piperazine, tetrahydropyran, and other rings.

As used herein, the term "3- to 8-membered heterocyclic ring" refers to a ring containing 3 to 8 ring atoms (at least one of which is a heteroatom, such as a nitrogen atom, an oxygen atom, or a sulfur atom).

As used herein, the term "aryl" refers to an aromatic monocyclic or polycyclic hydrocarbon group, such as 6- to 10-membered aryl, 5- to 8-membered aryl, *etc.* Specific examples include, but are not limited to, phenyl, naphthyl, anthracenyl, phenanthryl, *etc.* The "6- to 10-membered aryl" refers to an aryl group containing 6 to 10 ring atoms. The "C₆₋₁₀ aryl" refers to an aryl group containing 6 to 10 carbon atoms.

As used herein, the term "heteroaryl" refers to an aromatic cyclic group, in which at least one ring atom is a heteroatom, such as a nitrogen atom, an oxygen atom, or a sulfur atom. Optionally, ring atoms (*e.g*., carbon atoms, nitrogen atoms, or sulfur atoms) in the cyclic structure may be oxidized. Specific examples include, but are not limited to, 5- to 10-membered heteroaryl, 5- to 6-membered heteroaryl, 5- to 10-membered nitrogen-containing heteroaryl, 6- to 10-membered oxygen-containing heteroaryl, 6- to 8-membered nitrogen-containing heteroaryl, 5- to 8-membered oxygen-containing heteroaryl, *etc.,* such as furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidinyl, 1,4-dioxacyclohexadienyl, 2*H*-1,2-oxazinyl, 4*H*-1,2-oxazinyl, 6*H*-1,2-oxazinyl, 4*H*-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetrazinyl, azacycloheptatrienyl, 1,3-diazacycloheptatrienyl, azacyclooctatetraenyl, *etc.*

A bond in a structural formula represented herein by a wavy line "∼∼" is intended to indicate that the structure represents a *cis* or *trans* isomer, or a mixture of *cis* and *trans* isomers in any ratio.

On the basis of not violating common knowledge in the art, the above preferred conditions can be arbitrarily combined to obtain preferred examples of the present disclosure.

The reagents and raw materials used in the present disclosure are all commercially available.

The positive and progressive effect of the present disclosure is as follows:

The antibody-eribulin or the derivative thereof of the present disclosure can achieve at least one of the following technical effects:
(1) The high anti-tumor activity enables more effective killing of tumor cells, thus having better *in vivo* or *in vitro* anti-tumor effect than the antibody-eribulin or the derivative thereof linked to the same small molecule drug in the prior art.
(2) The high stability of bioactive molecules in the circulation can reduce the shedding of non-targeted bioactive molecules in non-target cells and increase the effective intracellular release of bioactive molecules, allowing the bioactive molecules to have a higher concentration ratio between tumor and blood, thus having a higher therapeutic index.
(3) By adding basic nitrogen atoms to the linker, the exposure of the entire antibody-eribulin or the derivative thereof in a relatively acidic tumor environment is increased, resulting in better tumor tissue targeting and an increase in the concentration ratio of bioactive molecules between tumor and blood, thus having a higher therapeutic index.

### Sequence and specific information thereof:

| **SEQ ID NO** | **Sequence name** | **Amino acid sequence** |
|---|---|---|
| 1 | 1D1-01 VH | EGQLVESGGGLVQPGGSLRLSCAASGFTISSYWMNWVRQAPGK GLEWVANIKQDGSEKYYVDSVQGRFTISRDNAKNSLYLQMNSL RAEDTAVYYCARRMYSSGWYWGAFDMWGQGTMVTVSS |
| 2 | 1D1-01 VL | EIVLTQSPGTLSLSPGERATLSCRASQSVSSIYLAWNQQKPGQAP RLLIYGTFKRATGIPDRFSGSGSGTDFTLTISSLEPEDFAVYYCQQ YDTSSITFGQGTRLEIK |
| 3 | 1D1-01 HC, namely 1D1-01 heavy chain | EGQLVESGGGLVQPGGSLRLSCAASGFTISSYWMNWVRQAPGK GLEWVANIKQDGSEKYYVDSVQGRFTISRDNAKNSLYLQMNSL RAEDTAVYYCARRMYSSGWYWGAFDMWGQGTMVTVSSAST KGPSVFPLAPSSKSTSGGTAALGCLVKDYFPEPVTVSWNSGALT SGVHTFPAVLQSSGLYSLSSVVTVPSSSLGTQTYICNVNHKPSNT KVDKKVEPKSCDKTHTCPPCPAPELLGGPSVFLFPPKPKDTLMIS RTPEVTCVVVDVSHEDPEVKFNWYVDGVEVHNAKTKPREEQY NSTYRVVSVLTVLHQDWLNGKEYKCKVSNKALPAPIEKTISKA KGQPREPQVYTLPPSRDELTKNQVSLTCLVKGFYPSDIAVEWES NGQPENNYKTTPPVLDSDGSFFLYSKLTVDKSRWQQGNVFSCS VMHEALHNHYTQKSLSLSPGK |
| 4 | 1D1-01 LC, namely 1D1-01 light chain | EIVLTQSPGTLSLSPGERATLSCRASQSVSSIYLAWNQQKPGQAP RLLIYGTFKRATGIPDRFSGSGSGTDFTLTISSLEPEDFAVYYCQQ YDTSSITFGQGTRLEIKRTVAAPSVFIFPPSDEQLKSGTASVVCLL NNFYPREAKVQWKVDNALQSGNSQESVTEQDSKDSTYSLSSTL TLSKADYEKHKVYACEVTHQGLSSPVTKSFNRGEC |
| 5 | 2E3-02 VH | EVQLLESGGGLVQPGGSLRLSCAASGFTFSSYAMNWVRQAPGK GLEWVSGISGSGGSTYYADSVQGRFTISRDNSKNTLYLQMNSLR |
| | | AEDTAVYYCAKARSGYDYFFDYWGQGTLVTVSS |
| 6 | 2E3-02 VL | EIVLTQSPGTLSLSPGERATLSCRASQRISSSFLAWYQQKPGQAP SLLIYGASSRATGIPDRFSGSGSGTDFTLTISSLEPEDFAVFYCQQ YSNSPLTFGGGTKVEIK |
| 7 | 2E3-02 HC, namely 2E3-02 heavy chain | EVQLLESGGGLVQPGGSLRLSCAASGFTFSSYAMNWVRQAPGK GLEWVSGISGSGGSTYYADSVQGRFTISRDNSKNTLYLQMNSLR AEDTAVYYCAKARSGYDYFFDYWGQGTLVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPELLGGPSVFLFPPKPKDTLMISRTPEVT CVVVDVSHEDPEVKFNWYVDGVEVHNAKTKPREEQYNSTYRV VSVLTVLHQDWLNGKEYKCKVSNKALPAPIEKTISKAKGQPRE PQVYTLPPSRDELTKNQVSLTCLVKGFYPSDIAVEWESNGQPEN NYKTTPPVLDSDGSFFLYSKLTVDKSRWQQGNVFSCSVMHEAL HNHYTQKSLSLSPGK |
| 8 | 2E3-02 LC, namely 2E3-02 light chain | EIVLTQSPGTLSLSPGERATLSCRASQRISSSFLAWYQQKPGQAP SLLIYGASSRATGIPDRFSGSGSGTDFTLTISSLEPEDFAVFYCQQ YSNSPLTFGGGTKVEIKRTVAAPSVFIFPPSDEQLKSGTASVVCLL NNFYPREAKVQWKVDNALQSGNSQESVTEQDSKDSTYSLSSTL TLSKADYEKHKVYACEVTHQGLSSPVTKSFNRGEC |
| 9 | 2E3-02 Kabat HCDR1 | SYAMN |
| 10 | 2E3-02 Kabat HCDR2 | GISGSGGSTYYADSVQG |
| 11 | 2E3-02 Kabat HCDR3 | ARSGYDYFFDY |
| 12 | 2E3-02 Kabat LCDR1 | RASQRISSSFLA |
| 13 | 2E3-02 Kabat LCDR2 | GASSRAT |
| 14 | 2E3-02 Kabat LCDR3 | QQYSNSPLT |
| 15 | 2E3-02 IMGT HCDR1 | GFTFSSYA |
| 16 | 2E3-02 IMGT HCDR2 | ISGSGGST |
| 17 | 2E3-02 IMGT HCDR3 | AKARSGYDYFFDY |
| 18 | 2E3-02 IMGT LCDR1 | QRISSSF |
| \ | 2E3-02 IMGT LCDR2 | GAS |
| 19 | 2E3-02 IMGT LCDR3 | QQYSNSPLT |
| 20 | IgG1 CH, namely IgG1 constant region | |
| 21 | Kappa CL, namely Kappa constant region | |

### BRIEF DESCRIPTION OF THE DRAWINGS

FIG. 1A shows the binding assay of anti-B7H3 antibody 1D1-01 to human B7H3-4IgG-His protein by ELISA.
FIG. 1B shows the binding assay of anti-B7H3 antibody 2E3-02 to human B7H3-4IgG-His protein by ELISA.
FIG. 2A shows the binding assay of anti-B7H3 antibody 1D1-01 to monkey B7H3-4IgG-His protein by ELISA.
FIG. 2B shows the binding assay of anti-B7H3 antibody 2E3-02 to monkey B7H3-4IgG-His protein by ELISA.
FIG. 3A shows the binding assay of anti-B7H3 antibody 1D1-01 to MCF-7 tumor cells by flow cytometry.
FIG. 3B shows the binding assay of anti-B7H3 antibody 2E3-02 to MCF-7 tumor cells by flow cytometry.
FIG. 4A shows the binding assay of anti-B7H3 antibody 1D1-01 to A549 tumor cells by flow cytometry.
FIG. 4B shows the binding assay of anti-B7H3 antibody 2E3-02 to A549 tumor cells by flow cytometry.
FIG. 5 shows the binding assay of anti-B7H3 antibody 2E3-02 to PC-3 tumor cells by flow cytometry.
FIG. 6A shows the endocytosis assay of candidate antibody 1D1-01 by tumor cells A549.
FIG. 6B shows the endocytosis assay of candidate antibody 2E3-02 by tumor cells A549.
FIG. 7A shows the inhibitory activity assay of B7H3-ADC-01 in SW480 cells.
FIG. 7B shows the inhibitory activity assay of B7H3-ADC-01 in A375 cells.
FIG. 7C shows the inhibitory activity assay of B7H3-ADC-01 in A549 cells.
FIG. 7D shows the inhibitory activity assay of B7H3-ADC-01 in HT29 cells.
FIG. 7E shows the inhibitory activity assay of HER2-ADC-04 in H358 cells.
FIG. 8A shows the tumor growth inhibition assay of B7H3-ADC-01 in NCI-HT29 xenograft model.
FIG. 8B shows the changes in body weight of mice in NCI-HT29 xenograft model.

### DETAILED DESCRIPTION OF THE PREFERRED EMBODIMENT

The present disclosure is further illustrated below by means of examples, but the present disclosure is not limited to the scope of the examples. The experimental methods for which specific conditions are not indicated in the following examples are selected according to conventional methods and conditions, or according to the product instructions.

The abbreviations in the present disclosure have the following meanings:

| | | | |
|---|---|---|---|
| OMs | Methanesulfonate | FA | Formic acid |
| OTs | Trifluoromethanesulfonate | ACN | Acetonitrile |
| OTf | *p*-Toluenesulfonate | CCK8 reagent | 2-(2-Methoxy-4-nitrophenyl)-3 -(4-nitrophenyl)-5-(2,4-disulfophenyl)-2*H*-tetrazolium monosodium salt |
| TBS | *tert*-Butyldimethylsilyl | FBS | Fetal bovine serum |
| MMT | *p*-Methoxytrityl | DMSO | Dimethyl sulfoxide |
| PB/PBS | Phosphate buffered saline | MTBE | Methyl *tert*-butyl ether |

The structures of the compounds described in the following examples were determined by nuclear magnetic resonance (¹H NMR) or mass spectrometry (MS).

The instrument for detecting nuclear magnetic resonance (¹H NMR) is a Bruker 400 MHz NMR instrument; the test solvent is deuterated methanol (CD₃OD), deuterated chloroform (CDCl₃), or hexadeuterodimethyl sulfoxide (DMSO-d₆); the internal standard substance is tetramethylsilane (TMS).

Abbreviations in nuclear magnetic resonance (NMR) spectra used in the examples are shown below.

s: singlet, d: doublet, t: triplet, q: quartet, dd: double doublet, qd: quartet doublet, ddd: double double doublet, ddt: double double triplet, dddd: double double double doublet, m: multiplet, br: broad, J: coupling constant, Hz: hertz, DMSO-d₆: deuterated dimethyl sulfoxide. δ value is expressed in ppm.

The instrument for detecting mass spectrometry (MS) is an Agilent (ESI) mass spectrometer (model Agilent 6120B).

### I. Synthesis of bioactive molecules and intermediates used in the synthesis of "drug-linker compounds"

### Example 1 (A1):

### N 2-Aminoacetyl-eribulin

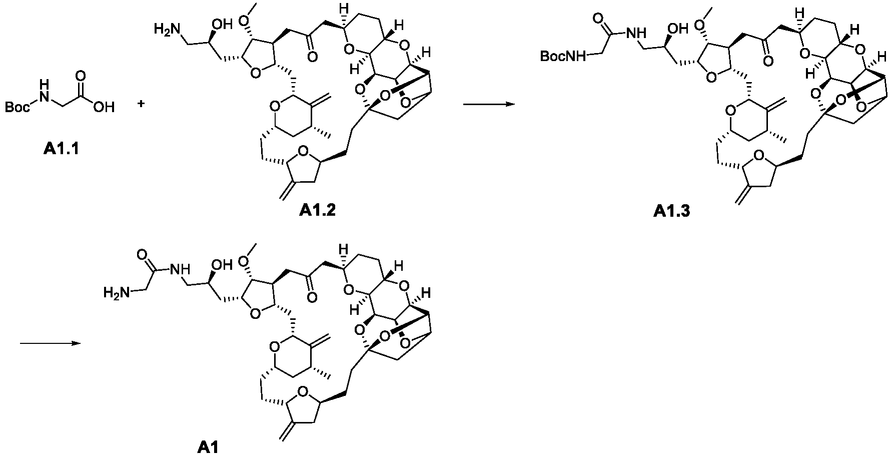

### Step 1: N-2-(tert-Butoxycarbonylamino)acetyl-eribulin

To a solution of compound (A1.1) (21 mg) in dichloromethane (1 mL) were added DIPEA (100 µL) and HBTU (45 mg), and the reaction mixture was stirred and reacted at room temperature for 20 minutes. Eribulin (A1.2, 73 mg) was then added thereto. The reaction mixture was stirred and reacted at room temperature for 10 hours, then diluted with ethyl acetate (50 mL), washed with water (20 mL × 3) and saturated sodium chloride aqueous solution (20 mL), dried over anhydrous sodium sulfate, concentrated under reduced pressure to remove the organic solvent, and separated by silica gel column chromatography (ethyl acetate: petroleum ether) to obtain compound A1.3. ESI-MS (m/z): 887.5 [M+H]⁺.

### Step 2: N-2-Aminoacetyl-eribulin

To *N-*2-(*tert*-butoxycarbonylamino)acetyl-eribulin (A1.3, 80 mg) were added dichloromethane (2 mL) and TFA (1 mL). The reaction mixture was stirred for 10 minutes, and then concentrated under reduced pressure to remove the low-boiling components. The crude product was dissolved in ethyl acetate (50 mL), added with saturated sodium bicarbonate aqueous solution (15 mL), and mixed thoroughly. The organic phase was washed with water (20 mL × 3) and saturated sodium chloride aqueous solution (20 mL), dried over anhydrous sodium sulfate, and concentrated under reduced pressure to remove the organic solvent to obtain compound N-2-aminoacetyl-eribulin (A1). ESI-MS (m/z): 787.5 [M+H]⁺.

### B. Synthesis of molecular fragment intermediates containing coupling linker segments

### Example 2 (B1): N⁶-(tert-Butoxycarbonyl)-N²-((6-(2-(methylthio)pyrimidin-5-yl)hex-5-ynoyl)-L-valyl)-L-lysine (compound B1)

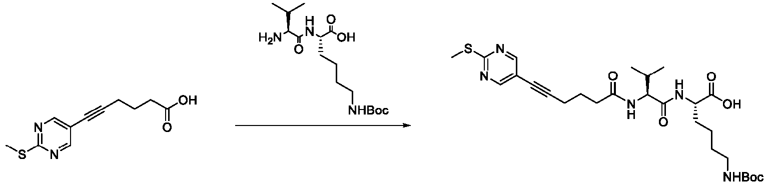

6-(2-(Methylthio)pyrimidin-5-yl)hex-5-ynoic acid (920 mg) and *N-*hydroxysuccinimide (537 mg) were dissolved in dichloromethane (50 mL), followed by addition of dicyclohexylcarbodiimide (963 mg). The reaction mixture was stirred at room temperature for 1 hour, and then concentrated under reduced pressure to remove dichloromethane. The residue was dissolved in *N,N*-dimethylformamide (50 mL), followed by addition of *N*²-*L*-valyl-*N*⁶-(Boc)-*L*-lysine (1.5 g), and the reaction mixture was stirred and reacted at room temperature for 16 hours. The reaction mixture was poured into 200 mL of water, and added with saturated sodium bicarbonate solution to adjust the pH to 11. The above aqueous solution was extracted twice with ethyl acetate, and the organic phase was discarded. The aqueous phase was added with citric acid to adjust the pH to 4 to 5, and extracted three times with ethyl acetate (100 mL × 3). The organic phase was washed with saturated brine (100 mL), dried over anhydrous sodium sulfate, filtered under reduced pressure, and subjected to rotary evaporation to obtain 2.8 g of crude product, which was purified by flash chromatography (DCM: MeOH = 30:1) to obtain the target compound *N*⁶-(*tert-*butoxycarbonyl)-*N*²-((6-(2-(methylthio)pyrimidin-5-yl)hex-5-ynoyl)-*L*-valyl)-*L*-lysine (1.0 g).

ESI-MS (m/z): 564.3 [M+H]⁺; ¹H NMR (400 MHz, DMSO-*d*₆) δ 12.45 (s, 1H), 8.68 (s, 2H), 8.12 (d, *J* = 7.3 Hz, 1H), 7.88 (d, *J* = 8.9 Hz, 1H), 6.76 (s, 1H), 4.27 - 4.19 (m, 1H), 4.11 (d, *J* = 4.9 Hz, 1H), 2.88 (d, *J* = 7.9 Hz, 3H), 2.73 (s, 1H), 2.59 (s, 1H), 2.52 (s, 4H), 2.40 - 2.24 (m, 3H), 2.03 - 1.88 (m, 2H), 1.77 (d, *J* = 3.2 Hz, 2H), 1.68 (d, *J =* 7.0 Hz, 1H), 1.60 - 1.49 (m, 1H), 1.36 (s, 15H), 0.85 (dd, *J =* 14.8, 6.7 Hz, 7H).

### Example 3 (B2): N⁶-(tert-Butoxycarbonyl)-N²-((6-(2-(methylsulfonyl)pyrimidin-5-yl)hex-5-ynoyl)-L-valyl)-L-lysine (compound B2)

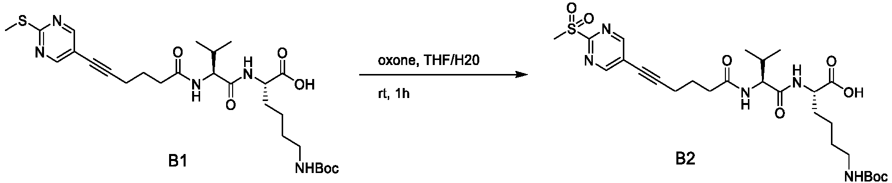

To a mixed solution of compound B1 (260 mg) in tetrahydrofuran (3 mL) and water (3 mL) was added potassium peroxymonosulfate (1.414 g), and the reaction mixture was stirred and reacted at room temperature for 1 hour. The reaction mixture was filtered, and the filtrate was purified by C18 column chromatography (acetonitrile/0.05% formic acid in water: 5% to 60%) to obtain the target compound *N*⁶-(*tert*-butoxycarbonyl)-*N*²-((6-(2-(methylsulfonyl)pyrimidin-5-yl)hex-5-ynoyl)-*L*-valyl)-*L*-lysine (compound B2) (120 mg).

LCMS (ESI) [M+H]⁺: 596; ¹H NMR (400 MHz, DMSO-*d*₆) δ 12.48 (s, 1H), 9.13 (s, 2H), 8.14 (d, *J* = 7.0 Hz, 1H), 7.90 (d, *J* = 9.1 Hz, 1H), 6.77 (s, 1H), 4.24 (t, *J* = 7.7 Hz, 1H), 4.12 (br s, 1H), 3.41 (s, 3H), 2.89 (d, *J* = 6.0 Hz, 2H), 2.43 - 2.29 (m, 2H), 2.03 - 1.93 (m, 2H), 1.82 (br s, 2H), 1.63 - 1.60 (m, 4H), 1.37 (s, 12H), 0.88 - 0.83 (m, 6H).

### Example 4 (B3): N⁶-(tert-Butoxycarbonyl)-N²-((6-(2-(methylthio)pyrimidine-5-carboxamido)hexanoyl)-L-valyl)-L-lysine (compound B3)

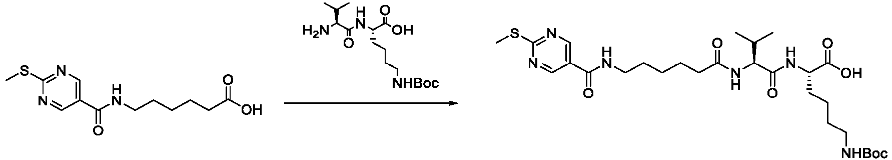

By the same method and reaction conditions as example B1, the known compounds shown in the reaction scheme as raw materials were used to obtain the target product *N*⁶-(*tert-*butoxycarbonyl)-*N*²-((6-(2-(methylthio)pyrimidine-5-carboxamido)hexanoyl)-*L*-valyl)-*L-*lysine (compound B3). ESI-MS (m/z): 611 [M+H]⁺.

### Example 5 (B4): N⁶-(tert-Butoxycarbonyl)-N²-((6-(4-(2-(methylthio)pyrimidin-5-yl)-1H-1,2,3-triazol-1-yl)hexanoyl)-L-valyl)-L-lysine (compound B4)

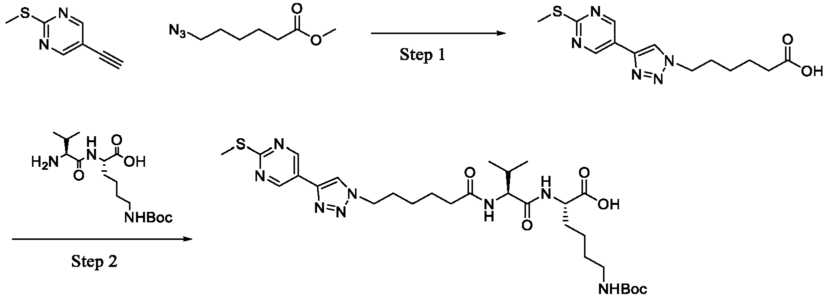

### Step 1: 6-(4-(2-(Methylthio)pyrimidin-5-yl)-1H-1,2,3-triazol-1-yl)hexanoic acid

2-Methylthio-5-ethynylpyrimidine (1.5 g) and methyl 6-azidohexanoate (1.71 g) were dissolved in a mixed solvent of *tert*-butanol and water (20 mL/25 mL) at room temperature, then sodium vitamin C (5.7 g) and cuprous bromide (2.9 g) were added thereto, and the mixture was stirred and reacted for 10 hours. The reaction mixture was added with ethyl acetate (200 mL), washed with water (100 mL × 5), dried, and the organic solvent was removed. The residue was subjected to silica gel column chromatography to obtain methyl 6-(4-(2-(methylthio)pyrimidin-5-yl)-1*H*-1,2,3-triazol-1-yl)hexanoate (2.8 g). ESI-MS (m/z): 321.9 [M+H]⁺. The above product was dissolved in tetrahydrofuran (100 mL), then lithium hydroxide (1 M, 20 mL) was added thereto, and the mixture was stirred for 3 hours. The reaction mixture was added with hydrochloric acid to adjust the pH to 2, then added with ethyl acetate (200 mL), washed with water (100 mL × 3), and dried. The organic solvent was removed to obtain the target product 6-(4-(2-(methylthio)pyrimidin-5-yl)-1*H*-1,2,3-triazol-1-yl)hexanoic acid. ESI-MS (m/z): 308.4 [M+H]⁺.

### Step 2: N⁶-(tert-Butoxycarbonyl)-N²-((6-(4-(2-(methylthio)pyrimidin-5-yl)-1H-1,2,3-triazol-1-yl)hexanoyl)-L-valyl)-L-lysine

Compound 6-(4-(2-(methylthio)pyrimidin-5-yl)-1*H*-1,2,3-triazol-1-yl)hexanoic acid (370 mg) and *N*-hydroxysuccinimide (152 mg) were dissolved in dichloromethane (5 mL) with stirring, followed by addition of dicycloethylcarbodiimide (273 mg), and the reaction mixture was stirred at room temperature for 1 hour. The reaction mixture was added with water (10 mL) and extracted with ethyl acetate (20 mL × 2). The organic phase was washed with saturated brine, dried over anhydrous sodium sulfate, filtered, and the filtrate was concentrated under reduced pressure to obtain a crude product. The crude product was dissolved in *N,N-*dimethylformamide (10 mL) with stirring, then compound *N*²-*L*-valyl-*N*⁶-(Boc)-*L*-lysine was added thereto, and the reaction mixture was reacted at room temperature for 16 hours. The reaction mixture was slowly added with citric acid to adjust the pH to approximately 5, then added with water, and extracted with ethyl acetate (10 mL × 3). The organic phase was washed with saturated brine, dried over anhydrous sodium sulfate, concentrated to remove ethyl acetate to obtain the target product *N*⁶-(*tert*-butoxycarbonyl)-*N*²-((6-(4-(2-(methylthio)pyrimidin-5-yl)-1*H*-1,2,3-triazol-1-yl)hexanoyl)-*L*-valyl)-*L*-lysine (compound B4, 400 mg).

ESI-MS (m/z): 635.3 [M+H]⁺; ¹H NMR (400 MHz, DMSO-*d*₆) δ 12.46 (s, 1H), 9.06 (s, 2H), 9.06 (s, 1H), 8.70 (s, 1H), 8.07 (d, *J =* 7.4 Hz, 1H), 7.77 (d, *J =* 9.0 Hz, 1H), 6.76 (t, *J* = 5.6 Hz, 1H), 4.41 (t, *J* = 7.0 Hz, 2H), 4.19 (dd, *J =* 8.9, 7.0 Hz, 1H), 4.14 - 4.05 (m, 1H), 2.92 - 2.85 (m, 2H), 2.56 (s, 3H), 2.24 - 2.10 (m, 2H), 1.97 - 1.81 (m, 3H), 1.71 - 1.64 (m, 1H), 1.61 - 1.50 (m, 3H), 1.36 (s, 9H), 1.28 - 1.22 (m, 6H), 0.88 - 0.76 (m, 6H).

### Example 6 (B5): N⁶-(tert-Butoxycarbonyl)-N²-((39-(2-(methylthio)pyrimidin-5-yl)-5,34-dioxo-3,9,12,15,18,21,24,27,30-nonaoxa-6,33-diazanonatriacont-38-ynoyl)-L-valyl)-L-lysine (B5)

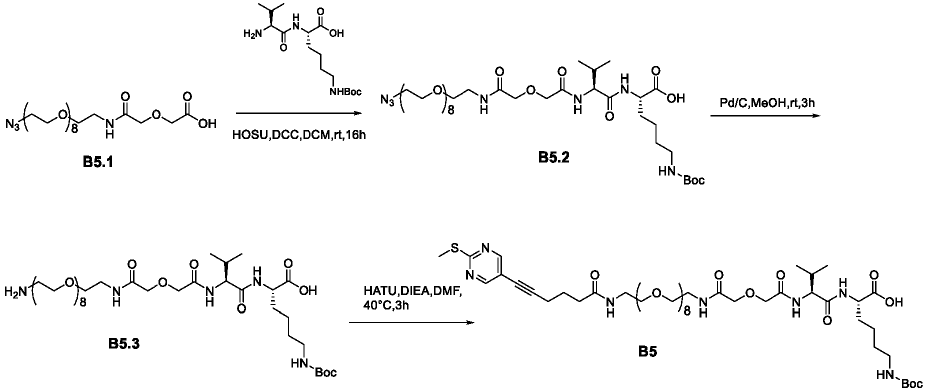

### Step 1:

Compound B5.1 (336 mg) was dissolved in dichloromethane (5 mL), then *N-*hydroxysuccinimide (98 mg, 0.85 mmol) and dicyclohexylcarbodiimide (175 mg, 0.85 mmol) were added thereto, and the reaction mixture was stirred and reacted at room temperature for 1 hour. Compound *N*²-*L*-valyl-*N*⁶-(Boc)-*L*-lysine (230 mg) was then added thereto, and the reaction mixture was stirred and reacted at room temperature overnight. The reaction mixture was concentrated to remove the solvent to obtain a crude product, which was purified by reversed-phase C18 column chromatography (acetonitrile/0.01% FA in water: 5% to 50%) to obtain the target compound (B5.2, 290 mg) as a colorless oil.

LCMS (ESI) [M+H]⁺ = 882.3.

### Step 2:

Compound B5.2 (300 mg) was dissolved in anhydrous methanol (5 mL), then Pd/C (10%, 60 mg) was added thereto, and the reaction system was replaced with hydrogen three times. The reaction mixture was stirred and reacted at room temperature overnight, filtered under reduced pressure, and concentrated to obtain the target compound B5.3 (293 mg) as a colorless oil.

LCMS (ESI) [M+H]⁺: 856.1.

### Step 3:

6-(2-(Methylthio)pyrimidin-5-yl)hex-5-ynoic acid (84 mg) was dissolved in *N,N-*dimethylformamide (3 mL), and then *N,N*-diisopropylethylamine (61 mg) and HATU (108 mg) were sequentially added thereto. The reaction mixture was stirred at room temperature for 20 minutes, then added with compound B5.3 (196 mg), and stirred and reacted at 40°C for 3 hours. The crude product was purified by reversed-phase C18 column chromatography (acetonitrile/0.01% FA in water: 5% to 65%) to obtain the target compound *N*⁶-(*tert-*butoxycarbonyl)-*N*²-((39-(2-(methylthio)pyrimidin-5-yl)-5,34-dioxo-3,9,12,15,18,21,24,27,30-nonaoxa-6,33-diazanonatriacont-38-ynoyl)-*L*-valyl)-*L*-lysine (B5) (170 mg).

LCMS (ESI) [M+H]⁺: 1074.0; ¹H NMR (400 MHz, DMSO-*d*₆) δ 8.68 (s, 2H), 8.23 (t, *J* = 5.7 Hz, 1H), 8.13 (d, *J* = 8.9 Hz, 1H), 7.99 (t, *J* = 5.6 Hz, 1H), 7.43 (d, *J* = 6.5 Hz, 1H), 6.69 (s, 1H), 4.09 (dd, *J* = 8.8, 6.5 Hz, 1H), 4.02 (s, 2H), 3.98 (d, *J* = 2.9 Hz, 2H), 3.74 - 3.70 (m, 1H), 3.50 (s, 30H), 3.45 (d, *J* = 6.2 Hz, 2H), 3.39 (d, *J* = 5.8 Hz, 2H), 3.26 (d, *J=* 6.1 Hz, 2H), 3.20 (q, *J* = 5.9 Hz, 2H), 2.81 (t, *J* = 6.6 Hz, 2H), 2.52 (s, 3H), 2.24 (t, *J* = 7.4 Hz, 2H), 2.12 - 2.06 (m, 1H), 1.79 - 1.74 (m, 2H), 1.67 - 1.59 (m, 1H), 1.50 (d, *J* = 5.3 Hz, 1H), 1.36 (s, 9H), 1.24 (s, 2H), 1.20 - 1.09 (m, 2H), 0.85 (t, *J* = 6.3 Hz, 6H).

### Example 7 (B6): N⁶,N⁶-dimethyl-N²-((6-(2-(methylthio)pyrimidin-5-yl)hex-5-ynoyl)-L-valyl)-L-lysine

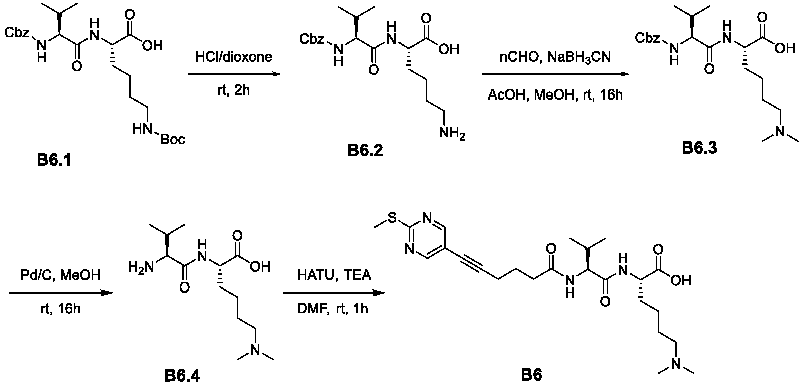

### Step 1:

To compound B6.1 (10.0 g) was added a solution of HCl-dioxane (100 mL), and the mixture was reacted at room temperature for 2 hours. The reaction mixture was concentrated under reduced pressure to obtain the target compound B6.2 (8.0 g) as a white solid.

LCMS (ESI) [M+H]⁺: 380.1. ¹H NMR (400 MHz, DMSO) δ 8.18 (d, *J* = 7.4 Hz, 1H), 7.40 - 7.30 (m, 5H), 7.26 (d, *J* = 8.8 Hz, 1H), 5.08 - 4.99 (m, 2H), 4.23 - 4.11 (m, 1H), 3.94 - 3.88 (m, 1H), 2.78 - 2.73 (m, 2H), 2.03 - 1.94 (m, 1H), 1.77 - 1.51 (m, 4H), 1.44 - 1.31 (m, 2H), 0.87 (dd, *J* = 17.3, 6.6 Hz, 6H).

### Step 2:

Compound B6.2 (3.0 g) and sodium acetate (1.90 g) were dissolved in a solution of methanol (100 mL), and the reaction mixture was reacted at room temperature for 10 minutes. Paraformaldehyde (2.8 g) was added thereto, and the reaction mixture was stirred and reacted at room temperature for 30 minutes. Sodium cyanoborohydride (1.0 g) was then added thereto, and the reaction mixture was stirred and reacted at room temperature for 16 hours. The reaction mixture was filtered, and the filtrate was purified by reversed-phase C18 column chromatography (acetonitrile/0.05% formic acid in water: 5% to 55%) to obtain the target compound B6.3 (1.70 g).

LCMS (ESI) [M+H]⁺: 408.1; ¹H NMR (400 MHz, DMSO) δ 7.86 (d, *J* = 7.3 Hz, 1H), 7.40 - 7.26 (m, 6H), 5.03 (s, 2H), 4.08 - 4.06 (m, 1H), 3.90 - 3.85 (m, 1H), 2.50 - 2.45 (m, 2H), 2.35 (s, 6H), 2.05 - 1.93 (m, 1H), 1.73 - 1.55 (m, 2H), 1.51 - 1.40 (m, 2H), 1.33 - 1.22 (m, 2H), 0.85 (dd, *J* = 16.5, 6.8 Hz, 6H).

### Step 3:

Compound B6.3 (1.6 g) was dissolved in methanol (80 mL) at room temperature, then Pd/C (10%, 0.16 g) was added thereto, and the reaction mixture was stirred and reacted at room temperature under hydrogen atmosphere for 12 hours. The reaction mixture was filtered, and the filtrate was concentrated under reduced pressure to obtain the target compound B6.4 (680 mg). LCMS (ESI) [M+H]⁺: 274.2.

### Step 4:

6-(2-(Methylthio)pyrimidin-5-yl)hex-5-ynoic acid (944 mg) was dissolved in *N,N-*dimethylformamide (30 mL), and then *N*,*N*-diisopropylethylamine (1.3 g) and 2-(7-azabenzotriazol-1-yl)-*N*,*N*,*N',N*'-tetramethyluronium hexafluorophosphate (HBTU, 1.8 g) were sequentially added thereto. The reaction mixture was stirred at room temperature for 20 minutes, then added with compound B6.4 (1.1 g), and stirred and reacted at 40°C for 3 hours. The crude product was purified by reversed-phase C18 column chromatography (acetonitrile/0.01% formic acid in water: 5% to 65%) to obtain the target compound *N⁶,N⁶*-dimethyl-*N*²-((6-(2-(methylthio)pyrimidin-5-yl)hex-5-ynoyl)-*L*-valyl)-*L*-lysine (B6) (1.2 g) as a white solid.

LCMS (ESI) [M+H]⁺: 492.1; ¹H NMR (400 MHz, DMSO) δ 8.68 (s, 2H), 8.00 (d, *J* = 7.6 Hz, 1H), 7.91 (d, *J* = 9.0 Hz, 1H), 4.20 (t, 1H), 4.08 (dd, *J* = 12.7, 7.7 Hz, 1H), 2.47 - 2.40 (m, 4H), 2.37 - 2.31 (m, 2H), 2.30 (s, 6H), 2.01 - 1.92 (m, 1H), 1.82 - 1.73 (m, 2H), 1.71 - 1.56 (m, 2H), 1.49 - 1.37 (m, 2H), 1.33 - 1.23 (m, 2H), 0.88 - 0.82 (m, 6H).

### Example 8 (B7): N⁶,N⁶-Dimethyl-N²-((6-(2-(methylsulfonyl)pyrimidin-5-yl)hex-5-ynoyl)-L-valyl)-L-lysine (B7)

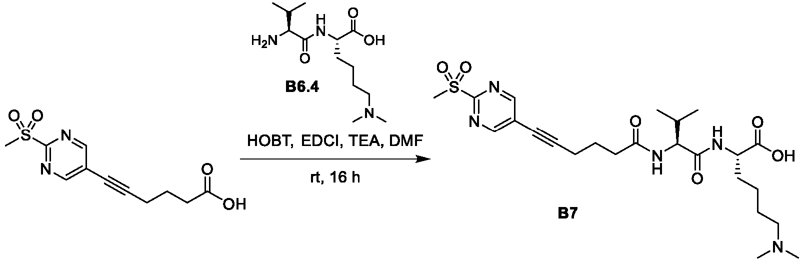

6-(2-(Methylsulfonyl)pyrimidin-5-yl)hex-5-ynoic acid (268 mg), compound B6.4 (328 mg), and triethylamine (322 mg) were dissolved in *N*,*N*-dimethylformamide (5 mL). 1-Hydroxybenzotriazole (HOBT, 162 mg) and 1-(3-dimethylaminopropyl)-3-ethylcarbodiimide hydrochloride (EDCI, 229 mg) were then added thereto, and the reaction mixture was stirred at room temperature for 16 hours. The reaction mixture was directly purified by reversed-phase C18 column chromatography (acetonitrile/0.05% formic acid in water system) to obtain the target compound *N*⁶,*N*⁶-dimethyl-*N*²-((6-(2-(methylsulfonyl)pyrimidin-5-yl)hex-5-ynoyl)-*L*-valyl)-*L*-lysine (B7, white solid, 327 mg).

LCMS (ESI) [M+H]⁺: 524.4. ¹H NMR (400 MHz, ) δ 9.13 (s, 2H), 7.95 (t, *J* = 8.8 Hz, 2H), 4.21 (dd, *J* = 8.8, 6.9 Hz, 1H), 4.08 - 4.03 (m, 1H), 3.41 (s, 3H), 2.55 (t, *J* = 7.0 Hz, 2H), 2.42 - 2.32 (m, 4H), 2.27 (s, 6H), 1.98 (dd, *J* = 13.6, 6.8 Hz, 1H), 1.86 - 1.77 (m, 2H), 1.74 - 1.55 (m, 2H), 1.47 - 1.37 (m, 2H), 1.31 - 1.23 (m, 2H), 0.85 (dd, *J=* 12.8, 6.8 Hz, 6H).

### Example 9 (B8): N²-(tert-Butoxycarbonyl)-N⁶-((6-(2-(methylsulfonyl)pyrimidin-5-yl)hex-5-ynoyl)-L-valyl)-L-lysine

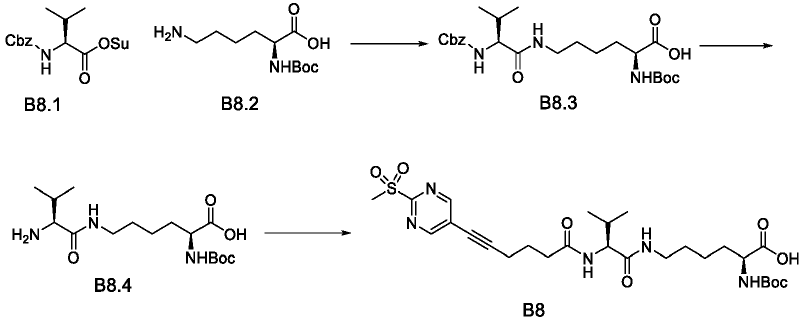

### Step 1:

Compound B8.2 (3 g) was dissolved in dichloromethane (30 mL), then DIPEA (4 mL) was added thereto, followed by addition of compound B8.1 (3.48 g). The mixture was stirred and reacted at room temperature for 20 hours. The reaction mixture was added with ethyl acetate (200 mL), sequentially washed with hydrochloric acid (0.1 M, 30 mL × 3) and water (30 mL × 3), dried over anhydrous sodium sulfate, and concentrated under reduced pressure to obtain the target mixture B8.3 (4.7 g).

### Step 2:

Compound B8.3 (4.7 g) was dissolved in methanol (80 mL) at room temperature, then Pd/C (10%, 0.6 g) was added thereto, and the reaction mixture was stirred and reacted at room temperature under hydrogen atmosphere for 12 hours. The reaction mixture was filtered, and the filtrate was concentrated under reduced pressure to obtain the target compound B8.4 (3.4 g). LCMS (ESI) [M+H]⁺: 346.2.

### Step 3:

6-(2-(Methylsulfonyl)pyrimidin-5-yl)hex-5-ynoic acid (2.68 g) was dissolved in *N,N-*dimethylformamide (50 mL), then triethylamine (3 mL) and HBTU (3.8 g) were added thereto, and the mixture was stirred at room temperature for 10 minutes. The reaction mixture was then added with ethyl acetate (200 mL), washed with saturated sodium bicarbonate (20 mL × 2), hydrochloric acid (0.1 M, 50 mL × 2), and water (50 mL × 2), dried over anhydrous sodium sulfate, and concentrated under reduced pressure to obtain an intermediate crude product. The above crude product was dissolved in DMF (30 mL), then DIPEA (1.6 mL) was added thereto, followed by addition of B8.4 (3.4 g). The reaction mixture was stirred at room temperature for 3 hours, then added with ethyl acetate (200 mL), washed with hydrochloric acid (0.1 M, 50 mL × 2) and water (50 mL × 2), dried over anhydrous sodium sulfate, and concentrated under reduced pressure to obtain a crude product, which was then purified by silica gel column chromatography to obtain the target compound *N*²-(*tert*-butoxycarbonyl)-*N*⁶-((6-(2-(methylsulfonyl)pyrimidin-5-yl)hex-5-ynoyl)-*L*-valyl)-*L*-lysine (B8, white solid, 3.8 g).

LCMS (ESI) [M+H]⁺: 596.4.

### Example 10 (B9): N⁶,N⁶-Dimethyl-N²-((6-(4-(2-(methylsulfonyl)pyrimidin-5-yl)-1H-1,2,3-triazol-1-yl)hexanoyl)-L-valyl)-L-lysine

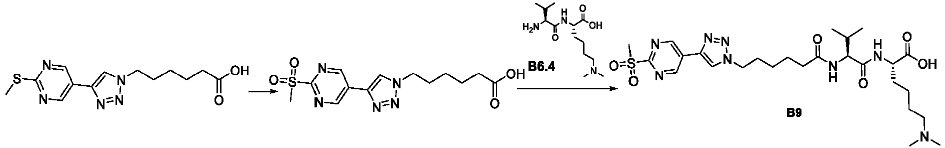

### Step 1:

Compound 6-(4-(2-(methylthio)pyrimidin-5-yl)-1*H*-1,2,3-triazol-1-yl)hexanoic acid (1.0 g, 3.3 mmol) was dissolved in a mixed solution of tetrahydrofuran and water (40 mL, 3: 1), and potassium peroxymonosulfate (10.0 g, 16.3 mmol) was added thereto. The reaction mixture was stirred and reacted at room temperature for 3 hours. The completion of the reaction was detected by LCMS. The reaction mixture was filtered, and the filter cake was washed with DMSO. The filtrates were combined and purified by reversed-phase chromatography (C18, acetonitrile: 0.1% formic acid = 5% to 55%) to obtain 6-(4-(2-(methylsulfonyl)pyrimidin-5-yl)-1*H*-1,2,3-triazol-1-yl)hexanoic acid (750 mg, yield: 67.9%) as a white solid.

LCMS (ESI) [M+H]⁺ = 340.1;

¹H NMR (400 MHz, DMSO-*d*₆) δ 9.48 (s, 2H), 8.95 (s, 1H), 4.49 (t, *J* = 7.0 Hz, 2H), 3.45 (s, 3H), 2.22 (t, *J* = 7.3 Hz, 2H), 1.95 - 1.84 (m, 2H), 1.61 - 1.50 (m, 2H), 1.36 - 1.26 (m, 2H).

### Step 2:

Compound 6-(4-(2-(methylsulfonyl)pyrimidin-5-yl)-1*H*-1,2,3-triazol-1-yl)hexanoic acid (300 mg, 0.88 mmol) was dissolved in *N*,*N*-dimethylformamide (6 mL), then HATU (337 mg, 0.88 mmol) and DIPEA (286 mg, 2.21 mmol) were added thereto, and the mixture was stirred and reacted at room temperature for 30 minutes. Dipeptide B6.4 (243 mg, 0.88 mmol) was then added thereto, and the mixture was stirred and reacted at room temperature for 2 hours. The completion of the reaction was detected by LCMS. The reaction mixture was purified by C18 column chromatography (acetonitrile/0.01% FAin water: 5% to 50%) to obtain the target compound *N*⁶,*N*⁶-dimethyl-*N*²-((6-(4-(2-(methylsulfonyl)pyrimidin-5-yl)-1*H*-1,2,3-triazol-1-yl)hexanoyl)-*L*-valyl)-*L*-lysine (300 mg, yield: 57%) as a white solid.

LCMS (ESI) [M+H]⁺ = 595.5;

¹H NMR (400 MHz, DMSO-*d*₆) δ 9.49 (s, 2H), 9.01 (s, 1H), 7.85 (br s, 1H), 7.84 (d, *J* = 8.9 Hz, 1H), 4.48 (t, *J* = 6.8 Hz, 2H), 4.17 - 4.15 (m, 1H), 4.02 (br s, 1H), 3.44 (s, 3H), 2.42 (br s, 2H), 2.30 (s, 6H), 2.18 - 2.14 (m, 2H), 1.99 - 1.96 (m, 1H), 1.88 (dd, *J* = 14.6, 7.1 Hz, 2H), 1.67 (br s, 1H), 1.59 - 1.53 (m, 2H), 1.43 (br s, 2H), 1.28 - 1.26 (m, 5H), 0.83 - 0.89 (m, 6H).

### Example 11 (B10): N⁶,N⁶-Diethyl-N²-((6-(2-(methylsulfonyl)pyrimidin-5-yl)hex-5-ynoyl)-L-valyl)-L-lysine

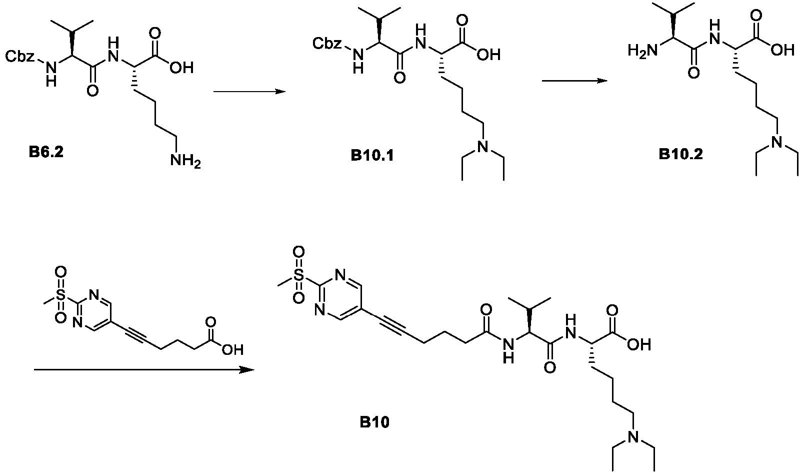

### Step 1:

Compound B6.2 (5.0 g) was dissolved in dichloromethane (100 mL), then acetaldehyde (3.2 g) was added thereto, and the reaction mixture was stirred and reacted at room temperature for 10 minutes. Sodium triacetoxyborohydride (12.8 g) was then added thereto in batches, and after the addition was completed, the reaction mixture was stirred and reacted at room temperature for 1 hour. LCMS showed the completion of the reaction. The reaction mixture was added with saturated ammonium chloride aqueous solution (20 mL), stirred for 1 hour, evaporated to dryness by rotary evaporation, filtered, and the filtrate was purified by reversed-phase C18 column chromatography (acetonitrile/0.05% formic acid in water: 5% to 55%) to obtain the target compound B10.1 (4.57 g) as a white solid.

LCMS (ESI) [M+H]⁺ = 436.4;

¹H NMR (400 MHz, DMSO-*d*₆) δ 7.70 (d, *J =* 7.0 Hz, 1H), 7.41 (d, *J =* 9.0 Hz, 1H), 7.38 - 7.26 (m, 5H), 5.08 - 4.99 (m, 2H), 4.00 (dd, *J* = 12.6, 6.5 Hz, 1H), 3.86 (dd, *J* = 8.6, 6.8 Hz, 1H), 2.74 (dd, *J =* 14.0, 6.9 Hz, 4H), 2.64 - 2.54 (m, 2H), 2.05 - 1.94 (m, 1H), 1.72 - 1.52 (m, 2H), 1.52 - 1.38 (m, 2H), 1.38 - 1.18 (m, 2H), 1.04 (t, *J* = 7.1 Hz, 6H), 0.87 - 0.81 (m, 6H).

### Step 2:

Compound B10.1 (1.6 g, 3.68 mmol) was dissolved in methanol (80 mL) at room temperature, then Pd/C (0.16 g) was added thereto, and the reaction mixture was stirred and reacted at room temperature under hydrogen atmosphere for 12 hours. LCMS showed the completion of the reaction. The reaction mixture was filtered, and the filtrate was concentrated under reduced pressure to obtain the target compound B10.2 (900 mg, yield: 82%) as an off-white solid.

¹H NMR (400 MHz, DMSO-*d*₆) δ 8.04 (br s, 1H), 4.02 - 3.99 (m, 1H), 3.10 (d, *J* = 4.5 Hz, 1H), 2.65 (q, *J* = 7.1 Hz, 4H), 2.55 - 2.51 (m, 2H), 2.06 - 1.93 (m, 1H), 1.73 - 1.54 (m, 2H), 1.47 - 1.38 (m, 2H), 1.30 - 1.21 (m, 2H), 1.01 (t, *J* = 7.1 Hz, 6H), 0.89 (d, J= 6.9 Hz, 3H), 0.79 (d, *J* = 6.8 Hz, 3H).

### Step 3:

Compound 6-(2-(methylsulfonyl)pyrimidin-5-yl)hex-5-ynoic acid (268 mg, 1 mmol) was dissolved in DMF (8 mL), then HATU (380 mg, 1 mmol) and triethylamine (322 mg, 2.5 mmol) were sequentially added thereto, and the reaction mixture was stirred at room temperature for 20 minutes. Compound B10.2 (301 mg, 1 mmol) was then added thereto, and the reaction mixture was stirred at room temperature for another 30 minutes. After the completion of the reaction was detected by LCMS, the reaction mixture was directly purified by reversed-phase C18 column chromatography (acetonitrile/0.05% formic acid in water system) to obtain the target compound *N*⁶,*N*⁶-diethyl-*N*²-((6-(2-(methylsulfonyl)pyrimidin-5-yl)hex-5-ynoyl)-*L*-valyl)-*L*-lysine (B10, 280 mg, yield: 51%) as a white solid.

LCMS (ESI) [M+H]⁺ = 552.3;

¹H NMR (400 MHz, DMSO-*d*₆) δ 9.13 (s, 2H), 7.95 (d, *J* = 8.9 Hz, 1H), 7.86 (d, *J =* 7.2 Hz, 1H), 4.18 (dd, *J* = 8.8, 6.8 Hz, 1H), 4.02 (dd, *J* = 12.8, 7.2 Hz, 1H), 3.41 (s, 3H), 2.74 - 2.69 (m, 4H), 2.62 - 2.52 (m, 4H), 2.44 - 2.29 (m, 2H), 2.04 - 1.94 (m, 1H), 1.86 - 1.77 (m, 2H), 1.72 - 1.54 (m, 2H), 1.51 - 1.39 (m, 2H), 1.33 - 1.23 (m, 2H), 1.02 (t, *J* = 7.2 Hz, 6H), 0.87 - 0.82 (m, 6H).

### Example 12 (B11): N⁶,N⁶-Diethyl-N²-((6-(4-(2-(methylsulfonyl)pyrimidin-5-yl)-1H-1,2,3-triazol-1-yl)hexanoyl)-L-valyl)-L-lysine

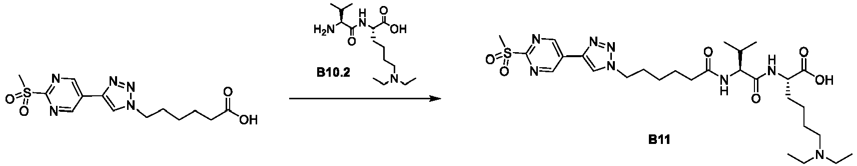

Compound 6-(4-(2-(methylsulfonyl)pyrimidin-5-yl)-1*H*-1,2,3-triazol-1-yl)hexanoic acid (500 mg, 1.475 mmol) was dissolved in *N,N*-dimethylformamide (10 mL), then HATU (560 mg, 1.475 mmol) and *N,N*-dii sopropyl ethyl amine (476 mg, 3.688 mmol) were added thereto, and the reaction mixture was stirred for 30 minutes. Compound B10.2 (444 mg, 1.475 mmol) was then added thereto, and the reaction mixture was stirred at room temperature for 1 hour. After the completion of the reaction was detected by LCMS, the reaction mixture was directly purified by reversed-phase C18 column chromatography (acetonitrile/0.05% formic acid in water system) to obtain the target compound *N*⁶,*N*⁶-diethyl-*N*²-((6-(4-(2-(methylsulfonyl)pyrimidin-5-yl)-1*H*-1,2,3-triazol-1-yl)hexanoyl)-*L*-valyl)-*L*-lysine (B11, 330 mg, yield: 34%) as a white solid.

LCMS (ESI) [M+H]⁺ = 623.4;

¹H NMR (400 MHz, DMSO) δ 9.49 (s, 2H), 9.04 (s, 1H), 7.84 (d, *J* = 8.9 Hz, 1H), 7.80 (d, *J* = 7.1 Hz, 1H), 4.47 (t, *J* = 7.0 Hz, 2H), 4.14 (dd, *J* = 8.8, 6.6 Hz, 1H), 4.06 - 3.95 (m, 1H), 3.44 (s, 3H), 2.67 - 2.64 (m, 4H), 2.55 (t, *J* = 7.4 Hz, 2H), 2.21 - 2.10 (m, 2H), 2.02 - 1.94 (m, 1H), 1.92 - 1.84 (m, 2H), 1.72 - 1.63 (m, 1H), 1.72 - 1.63 (m, 3H), 1.59 - 1.54 (m, 2H), 1.32 - 1.21 (m, 4H), 1.01 (t, *J* = 7.1 Hz, 6H), 0.81 (dd, *J* = 9.6, 6.8 Hz, 6H).

### Example 13 (B12): N⁶,N⁶-Dipropyl-N²-((6-(2-(methylsulfonyl)pyrimidin-5-yl)hex-5-ynoyl)-L-valyl)-L-lysine

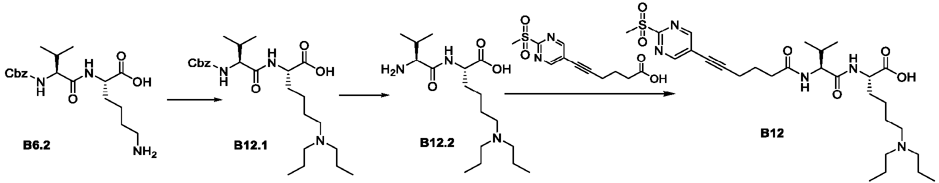

### Step 1:

Compound B6.2 (5.0 g) was dissolved in dichloromethane (100 mL), then *n-*propionaldehyde (4.2 g) was added thereto, and the reaction mixture was stirred and reacted at room temperature for 10 minutes. Sodium triacetoxyborohydride (12.8 g) was then added thereto in batches, and the reaction mixture was stirred and reacted at room temperature for 1 hour. LCMS showed the completion of the reaction. The reaction mixture was added with saturated ammonium chloride aqueous solution (20 mL), stirred for 1 hour, evaporated to dryness by rotary evaporation, filtered, and the filtrate was purified by reversed-phase C18 column chromatography (acetonitrile/0.05% formic acid in water: 5% to 55%) to obtain the target compound B12.1 (4.57 g, yield: 82.0%) as a white solid.

LCMS (ESI) [M+H]⁺ = 464.0;

¹H NMR (400 MHz, DMSO-*d*₆) δ 7.81 (d, *J =* 7.3 Hz, 1H), 7.38 - 7.28 (m, 5H), 5.04 (d, *J* = 1.7 Hz, 2H), 4.12 - 4.02 (m, 1H), 3.94 - 3.82 (m, 1H), 2.65 - 2.52 (m, 6H), 2.06 - 1.94 (m, 1H), 1.76 - 1.64 (m, 1H), 1.64 - 1.53 (m, 1H), 1.52 - 1.40 (m, 6H), 1.34 - 1.18 (m, 2H), 0.92 - 0.80 (m, 12H).

### Step 2:

Compound B12.1 (2.0 g, 4.32 mmol) was dissolved in methanol (80 mL) at room temperature, then Pd/C (0.16 g) was added thereto, and the reaction mixture was stirred and reacted at room temperature under hydrogen atmosphere for 12 hours. LCMS showed the completion of the reaction. The reaction mixture was filtered, and the filtrate was concentrated under reduced pressure to obtain the target compound B12.2 (1.2 g, yield: 85.5%) as a white solid.

### Step 3:

Compound 6-(2-(methylsulfonyl)pyrimidin-5-yl)hex-5-ynoic acid (100 mg, 0.373 mmol) was dissolved in *N*,*N*-dimethylformamide (1 mL), then HATU (142 mg, 0.373 mmol) and *N*,*N*-diisopropylethylamine (120 mg, 0.93 mmol) were added thereto, and the system was stirred for 30 minutes. Compound B12.2 (122 mg, 0.371 mmol) was then added thereto, and the reaction mixture was stirred at room temperature for 1 hour. After the completion of the reaction was detected by LCMS, the reaction mixture was directly purified by reversed-phase C18 column chromatography (acetonitrile/0.05% formic acid in water system) to obtain the target compound *N*⁶,*N*⁶-dipropyl-*N*⁶-((6-(2-(methylsulfonyl)pyrimidin-5-yl)hex-5-ynoyl)-*L-*valyl)-Z-lysine (50 mg, yield: 28%) as a light yellow solid.

LCMS (ESI) [M+H]⁺ = 580.0;

¹H NMR (400 MHz, DMSO-*d*₆) δ 8.24 (s, 2H), 7.98 - 7.93 (m, 2H), 4.24 - 4.16 (m, 1H), 4.10 (d, *J* = 5.2 Hz, 1H), 3.41 (s, 3H), 2.79 - 2.64 (m, 6H), 2.55 (t, *J* = 7.1 Hz, 2H), 2.45 - 2.26 (m, 2H), 2.06 - 1.91 (m, 1H), 1.89 - 1.78 (m, 2H), 1.76 - 1.66 (m, 1H), 1.64 - 1.57 (m, 1H), 1.57 - 1.42 (m, 6H), 1.37 - 1.24 (m, 2H), 0.93 - 0.78 (m, 12H).

### Example 14 (B13): N⁶,N⁶-Dipropyl-N²-((6-(4-(2-(methylsulfonyl)pyrimidin-5-yl)-1H-1,2,3-triazol-1-yl)hexanoyl)-L-valyl)-L-lysine

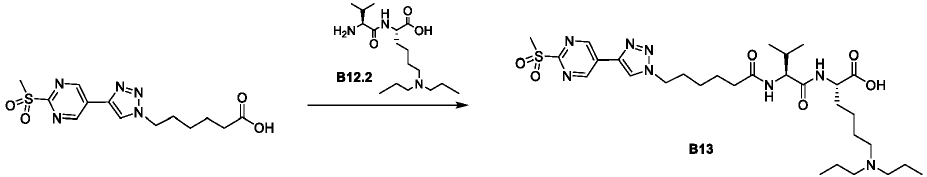

Compound 6-(4-(2-(methylsulfonyl)pyrimidin-5-yl)-1*H*-1,2,3-triazol-1-yl)hexanoic acid (500 mg, 1.475 mmol) was dissolved in *N,N*-dimethylformamide (10 mL), then HATU (560 mg, 1.475 mmol) and *N,N*-diisopropylethylamine (476 mg, 3.688 mmol) were added thereto, and the system was stirred for 30 minutes. Compound B12.2 (485 mg, 1.475 mmol) was then added thereto, and the reaction mixture was stirred at room temperature for 1 hour. After the completion of the reaction was detected by LCMS, the reaction mixture was directly purified by reversed-phase C18 column chromatography (acetonitrile/0.05% formic acid in water system) to obtain the target compound *N*⁶,*N*⁶-dipropyl-*N*²-((6-(4-(2-(methylsulfonyl)pyrimidin-5-yl)-1*H*-1,2,3-triazol-1-yl)hexanoyl)-*L*-valyl)-*L*-lysine (B13, 320 mg, yield: 33%) as a white solid.

LCMS (ESI) [M+H]⁺ = 651.5;

¹H NMR (400 MHz, DMSO) δ 9.49 (s, 2H), 9.00 (s, 1H), 7.95 (d, *J* = 7.2 Hz, 1H), 7.79 (d, *J* = 8.9 Hz, 1H), 4.47 (t, *J* = 6.9 Hz, 2H), 4.21 - 4.11 (m, 1H), 4.09 - 4.01 (m, 1H), 3.44 (s, 3H), 2.45 - 2.35 (m, 6H), 2.23 - 2.09 (m, 2H), 1.97 - 1.86 (m, 3H), 1.72 - 1.64 (m, 1H), 1.61 - 1.50 (m, 3H), 1.43 - 1.34 (m, 6H), 1.32 - 1.22 (m, 4H), 0.86 - 0.77 (m, 12H).

### Example 15 (B14): tert-Butyl (S)-(6-((4-(hydroxymethyl)phenyl)amino)-5-(6-(2-(methylthio)pyrimidin-5-yl)hex-5-ynamido)-6-oxohexyl)carbamate

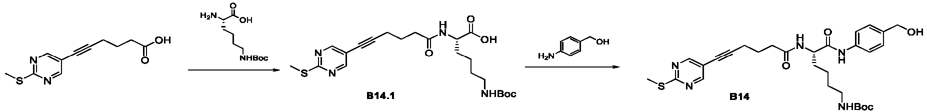

### Step 1:

To *N,N*-dimethylformamide (30 mL) were sequentially added compound 6-(2-(methylthio)pyrimidin-5-yl)hex-5-ynoic acid (3.70 g, 15.7 mmol), diisopropylethylamine (11.0 mL, 62.8 mmol), and HBTU (8.90 g, 23.6 mmol), and the reaction mixture was reacted at room temperature for 30 minutes. Compound Boc-protected lysine (3.86 g, 15.7 mmol) was then added thereto, and the reaction mixture was reacted at room temperature for 2 hours. The completion of the reaction was detected by LCMS. The reaction mixture was added with citric acid aqueous solution (30 mL) to adjust the pH to 5, and extracted with dichloromethane (50 mL × 3). The organic phases were combined, dried over anhydrous sodium sulfate, filtered under reduced pressure, and the filtrate was concentrated under reduced pressure to obtain the target compound B14.1 (7.0 g) as a yellow oil. LCMS (ESI) [M+H]⁺ = 465.1.

### Step 2:

To *N*,*N*-dimethylformamide (100 mL) were sequentially added compound B14.1 (7.00 g, 5.60 mmol), diisopropylethylamine (10.7 mL, 60.4 mmol), and HBTU (8.60 g, 22.7 mmol), followed by addition of *p*-aminobenzyl alcohol (3.71 g, 30.2 mmol), and the reaction mixture was reacted at room temperature for 2 hours. The completion of the reaction was detected by LCMS. The reaction mixture was added with ethyl acetate (300 mL), washed with saturated brine (100 mL × 3), dried over anhydrous sodium sulfate, filtered under reduced pressure, and the filtrate was concentrated under reduced pressure to obtain a solid. The crude product was purified by column chromatography (dichloromethane: methanol = 10/1) to obtain the target compound *tert*-butyl (*S*)-(6-((4-(hydroxymethyl)phenyl)amino)-5-(6-(2-(methylthio)pyrimidin-5-yl)hex-5-ynamido)-6-oxohexyl)carbamate (B14, 6.00 g, yield: 69.9%) as a yellow oil.

LCMS (ESI) [M+H]⁺ = 570.1;

¹H NMR (400 MHz, CDCl₃) δ 9.24 (s, 1H), 8.46 (s, 2H), 7.51 - 7.47 (m, 2H), 7.31 - 7.23 (m, 3H), 7.02 - 6.95 (m, 1H), 4.83 (br s, 1H), 4.63 - 4.60 (m, 2H), 3.19 - 2.96 (m, 4H), 2.57 (s, 3H), 2.52 - 2.48 (m, 2H), 2.47 - 2.41 (m, 2H), 1.99 - 1.92 (m, 2H), 1.87 - 1.77 (m, 2H), 1.76 - 1.63 (m, 2H), 1.43 (s, 9H).

### Example 16 (B15): tert-Butyl (S)-(6-((4-(hydroxymethyl)phenyl)amino)-5-(6-(2-(methylthio)pyrimidin-5-yl)-1H-1,2,3-triazol-1-yl)hexanamido)-6-oxohexyl)carbamate

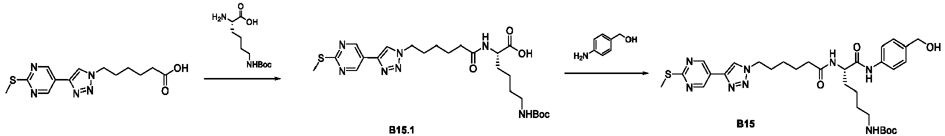

### Step 1:

To *N,N*-dimethylformamide (30 mL) were sequentially added compound 6-(4-(2-(methylsulfonyl)pyrimidin-5-yl)-1*H*-1,2,3-triazol-1-yl)hexanoic acid (1.60 g, 5.21 mmol), diisopropylethylamine (2.68 mL, 20.8 mmol), and HBTU (2.97 g, 7.82 mmol), and the reaction mixture was reacted at room temperature for 30 minutes. Boc-protected lysine (1.28 g, 5.21 mmol) was then added thereto, and the reaction mixture was reacted at room temperature for 2 hours. The completion of the reaction was detected by LCMS. The reaction mixture was added with citric acid aqueous solution (30 mL) to adjust the pH to 5, and extracted with dichloromethane (50 mL × 3). The organic phases were combined, dried over anhydrous sodium sulfate, filtered under reduced pressure, and the filtrate was concentrated under reduced pressure to obtain the target compound B15.1 (3.0 g, crude product) as a yellow oil.

LCMS (ESI) [M+H]⁺ = 536.0.

### Step 2:

To *N,N*-dimethylformamide (30 mL) were sequentially added compound B15.1 (3.00 g, 5.60 mmol), diisopropylethylamine (2.89 g, 22.4 mmol), and HBTU (3.19 g, 8.40 mmol), followed by addition of *p*-aminobenzyl alcohol (1.38 g, 11.2 mmol), and the reaction mixture was reacted at room temperature for 2 hours. The completion of the reaction was detected by LCMS. The reaction mixture was added with ethyl acetate (200 mL), washed with saturated brine (80 mL × 3), dried over anhydrous sodium sulfate, filtered under reduced pressure, and the filtrate was concentrated under reduced pressure to obtain a solid. The crude product was purified by column chromatography (dichloromethane: methanol = 10/1) to obtain the target compound *tert*-butyl (*S*)-(6-((4-(hydroxymethyl)phenyl)amino)-5-(6-(2-(methylthio)pyrimidin-5-yl)-1*H*-1,2,3-triazol-1-yl)hexanamido)-6-oxohexyl)carbamate (B15, 2.90 g) as a yellow oil.

LCMS (ESI) [M+H]⁺ = 641.1;

¹H NMR (400 MHz, CDCl₃) δ 9.13 (s, 1H), 8.95 (s, 2H), 7.94 (s, 1H), 7.47 (d, *J* = 8.4 Hz, 2H), 7.24 (d, *J* = 8.4 Hz, 2H), 6.83 - 6.77 (m, 1H), 4.93 - 4.80 (m, 1H), 4.64 - 4.58 (m, 2H), 4.40 - 4.29 (m, 2H), 3.12 - 2.97 (m, 4H), 2.60 (s, 3H), 2.31 - 2.23 (m, 2H), 1.96 - 1.83 (m, 4H), 1.76 - 1.61 (m, 4H), 1.41 (s, 9H), 1.37 - 1.29 (m, 4H).

### Example 17 (B16): N⁶,N⁶-Dibutyl-N²-((6-(2-(methylsulfonyl)pyrimidin-5-yl)hex-5-ynoyl)-L-valyl)-L-lysine

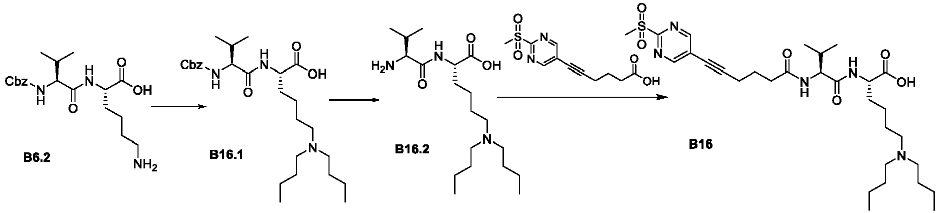

### Step 1:

Compound B6.2 (10.0 g) was dissolved in a solution of dichloromethane (200 mL), then n-butyraldehyde (10.4 g) was added thereto, and the reaction mixture was stirred and reacted at room temperature for 10 minutes. Sodium triacetoxyborohydride (25.6 g) was then added thereto in batches, and the reaction mixture was stirred and reacted at room temperature for 1 hour. LCMS showed the completion of the reaction. The reaction mixture was added with saturated ammonium chloride aqueous solution, stirred for 1 hour, evaporated to dryness by rotary evaporation, filtered, and the filtrate was purified by reversed-phase C18 column chromatography (acetonitrile/0.05% formic acid in water: 5% to 55%) to obtain the target compound B 16.1 (4.9 g) as a white solid.

LCMS (ESI) [M+H]⁺ = 492.7;

¹H NMR (400 MHz, CDCl₃) δ 7.35 - 7.30 (m, 5H), 5.14 - 5.08 (m, 2H), 4.33 - 4.30 (m, 1H), 4.14 - 4.12 (m, 1H), 2.94 - 2.80 (m, 6H), 2.13 - 2.11 (m, 1H), 1.85 - 1.82 (m, 2H), 1.58 - 1.55 (m, 4H), 1.40 - 1.22 (m, 8H), 0.98 - 0.87 (m, 12H).

### Step 2:

Compound B16.1 (5.0 g) was dissolved in a solution of methanol (100 mL) at room temperature, then Pd/C (10%, 1 g) was added thereto, and the reaction mixture was stirred and reacted under hydrogen atmosphere for 12 hours. LCMS showed the completion of the reaction. The reaction mixture was filtered, and the filtrate was concentrated under reduced pressure to obtain the target compound B16.2 (3.68 g) as a white solid.

LCMS (ESI) [M+H]⁺ = 358.3.

### Step 3:

Compound 6-(2-(methylsulfonyl)pyrimidin-5-yl)hex-5-ynoic acid (1.0 g) was dissolved in *N*,*N*-dimethylformamide (15 mL), then *N,N-*diisopropylethylamine (1.2 g) and HATU (1.4 g) were sequentially added thereto, and the reaction mixture was stirred for 30 minutes. Compound B16.2 (1.3 g) was then added thereto, and the reaction mixture was stirred at room temperature for 1 hour. After the completion of the reaction was detected by LCMS, the reaction mixture was directly purified by preparative chromatography (0.01% trifluoroacetic acid in water, acetonitrile) to obtain the target compound *N*⁶,*N*⁶-dibutyl-*N*²-((6-(2-(methylsulfonyl)pyrimidin-5-yl)hex-5-ynoyl)-*L*-valyl)-*L*-lysine (B16, 500 mg) as a yellow solid.

LCMS (ESI) [M+H]⁺ = 608.7;

¹H NMR (400 MHz, DMSO) δ 9.37 (s, 1H), 9.13 (s, 2H), 8.19 (d, *J =* 7.3 Hz, 1H), 7.92 (d, *J* = 8.8 Hz, 1H), 4.25 - 4.20 (m, 1H), 4.20 - 4.13 (m, 1H), 3.42 (s, 3H), 3.06 - 2.99 (m, 6H), 2.57 - 2.53 (m, 2H), 2.41 - 2.30 (m, 2H), 1.99 - 1.95 (m, 2H), 1.87 - 1.79 (m, 2H), 1.78 - 1.71 (m, 1H), 1.62 - 1.56 (m, 4H), 1.34 - 1.32 (m, 8H), 0.94 - 0.85 (m, 12H).

### Example 18 (B17): (S)-1-Ethyl-4-(3-methyl-2-(6-(2-(methylsulfonyl)pyrimidin-5-yl)hex-5-ynamido)butanamido)piperidine-4-carboxylic acid

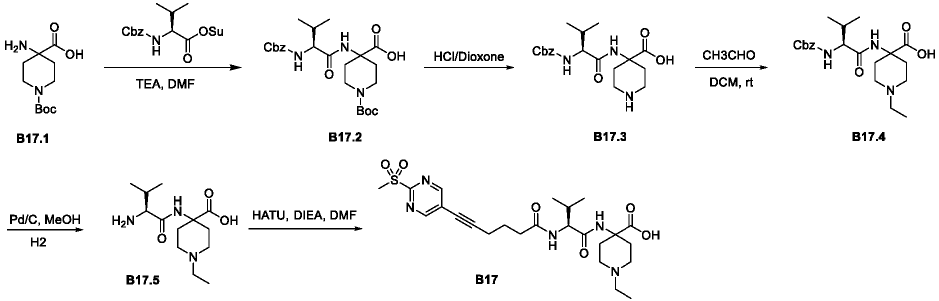

### Step 1:

Compound B17.1 (5.0 g, 20.49 mmol) was dissolved in DMF (100 mL), then *N-*((benzyloxy)carbonyl)-*L*-valinate 2,5-dioxopyrrolidine-*N*-hydroxy ester (7.13 g, 20.49 mmol) was added thereto, and the reaction mixture was stirred and reacted at 100°C for 48 hours. LCMS showed the completion of the reaction. The reaction mixture was purified by reversed-phase C18 column chromatography (acetonitrile/0.05% formic acid in water: 5% to 55%) to obtain the target compound B17.2 (1.3 g, yield: 13.3%) as a yellow solid.

LCMS (ESI) [M-boc]⁺ = 378.2;

¹H NMR (400 MHz, DMSO) δ 7.38 - 7.33 (m, 5H), 7.32 - 7.27 (m, 1H), 7.20 (d, *J* = 9.0 Hz, 1H), 5.14 - 5.02 (m, 2H), 3.97 - 3.87 (m, 1H), 3.70 - 3.59 (m, 2H), 3.08 - 2.95 (m, 2H), 2.04 - 1.97 (m, 1H), 1.95 - 1.87 (m, 2H), 1.76 - 1.69 (m, 2H), 1.40 (s, 9H), 0.91 - 0.84 (m, 6H).

### Step 2:

Compound B17.2 (1.3 g, 2.71 mmol) was dissolved in a solution of ethyl acetate (10 mL) at room temperature, then dioxane-HCl (3 N, 10 mL) was added thereto, and the reaction mixture was stirred and reacted at room temperature for 1 hour. LCMS showed the completion of the reaction. The reaction mixture was concentrated under reduced pressure to obtain the target compound B17.3 (1.06 g, yield: 94.4%) as a yellow solid.

LCMS (ESI) [M+H]⁺ = 378.4.

### Step 3:

Compound B17.3 (1.06 g, 2.81 mmol) was dissolved in dichloromethane (10 mL), then acetaldehyde (0.75 g, 16.87 mmol) was added thereto, and the reaction mixture was stirred and reacted at room temperature for 10 minutes. Sodium triacetoxyborohydride (3 g, 14.05 mmol) was then added thereto in batches, and the reaction mixture was stirred and reacted at room temperature for 1 hour. LCMS showed the completion of the reaction. The reaction mixture was added with saturated ammonium chloride aqueous solution, stirred for 1 hour, and evaporated under reduced pressure to remove dichloromethane. The residual aqueous phase was separated by reversed-phase chromatography (acetonitrile/0.05% formic acid in water: 5% to 55%) to obtain the target compound B17.4 (1.13 g, yield: 99.23%) as a white solid.

LCMS (ESI) [M+H]⁺ = 406.1;

¹H NMR (400 MHz, MeOD) δ 7.42 - 7.25 (m, 5H), 5.22 - 5.06 (m, 2H), 3.91 (d, *J =* 7.0 Hz, 1H), 3.52 - 3.36 (m, 2H), 3.25 - 2.93 (m, 4H), 2.66 - 2.54 (m, 1H), 2.47 - 2.36 (m, 1H), 2.27 - 2.14 (m, 2H), 2.13 - 2.06 (m, 1H), 1.35 - 1.29 (m, 4H), 0.98 - 0.90 (m, 6H).

### Step 4:

Compound B17.4 (1.13 g, 2.78 mmol) was dissolved in a solution of methanol (20 mL) at room temperature, then Pd/C (0.5 g) was added thereto, and the reaction mixture was stirred and reacted under hydrogen atmosphere for 12 hours. LCMS showed the completion of the reaction. The reaction mixture was filtered, and the filtrate was concentrated under reduced pressure to obtain the target compound B17.5 (0.58 g, yield: 76.18%) as a white solid. LCMS (ESI) [M+H]⁺ = 272.4.

### Step 5:

Compound 6-(2-(methylsulfonyl)pyrimidin-5-yl)hex-5-ynoic acid (1 g, 3.73 mmol) was dissolved in *N*,*N*-dimethylformamide (5 mL), then *N*,*N*-diisopropylethylamine (1.2 g, 9.32 mmol) and HATU (1.42 g, 3.73 mmol) were sequentially added thereto, and the reaction mixture was stirred for 30 minutes. Compound B17.5 (1.01 g, 3.73 mmol) was then added thereto, and the reaction mixture was stirred at room temperature for 1 hour. After the completion of the reaction was detected by LCMS, the reaction mixture was directly purified by preparative chromatography (0.01% trifluoroacetic acid in water, acetonitrile) to obtain the target compound (*S*)-1-ethyl-4-(3-methyl-2-(6-(2-(methylsulfonyl)pyrimidin-5-yl)hex-5-ynamido)butanamido)piperidine-4-carboxylic acid (B17, 500 mg, yield: 28.7%) as a yellow solid.

LCMS (ESI) [M+H]⁺ = 522.5;

¹H NMR (400 MHz, DMSO) δ 9.12 (s, 2H), 8.08 (s, 1H), 7.88 (d, *J* = 9.0 Hz, 1H), 4.28 - 4.25 (m, 1H), 3.41 (s, 3H), 2.89 - 2.78 (m, 2H), 2.57 - 2.52 (m, 4H), 2.45 - 2.30 (m, 4H), 2.07 - 2.01 (m, 2H), 1.99 - 1.96 (m, 1H), 1.95 - 1.88 (m, 2H), 1.85 - 1.79 (m, 2H), 1.04 (t, *J* = 7.1 Hz, 3H), 0.88 - 0.83 (m, 6H).

### Example 19 (B18): 4-((S)-2-((S)-2-(2,2-Dimethyl-4-(4-(2-(methylsulfonyl)pyrimidin-5-yl)-1H-1,2,3-triazol-1-yl)butanamido)-3-methylbutanamido)-6-(dimethylamino)hexanamido)benzyl (4-nitrophenyl) carbonate

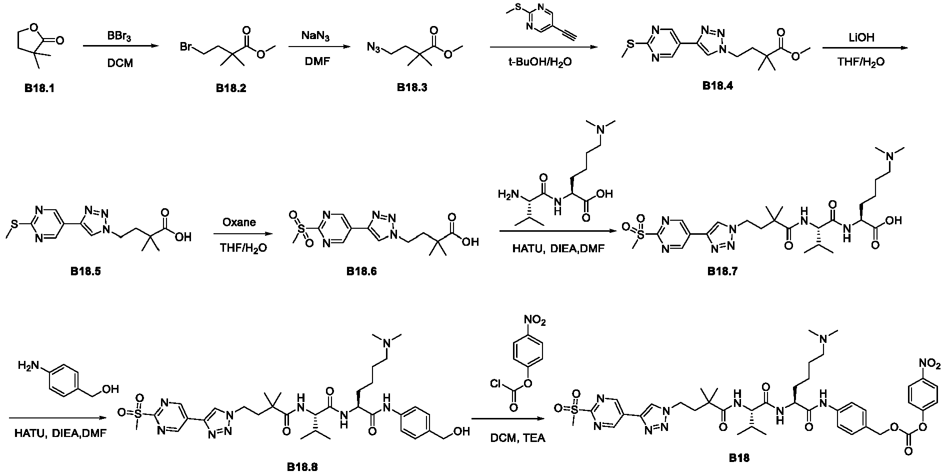

### Step 1:

Compound B18.1 (500 mg, 4.39 mmol) was dissolved in ultra-dry dichloromethane (5 mL), and boron tribromide (1.15 g, 4.6 mmol) was added dropwise thereto at 0C. After the dropwise addition was completed, the reaction mixture was warmed to room temperature and stirred for 24 hours. The reaction mixture was then cooled to 0C, added dropwise with methanol (5 mL) to quench the reaction, and stirred at room temperature for another 24 hours. The reaction mixture was added with saturated sodium bicarbonate aqueous solution (30 mL) and extracted with dichloromethane (30 mL ×3). The organic phases were combined, dried over anhydrous sodium sulfate, filtered, and evaporated to remove the solvent to obtain B18.2 (830 mg, yield: 92.5%) as a brown oily liquid.

¹H NMR (400 MHz, CDCl₃) δ 3.69 (s, 3H), 3.40 - 3.27 (m, 2H), 2.20 - 2.09 (m, 2H), 1.21 (s, 6H).

### Step 2:

Compound 18.2 (650 mg, 3.13 mmol) was dissolved in DMF (10 mL), then sodium azide (1.02 g, 15.63 mmol) was added thereto, and the reaction mixture was heated to 80C and stirred overnight. The reaction mixture was then cooled to room temperature, added with water (30 mL), and extracted with MTBE (30 mL ×3). The organic phases were combined, dried over anhydrous sodium sulfate, filtered, and evaporated to dryness by rotary evaporation to obtain the target compound B18.3 (470 mg, yield: 88.9%) as a light yellow oily liquid.

¹H NMR (400 MHz, CDCl₃) δ 3.69 (s, 3H), 3.31 - 3.23 (m, 2H), 1.89 - 1.81 (m, 2H), 1.22 (s, 6H).

### Step 3:

Compound 18.3 (365 mg, 2.13 mmol), 5-ethynyl-2-(methylthio)pyrimidine (320 mg, 2.13 mmol) were dissolved in tert-butanol (5 mL) and water (5 mL), then sodium vitamin C (84 mg, 0.43 mmol) and anhydrous copper sulfate (34 mg, 0.21 mmol) were added thereto, and the reaction mixture was stirred at room temperature for 1 hour. The reaction mixture was added with water (25 mL) and extracted with ethyl acetate (30 mL ×3). The organic phases were combined, dried over anhydrous sodium sulfate, filtered, and evaporated to dryness by rotary evaporation. The residue was purified by silica gel column chromatography (PE: EA = 3:1) to obtain the target compound B18.4 (600 mg, yield: 88.2%) as a white solid.

LCMS (ESI) [M+H]⁺ = 322.1;

¹H NMR (400 MHz, CDCl₃) δ 8.95 (s, 2H), 7.85 (s, 1H), 4.53 - 4.39 (m, 2H), 3.69 (s, 3H), 2.61 (s, 3H), 2.29 - 2.17 (m, 2H), 1.31 (s, 6H).

### Step 4:

Compound 18.4 (500 mg, 1.60 mmol) was dissolved in tetrahydrofuran (5 mL) and water (5 mL), then lithium hydroxide (112 mg, 4.70 mmol) was added thereto, and the reaction mixture was stirred at room temperature for 5 hours. The reaction mixture was added with 1 N hydrochloric acid to adjust the pH to 3 to 4, and a large amount of white solid was precipitated. After filtration, the filter cake was the target compound B18.5 (450 mg, yield: 93.5%) as a white solid.

LCMS (ESI) [M+H]⁺ = 308.0;

¹H NMR (400 MHz, DMSO) δ 12.43 (s, 1H), 9.06 (s, 2H), 8.76 (s, 1H), 4.55 - 4.31 (m, 2H), 2.56 (s, 3H), 2.15 - 2.04 (m, 2H), 1.20 (s, 6H).

### Step 5:

Compound B18.5 (1.5 g, 4.90 mmol) was dissolved in tetrahydrofuran (10 mL) and water (10 mL), then Oxane (15.0 g, 24.5 mmol) was added thereto, and the reaction mixture was stirred at room temperature overnight. The reaction mixture was poured into water (200 mL) and filtered. The filter cake was the target compound B18.6 (1.6 g, yield: 93.5%) as a white solid.

LCMS (ESI) [M+H]⁺ = 340.1;

¹H NMR (400 MHz, DMSO) δ 9.48 (s, 2H), 8.98 (s, 1H), 4.54 - 4.48 (m, 2H), 3.44 (s, 3H), 2.16 - 2.09 (m, 2H), 1.21 (s, 6H).

### Step 6:

Compound B18.6 (1.9 g, 5.32 mmol) was dissolved in DMF (40 mL), then HATU (2.13 g, 5.32 mmol) and DIEA (1.7 g, 13.3 mmol) were added thereto, and the reaction mixture was stirred at room temperature for 30 minutes. *N*²-(*L*-Valyl)-*N*⁶,*N*⁶-dimethylamino-*L*-lysine (1.45 g, 5.32 mmol) was then added thereto, and the reaction mixture was stirred for another 1 hour. The reaction mixture was directly purified by reversed-phase chromatography (H₂O: ACN = 5% to 100%) to obtain the target compound B18.7 (1.5 g, yield: 44.5%) as a white solid.

LCMS (ESI) [M+H]⁺ = 595.5;

¹H NMR (400 MHz, DMSO) δ 9.51 (s, 2H), 9.05 (s, 1H), 7.89 (d, *J* = 7.1 Hz, 1H), 7.48 (d, *J* = 8.6 Hz, 1H), 4.45 - 4.35 (m, 2H), 4.13 (t, 1H), 4.07 - 4.09 (m, 1H), 3.44 (s, 3H), 2.34 (t, *J =* 7.3 Hz, 2H), 2.23 (s, 6H), 2.19 - 2.06 (m, 4H), 1.73 - 1.57 (m, 2H), 1.45 - 1.36 (m, 2H), 1.31 - 1.27 (m, 1H), 1.22 (d, *J =* 9.6 Hz, 6H), 0.91 - 0.86 (m, 6H).

### Step 7:

Compound 18.7 (360 mg, 0.61 mmol) and (4-aminophenyl)ethanol (75 mg, 0.61 mmol) were dissolved in DMF (5 mL), then HATU (230 mg, 0.61 mmol) and DIEA (195 mg, 1.52 mmol) were added thereto, and the reaction mixture was stirred at room temperature for 1 hour. The reaction mixture was directly purified by reversed-phase chromatography (H₂O: ACN = 5% to 100%) to obtain the target compound B18.8 (255 mg, yield: 60.1%) as a white solid.

LCMS (ESI) [M+H]⁺ = 700.4;

¹H NMR (400 MHz, DMSO) δ 9.93 (d, *J* = 87.4 Hz, 1H), 9.47 (s, 1H), 9.39 (s, 1H), 8.89 (d, *J* = 57.3 Hz, 1H), 8.54 - 8.16 (m, 1H), 7.68 (d, *J* = 7.8 Hz, 0.5H), 7.56 - 7.48 (m, 2H), 7.47 (d, *J* = 8.3 Hz, 0.5H), 7.19 (d, *J* = 8.4 Hz, 1H), 7.13 (d, *J* = 8.5 Hz, 1H), 5.10 - 5.04 (m, 1H), 4.44 - 4.36 (m, 4H), 4.21 - 4.09 (m, 1H), 3.45 (s, 3H), 2.75 - 2.65 (m, 2H), 2.23 - 2.04 (m, 4H), 1.83 - 1.63 (m, 2H), 1.59 - 1.51 (m, 2H), 1.42 - 1.35 (m, 1H), 1.35 - 1.16 (m, 12H), 0.94 - 0.87 (m, 6H).

### Step 8:

Compound 18.8 (250 mg, 0.36 mmol) and 4-nitrophenyl carbonochloridate (288 mg, 1.43 mmol) were dissolved in DCM (10 mL), then TEA (72 mg, 0.72 mmol) was added thereto, and the reaction mixture was stirred at room temperature for 3 hours. The reaction mixture was concentrated, and the residue was purified by reversed-phase chromatography (H₂O: ACN = 5% to 100%) to obtain B18 (120 mg, yield: 38.9%) as a yellow solid.

LCMS (ESI) [M+H]⁺ = 865.2;

¹H NMR (400 MHz, DMSO) δ 10.04 (d, *J =* 73.3 Hz, 1H), 9.46 (s, 1H), 9.36 (s, 1H), 8.86 (d, *J* = 56.6 Hz, 1H), 8.31 (d, *J =* 9.1 Hz, 2H), 8.08 (d, *J* = 9.2 Hz, 1H), 7.64 (d, *J* = 8.0 Hz, 1H), 7.59 - 7.55 (m, 3H), 7.42 (d, *J* = 8.3 Hz, 1H), 7.37 (d, *J* = 8.5 Hz, 1H), 7.27 (d, *J* = 8.5 Hz, 1H), 6.83 (d, *J =* 9.1 Hz, 1H), 5.19 (d, *J* = 14.8 Hz, 2H), 4.45 - 4.38 (m, 2H), 4.37 - 4.28 (m, 1H), 4.21 - 4.14 (m, 1H), 3.44 (s, 3H), 2.18 - 2.12 (m, 3H), 2.10 - 2.06 (m, 8H), 1.81 - 1.63 (m, 2H), 1.44 - 1.32 (m, 4H), 1.23 (d, *J* = 7.4 Hz, 7H), 0.94 - 0.88 (m, 6H).

### Example 20 (B19): 4-((S)-2-((S)-2-(2,2-Dimethyl-4-(4-(2-(methylsulfonyl)pyrimidin-5-yl)-1H-1,2,3-triazol-1-yl)butanamido)-3-methylbutanamido)-5-ureidopentanamido)benzyl (4-nitrophenyl) carbonate

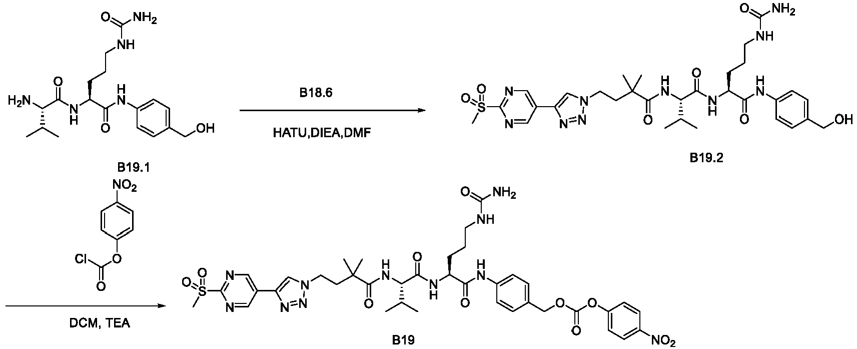

### Step 1:

Compounds 19.1 (447 mg, 1.39 mmol) and 18.6 (500 mg, 1.39 mmol) were dissolved in DMF (5 mL), then HATU (500 mg, 1.39 mmol) and DIEA (425 mg, 3.30 mmol) were added thereto, and the reaction mixture was stirred at room temperature for 1 hour. The reaction mixture was directly purified by reversed-phase chromatography (H₂O: ACN = 5% to 100%) to obtain the target compound B19.2 (900 mg, yield: 97.0%) as a white solid.

LCMS (ESI) [M+H]⁺ = 701.5;

¹H NMR (400 MHz, DMSO) δ 10.01 (s, 1H), 9.46 (s, 2H), 8.96 (s, 1H), 8.15 (d, *J* = 7.5 Hz, 1H), 7.53 - 7.50 (m, 2H), 7.47 (d, *J* = 8.6 Hz, 1H), 7.18 (d, *J* = 8.5 Hz, 2H), 6.05 (t, *J* = 5.7 Hz, 1H), 5.43 (s, 2H), 5.08 (t, *J* = 5.7 Hz, 1H), 4.45 - 4.38 (m, 5H), 4.18 (t, *J* = 8.2 Hz, 1H), 3.63 - 3.58 (m, 1H), 3.44 (s, 3H), 3.07 - 2.94 (m, 2H), 2.23 - 2.01 (m, 4H), 1.80 - 1.71 (m, 2H), 1.24 (d, *J =* 9.5 Hz, 6H), 0.92 - 0.87 (m, 6H).

### Step 2:

Compound B19.2 (400 mg, 0.57 mmol) and 4-nitrophenyl carbonochloridate (574 mg, 2.86 mmol) were dissolved in DCM (10 mL), then TEA (173 mg, 1.71 mmol) was added thereto, and the reaction mixture was stirred at room temperature for 3 hours. The reaction mixture was concentrated, and the residue was purified by reversed-phase chromatography (H₂O: ACN = 5% to 100%) to obtain the target compound B19 (150 mg, yield: 30.4%) as a yellow solid.

LCMS (ESI) [M+H]⁺ = 866.5;

¹H NMR (400 MHz, DMSO) δ 10.18 (s, 1H), 9.47 (s, 2H), 8.95 (s, 1H), 8.32 (d, *J* = 9.2 Hz, 1H), 8.18 (d, *J* = 7.2 Hz, 1H), 7.93 (d, *J* = 8.8 Hz, 1H), 7.63 - 7.56 (m, 3H), 7.55 - 7.47 (m, 1H), 7.45 (d, *J* = 8.6 Hz, 1H), 7.37 (d, *J* = 8.6 Hz, 1H), 7.19 (d, *J* = 8.7 Hz, 1H), 6.04 (t, *J* = 4.4 Hz, 1H), 5.43 (s, 2H), 5.21 (s, 1H), 4.48 - 4.37 (m, 4H), 4.22 - 4.15 (m, 1H), 3.44 (s, 3H), 3.05 - 2.96 (m, 2H), 2.20 - 2.09 (m, 3H), 2.04 - 1.98 (m, 2H), 1.51 - 1.43 (m, 2H), 1.25 (d, 6H), 0.92 - 0.87 (m, 6H).

### II. Synthesis of drug-linker compounds

### Example 21 (2.1): 4-((S)-2-((S)-2-(2,2-Dimethyl-4-(4-(2-(methylsulfonyl)pyrimidin-5-yl)-1H-1,2,3-triazol-1-yl)butanamido)-3-methylbutanamido)-5-ureidopentanamido)benzyl (eribulinyl) carbamate (DL-001)

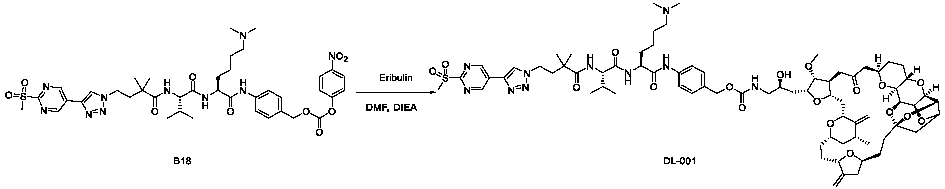

Compound B18 (36 mg, 0.041 mmol) and compound eribulin (20 mg, 0.027 mmol) were dissolved in DMF (2 mL). DIEA (7 mg, 0.054 mmol) was then added thereto, and after the addition was completed, the reaction mixture was stirred at room temperature for 1 hour. The crude product was purified by preparative chromatography (0.01% FA in water, MeCN) to obtain the target compound DL001 (21.8 mg, yield: 52.6%) as a white solid.

LCMS (ESI) [M+H]⁺ = 1455.6;

¹H NMR (400 MHz, DMSO) δ 10.01 (d, *J* = 69.6 Hz, 1H), 9.46 (s, 1H), 9.39 (s, 1H), 8.88 (d, *J* = 47.0 Hz, 1H), 8.50 (d, *J* = 7.3 Hz, 0.5H), 8.37 (s, 2H), 8.16 (d, *J* = 6.7 Hz, 0.5H), 7.67 - 7.46 (m, 3H), 7.23 (d, *J* = 8.5 Hz, 1H), 7.17 (d, *J* = 8.4 Hz, 1H), 7.07 (t, *J* = 5.4 Hz, 1H), 5.05 (s, 1H), 4.99 (s, 1H), 4.90 (d, *J* = 3.6 Hz, 1H), 4.87 (s, 1H), 4.83 (s, 1H), 4.75 (s, 1H), 4.63 (s, 1H), 4.55 (t, *J* = 4.0 Hz, 1H), 4.43 - 4.33 (m, 3H), 4.26 (d, *J* = 10.3 Hz, 1H), 4.21 - 4.14 (m, 2H), 4.11 (d, *J* = 10.6 Hz, 4H), 4.01 (d, *J* = 9.8 Hz, 1H), 3.84 - 3.73 (m, 4H), 3.72 - 3.65 (m, 3H), 3.44 (s, 3H), 3.27 - 3.22 (m, 5H), 2.96 (t, *J* = 5.6 Hz, 2H), 2.84 (d, *J* = 9.5 Hz, 1H), 2.80 - 2.64 (m, 3H), 2.60 - 2.55 (m, 1H), 2.36 - 2.29 (m, 2H), 2.27 - 2.23 (m, 2H), 2.21 - 2.11 (m, 6H), 2.08 (d, *J* = 4.8 Hz, 6H), 2.06 - 2.03 (m, 1H), 2.03 - 1.95 (m, 2H), 1.94 - 1.88 (m, 4H), 1.74 - 1.60 (m, 7H), 1.56 - 1.45 (m, 3H), 1.38 - 1.28 (m, 6H), 1.24 - 1.20 (m, 6H), 1.03 (d, *J* = 6.3 Hz, 3H), 0.97 (d, *J* = 12.0 Hz, 1H), 0.92 - 0.86 (m, 6H).

### Example 22 (2.2): 4-((S)-2-((S)-2-(2,2-Dimethyl-4-(4-(2-(methylsulfonyl)pyrimidin-5-yl)-1H-1,2,3-triazol-1-yl)butanamido)-3-methylbutanamido)-5-ureidopentanamido)benzyl (eribulinyl) carbamate (DL-002)

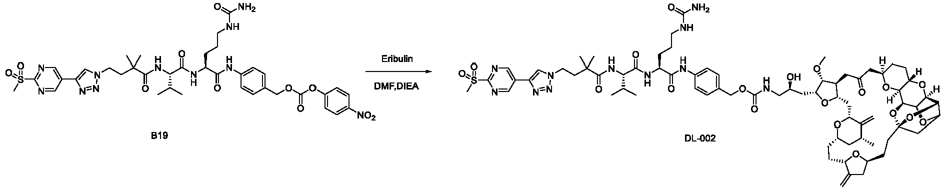

Compound B19 (48 mg, 0.055 mmol) and compound eribulin (20 mg, 0.027 mmol) were dissolved in DMF (2 mL). DIEA (8.7 mg, 0.068 mmol) was then added thereto, and after the addition was completed, the reaction mixture was stirred at room temperature for 1 hour. The crude product was purified by preparative chromatography (0.01% FA in water, MeCN) to obtain the target compound DL-002 (13.34 mg, yield: 33.6%) as a white solid.

LCMS (ESI) [M+H]⁺ = 1456.6;

¹H NMR (400 MHz, DMSO) δ 10.07 (s, 1H), 9.46 (s, 2H), 8.94 (s, 1H), 8.37 (s, 1H), 8.14 (d, *J* = 7.2 Hz, 1H), 7.55 (d, *J* = 8.3 Hz, 2H), 7.43 (d, *J* = 8.5 Hz, 1H), 7.24 (d, *J* = 8.4 Hz, 2H), 7.07 (t, *J* = 5.0 Hz, 1H), 6.00 (t, *J* = 5.3 Hz, 1H), 5.42 (s, 2H), 5.05 (s, 1H), 5.00 (s, 1H), 4.95 - 4.87 (m, 2H), 4.84 (s, 1H), 4.76 (s, 1H), 4.64 (s, 1H), 4.58 - 4.54 (m, 1H), 4.47 - 4.38 (m, 3H), 4.26 (d, *J* = 10.4 Hz, 1H), 4.21 - 4.14 (m, 2H), 4.11 (s, 3H), 4.05 - 3.98 (m, 1H), 3.84 - 3.73 (m, 2H), 3.72 - 3.65 (m, 1H), 3.57 - 3.46 (m, 4H), 3.44 (s, 3H), 3.25 (s, 4H), 3.06 - 2.91 (m, 4H), 2.84 (d, *J* = 9.6 Hz, 1H), 2.81 - 2.67 (m, 2H), 2.61 - 2.54 (m, 1H), 2.38 - 2.04 (m, 10H), 2.02 - 1.87 (m, 6H), 1.75 - 1.61 (m, 6H), 1.56 - 1.43 (m, 4H), 1.39 - 1.27 (m, 4H), 1.23 (d, *J* = 7.1 Hz, 6H), 1.18 (d, *J* = 14.7 Hz, 1H), 1.08 - 1.01 (m, 3H), 0.98 (d, *J* = 12.1 Hz, 1H), 0.93 - 0.86 (m, 6H).

### III. Preparation and identification of antibodies

### Example 23 (3.1.1): Preparation of B7H3 antigen and anti-B7H3 antibody

The nucleic acid sequence for amino acids at positions 29 to 245 (NCBI protein No.: NP_001316557.1) of human 2Ig B7H3 was selected, in which a 6× His detection tag was added to the C-terminus and named as human B7H3-2IgG-His. The nucleic acid sequence for amino acids at positions 27 to 461 (Uniport protein No. Q5ZPR3-1) of human 4Ig B7H3 was selected, in which a 6× His purification tag was added to the C-terminus and named as human B7H3-4IgG-His. The nucleic acid sequence for amino acids at positions 27 to 465 (Uniport protein No.: F7G5V3) of monkey B7H3 4Ig was selected, in which a 6× His purification tag was added to the C-terminus and named as monkey B7H3-4IgG-His. For B7H3-C1, see the antibody numbered M30-H1-L4 in CN103687945B. For B7H3-C2, see the antibody numbered mAb-C-DUBAin CN109069633A.

The genes of the above three B7H3 antigens were synthesized, and the genes of antibodies B7H3-C1 and B7H3-C2 were synthesized by codon optimization (Sangon Biotech (Shanghai) Co., Ltd.). These genes were then constructed into PTT5 expression vector, and a large number of plasmids were extracted. The extracted and prepared expression vectors were transiently transfected into and expressed in HEK293F cells for 7 days, and the expression supernatants were prepared by Protein A to obtain antigen protein, antibody B7H3-C1, and antibody B7H3-C2.

The VH amino acid sequences SEQ ID NO: 1 and SEQ ID NO: 5 of 1D1 and 2E3 were respectively added with the amino acid sequence of the constant region of IgG1 (SEQ ID NO: 20) by codon optimization and gene synthesis (Sangon Biotech (Shanghai) Co., Ltd.), and then constructed into PTT5 vector, named PTT5-01-CH and PPT5-02-CH, respectively. The VL amino acid sequences SEQ ID NO: 2 and SEQ ID NO: 6 of 1D1 and 2E3 were respectively added with the Kappa constant region sequence (SEQ ID NO: 21) by codon optimization and gene synthesis (Sangon Biotech (Shanghai) Co., Ltd.), and then constructed into PTT5 vector, named PTT5-01-CL and PTT5-01-CL, respectively. Anti-B7H3 antibody expression plasmids PTT5-01-CH/PTT5-01-CL and PTT5-02-CH/PTT5-02-CL were co-transfected into HEK293F cells by PEI max reagent and expressed in a 5% CO₂ shaker at 37°C for 7 days. Supernatants were collected and purified by ProA magnetic beads to obtain anti-B7H3 antibodies named 1D1-01 (whose heavy chain sequence is SEQ ID NO: 3, and whose light chain sequence is SEQ ID NO: 4) and 2E3-02 (whose heavy chain sequence is SEQ ID NO: 7, and whose light chain sequence is SEQ ID NO: 8), respectively.

### Example 24 (3.1.2): Protein affinity assay of anti-B7H3 antibody

The affinity of B7H3 full human antibody to human B7H3-4IgG-His and monkey B7H3-4IgG-His proteins was tested by ELISA. Specific experimental procedures: Human B7H3-4IgG-His or monkey B7H3-4IgG-His proteins were diluted to a final concentration of 1 µg/mL using carbonate buffer with a pH value of 9.6, then added to a 96-well ELISA plate at 100 µL/well, and coated overnight at 4°C. The coating solution was discarded, and the plate was washed once with PBST. 100 µL of PBS (containing 2% BSA) was added to each well, and blocked at 37°C for 1 hour. 100 µL of B7H3 full human antibody diluted with PBS (containing 2% BSA) (starting at 10 µg, 3-fold serial dilution in duplicate wells) was then added to each well, and incubated at 37°C for 2 hours. The plate was washed three times with PBST, added with horseradish peroxidase-labeled anti-human Fc antibody (diluted at 1:10000 with PBS) at 100 µL/well, and incubated at 37°C for 1 hour. The plate was washed five times with PBST. TMB chromogenic solution was added at 100 µL/well to develop color at 37°C for 7 minutes. Stop solution was added at 50 µL/well to terminate the reaction. The absorbance was measured at 450 nm. EC₅₀ values were calculated from original data.

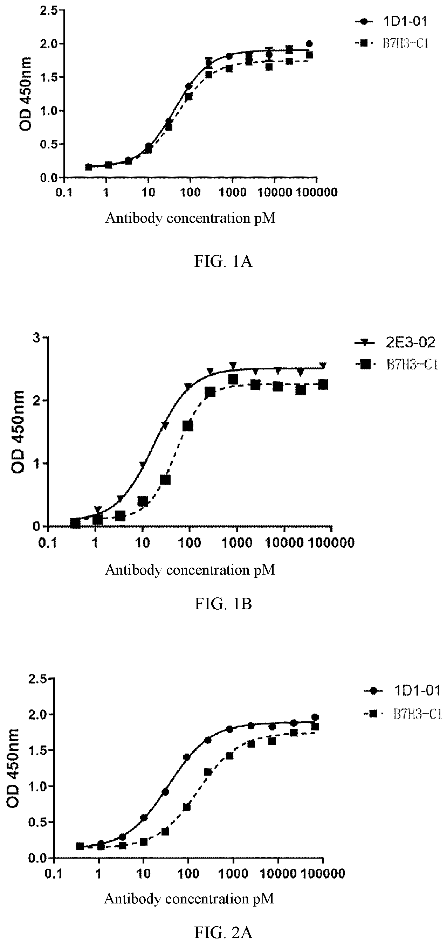

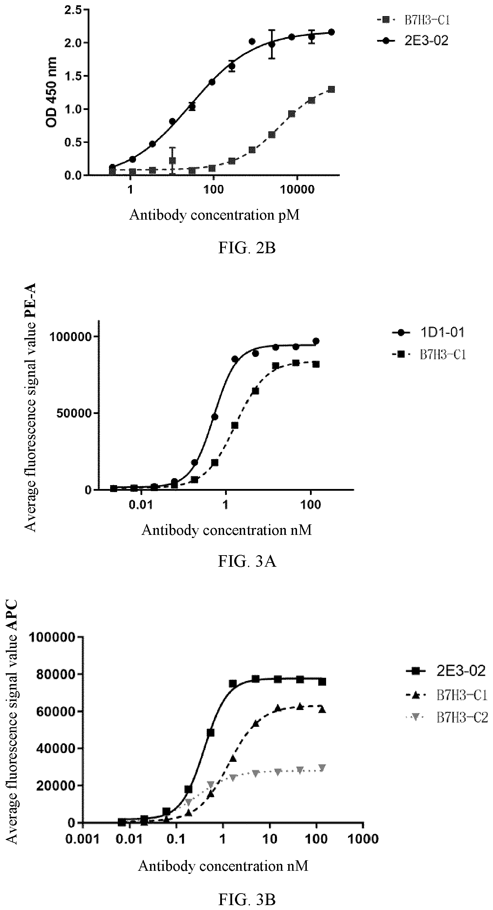

The results are shown in FIGS. 1A, 1B, 2A, and 2B, and the detailed results are shown in Tables 1-1 and 1-2. 1D1-01 and 2E3-02 both had high affinity to human B7H3-4IgG, with 43.56 pM and 17.54 pM, respectively, in which 2E3-02 had a stronger affinity than that of B7H3-C1 at 51.85 pM. 1D1-01 and 2E3-02 had high binding capacity to monkey B7H3-4IgG, with 35.82 pM and 27.72 pM, respectively.

**Table 1-1. Test results for protein affinity of antibody 1D1-01**

| Antibody | Human B7H3-4IgG-His EC₅₀ (pM) | Monkey B7H3-4IgG-His EC₅₀ (pM) |
|---|---|---|
| 1D1-01 | 43.56 | 35.82 |
| B7H3-C1 | 48.03 | 166.52 |

**Table 1-2. Test results for protein affinity of antibody 2E3-02**

| Antibody | Human B7H3-4IgG-His EC₅₀ (pM) | Monkey B7H3-4IgG-His EC₅₀ (pM) |
|---|---|---|
| 2E3-02 | 17.54 | 27.72 |
| B7H3-C1 | 51.85 | 3921 |

### Example 25 (3.1.4): Cell affinity assay of anti-B7H3 antibody

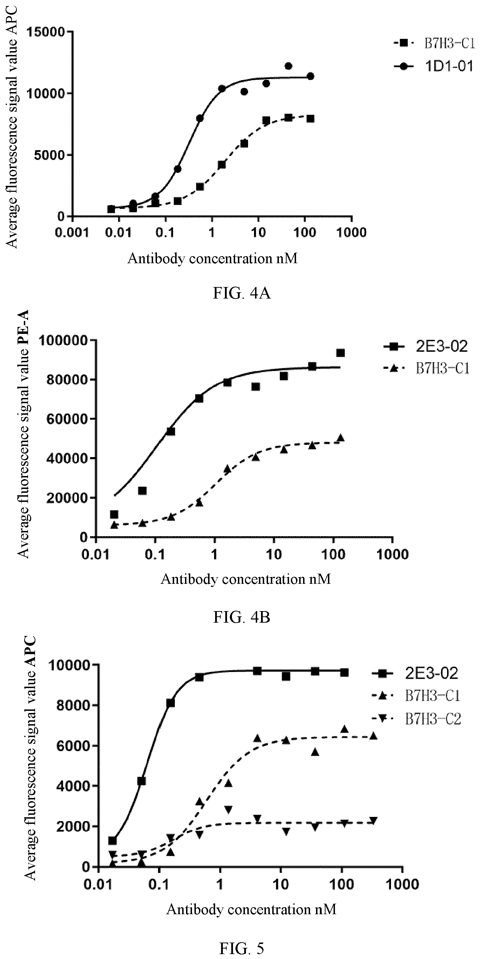

B7H3 protein is highly expressed in a variety of solid tumor cells. The affinity of B7H3 full human antibody to tumor cells was tested by FACS. Specific experimental procedures: MCF-7 breast cancer cells, A549 human non-small cell lung cancer cells, and PC3 human prostate cancer cells grown to logarithmic phase were digested by trypsin, and resuspended to 5 * 10⁵ cells/mL in PBS buffer containing 2% BSA. 100 µL of cell suspension was added to a 96-well plate. B7H3-C1, B7H3-C2, 1D1-01, and 2E3-02 were diluted in PBS buffer containing 2% BSA, with 3-fold serial dilution at an initial concentration of 40 µg/mL. 100 µL of antibody suspension was then added into the 96-well plate and mixed homogeneously. The plate was incubated at 4°C for 1 hour, washed twice with pre-cooled PBS at 300 µL/well each time, and centrifuged at 500 g for 5 minutes. 1:50 fluorescent secondary antibody APC anti-human IgG or PE anti-human IgG (purchased from Biolegend) was added at 100 µL/well and mixed homogeneously. The plate was incubated at 4°C for 0.5 hours, washed twice with pre-cooled PBS at 300 µL/well each time, centrifuged at 500 g for 5 minutes, and resuspended in 500 µL of PBS. The average fluorescence signal value was measured by Beckman flow cytometer. The EC₅₀ value was calculated from the average fluorescence signal value. The results are shown in FIGS. 3A, 3B, 4A, 4B, and 5, and the specific data are shown in Tables 2-1 and 2-2.

**Table 2-1. Test results for cell affinity of antibody 1D1-01**

| Antibody | MCF-7 tumor cells | | A549 tumor cells | |
|---|---|---|---|---|
| | EC₅₀ (nM) | Maximum MFI | EC₅₀ (nM) | Maximum MFI |
| 1D1-01 | 0.524 | 94465 | 0.322 | 11288 |
| B7H3-C1 | 1.709 | 83992 | 1.865 | 8273 |

**Table 2-2. Test results for cell affinity of antibody 2E3-02**

| Antibody | MCF-7 tumor cells | | A549 tumor cells | | PC-3 tumor cells | |
|---|---|---|---|---|---|---|
| | EC₅₀ (nM) | Maximum MFI | EC₅₀ (nM) | Maximum MFI | EC₅₀ (nM) | Maximum MFI |
| 2E3-02 | 0.398 | 77732 | 0.150 | 84806 | 0.063 | 9729 |
| B7H3-C1 | 1.360 | 63194 | 1.054 | 47956 | 0.618 | 6436 |
| B7H3-C2 | 0.278 | 27925 | Not detected | Not detected | 0.167 | 2170 |

As shown in Tables 2-1 and 2-2, for high B7H3-expressing MCF-7 tumor cells, the affinities of 1D1-01 and 2E3-02 were 0.524 nM and 0.398 nM, respectively, both of which were much higher than that of B7H3-C1, while the maximum fluorescence signal values were 12% and 23% higher than that of B7H3-C1, respectively, in which the maximum fluorescence signal value of 2E3-02 was 2.78 times higher than that of B7H3-C2. For middle B7H3-expressing A549 tumor cells, the affinities of 1D1-01 and 2E3-02 were 0.322 nM and 0.150 nM, respectively, both of which were much higher than that of B7H3-C1, while the maximum fluorescence signal values were 36% and 77% higher than that of B7H3-C1, respectively. For low B7H3-expressing PC-3 tumor cells, the affinity of 2E3-02 at 0.06 nM was much higher than that of B7H3-C1 at 0.618 nM, and also higher than that of B7H3-C2 at 0.167 nM, while the maximum fluorescence signal values were 1.51 times and 4.48 times higher than those of B7H3-C1 and B7H3-C2, respectively. In conclusion, anti-B7H3 antibodies 1D1-01 and 2E3-02 have better affinity to tumor cells than that of B7H3-C1 and B7H3-C2.

### Example 26 (3.1.5): Endocytic activity assay of anti-B7H3 antibody

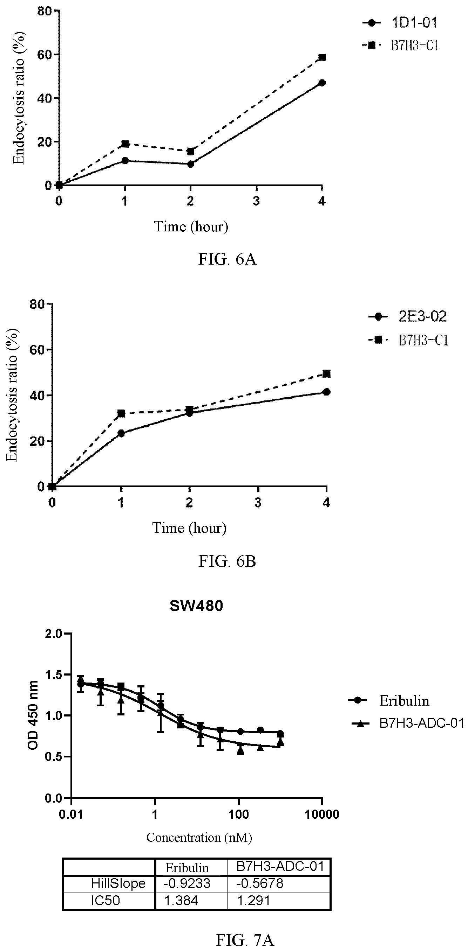

The endocytosis of anti-B7H3 antibody by A549 human non-small cell lung cancer cells was tested by FACS. A549 cells grown to logarithmic growth phase were digested by trypsin, collected by centrifugation, and washed three times with pre-cooled PBS. The cells were resuspended in PBS (containing 1% BSA), respectively added with anti-B7H3 antibody to be tested at a concentration of 10 µg/mL, and incubated at 4°C for 1 hour. The cells were collected by centrifugation, washed three times with PBS, resuspended in DMEM + 10% FBS, divided into 4 parts, and incubated at 37°C for 0, 1, 2, and 4 hours, respectively. After completion of the incubation phase, the cells were collected by centrifugation, washed three times with pre-cooled PBS, and resuspended in 50 µL of 1% BSA (in PBS). 1 µL of fluorescent secondary antibody APC anti-human IgG (Biolegend) was then added to each well, mixed homogeneously, and incubated at 4°C for 0.5 hours. The cells were collected by centrifugation, washed three times with pre-cooled PBS, resuspended in 200 µL of PBS, and tested on the apparatus. According to the formula: endocytosis ratio (%) = [1 - (average fluorescence value of the test sample at this time point - average fluorescence value of the negative control sample at this time point) / (average fluorescence value of the test sample at 0 hours - average fluorescence value of the negative control sample at 0 hours)] * 100. The results of 1D1-01 and 2E3-02 are shown in FIGS. 6A and 6B, respectively. Anti-B7H3 antibodies 1D1-01 and 2E3-02 have strong endocytic activity for A549 tumor cells, reaching approximately 40% at 4 hours, which is comparable to antibody B7H3-C1.

### IV. Coupling of compounds containing cellular bioactive molecules and linkers to antibodies

### Example 27 (4.1): Preparation of B7H3-ADC-01

0.3 mL of B7H3 antibody (2E3-02, 17 mg/mL) was taken and diluted with 0.13 mL of 20 mM PB + 150 mM NaCl + 20 mM sodium edetate solution (pH 7.6). The mixture was then added with 0.23 mL of 20 mM PB + 150 mM NaCl solution (pH 7.6), mixed homogeneously, and added with 1 M K₂HPO₄ solution to adjust the pH to 7.4. The mixture was then added with 10 mM TCEP (tris(2-carboxyethyl)phosphine, 0.017 mL, 0.17 µmol) solution, mixed homogeneously, and left at room temperature for 30 minutes. The above mixture system was added with a solution of DL001 (1.0 mg, 20-fold molar amount of antibody substance) in dimethyl sulfoxide (0.1 mL), mixed homogeneously, and allowed to stand at room temperature for 2 hours. Upon completion, 6.1 µL of 100 mM cysteine was added to terminate the reaction. Finally, the buffer was replaced with 20 mM PB buffer (pH 6.4) using a G-25 gel column. The coupling product of DL-001 and B7H3 antibody, B7H3-ADC-01 (DAR8) was obtained. The DAR value was measured to be 8.0 by mass spectrometry.

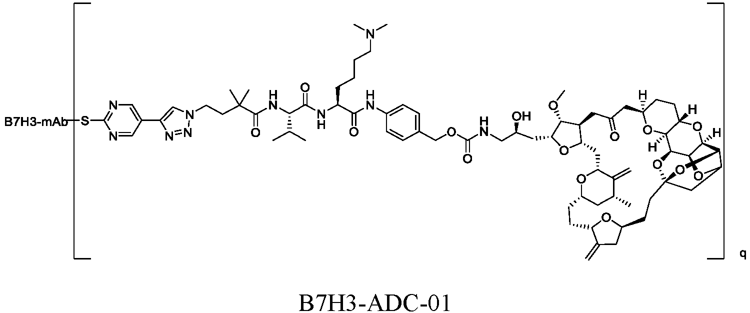

The method for determining DAR values by mass spectrometry is as follows:
Chromatographic conditions:
Chromatographic column: PLRP-S, 2.1 * 50 mm, 5 µm;
mobile phase A: 0.1% FA/H₂O; mobile phase B: 0.1% FA/ACN;
column temperature: 30°C; sample chamber temperature: 8°C; flow rate: 0.6 mL/min; injection volume: 2 µL.

| | | | | | |
|---|---|---|---|---|---|
| Time (min) | 1 | 5 | 5.1 | 7 | 10 |
| Mobile phase A | 90 | 40 | 10 | 90 | 90 |
| Mobile phase B | 10 | 60 | 90 | 10 | 10 |

Sample treatment: 50 µg of sample ADC-01 was taken and added with 2 µL of 1 M DTT. The mixture was diluted to a concentration of approximately 1.0 mg/mL by adding ultrapure water to 50 µL, mixed homogeneously, and reduced at room temperature for 30 minutes.

LC/MS model: Agilent 1290-6545XT Q-TOF.

Mass spectrometry conditions: Gas temp: 320°C, drying gas: nebulizer: 35 psi; sheath gas temp: 350°C; sheath gas flow: 11 L/min; m/z 500 to 3000.

| B7H3-ADC-01 | | | | | |
|---|---|---|---|---|---|
| Peptide chain | Mass measured value | Area | Total | Ratio | DAR |
| LC + DAR1 | 24604.2826 | 6848 | 117817 | 1.0 | 8.0 |
| | 24621.89724 | 101572 | | | |
| | 24643.84308 | 9397 | | | |
| HC + DAR3 | 54498.75265 | 37537 | 46416 | 1.0 | |

In the table, LC represents a light chain of an antibody; HC represents a heavy chain of an antibody; DAR1 represents a conjugate containing a light or heavy chain conjugated with one payload molecule; DAR2 represents a conjugate containing a light or heavy chain conjugated with two payload molecules; DAR3 represents a conjugate containing a light or heavy chain conjugated with three payload molecules. Hereinafter, LC, HC, DAR1, DAR2, and DAR3 are as described above.

It was determined that the light chain of antibody 2E3-02 was conjugated with one payload molecule (DAR1 percentage of 100.0%), while the heavy chain was conjugated with three payload molecules (DAR3 percentage of 100%), and thus the drug-to-antibody coupling ratio (DAR value) of B7H3-ADC-01 was calculated to be 8.0. According to the fact that the light chain was conjugated with one payload molecule (DAR1 percentage of 100%) and the heavy chain was conjugated with three payload molecules (DAR3 percentage of 100%), q is 8.

### Example 28 (4.2): Preparation of B7H3-ADC-02

Using the same procedure as for B7H3-ADC-01 and replacing DL-001 with DL-002, the coupling product of DL-002 and B7H3 antibody 2E3-02, B7H3-ADC-02 (DAR8) was obtained. The DAR value was measured to be 8.0 by mass spectrometry.

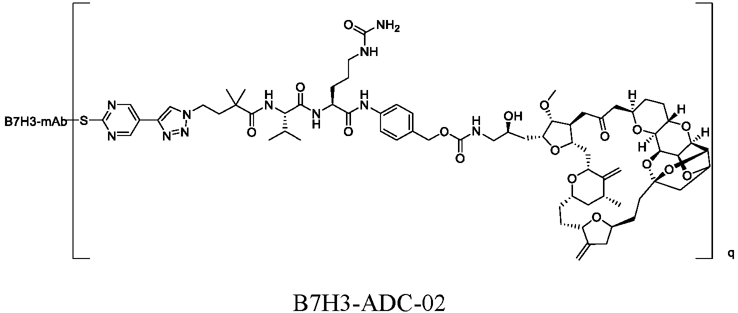

### Example 29 (4.3): Preparation of B7H3-ADC-03

Using the same procedure as for B7H3-ADC-01 and reducing the feeding amount of DL-001 from 20-fold molar amount of antibody substance to 4.4-fold molar amount of antibody substance, the coupling product of DL-001 and B7H3 antibody 2E3-02, B7H3-ADC-03 (DAR4.2) was obtained. Using the same method as in Example 4.1, the DAR value was measured to be 4.2.

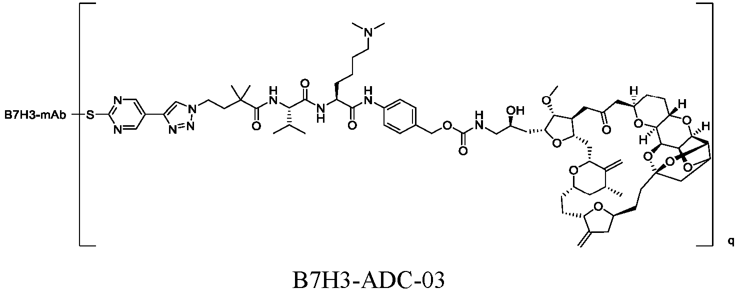

| B7H3-ADC-03 | | | | | |
|---|---|---|---|---|---|
| Peptide chain | Mass measured value | Area | Total | Ratio | DAR |
| LC | 23246.2348 | 75343 | 75343 | 0.76 | 4.2 |
| LC + DAR1 | 24622.3496 | 23460 | 23460 | 0.24 | |
| HC | 50371.0644 | 4819 | 4819 | 0.12 | |
| HC + DAR1 | 51747.0951 | 8429 | 8429 | 0.21 | |
| HC + DAR2 | 53123.0816 | 14253 | 14253 | 0.36 | |
| HC + DAR3 | 54498.8129 | 12080 | 12080 | 0.31 | |

It was determined that the light chain of antibody 2E3-02 was conjugated with 0 to 1 payload molecule (LC and DAR1 percentages of 76.0% and 24.0%, respectively), while the heavy chain was conjugated with 0 to 3 payload molecules (HC, DAR1, DAR2, and DAR3 percentages of 12.0%, 21.0%, 36.0%, and 31.0%, respectively), and thus the drug-to-antibody coupling ratio (DAR value) of B7H3-ADC-03 was calculated to be 4.2. According to the distribution of conjugated payload molecules on the heavy and light chains, q can be 1, 2, 3, 4, 5, 6, 7, or 8.

### Example 30 (4.4): Preparation of Her2-ADC-04

Using the same procedure as for B7H3-ADC-01, replacing the antibody with trastuzumab, and replacing DL-001 with DL-002, the coupling product of DL-002 and trastuzumab, Her2-ADC-04 was obtained. Using the same method as in Example 4.1, the DAR value was measured to be 7.9.

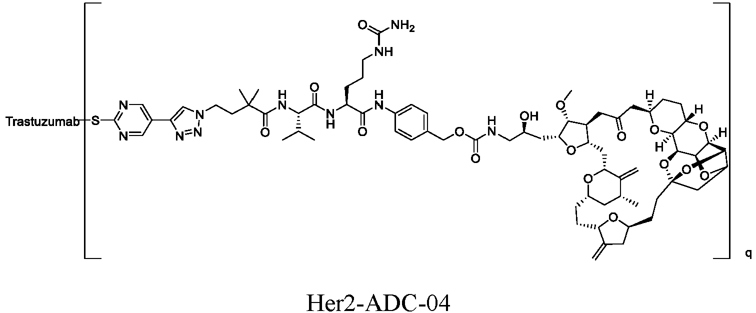

| Her2-ADC-04 | | | | | |
|---|---|---|---|---|---|
| Peptide chain | Theoretical value | Measured value | Area | Ratio | DAR |
| LC | 23438.00 | 23438.74 | 3813.493 | 0.03 | 7.9 |
| LC + DAR1 | 24813.70 | 24815.12 | 250374.9 | 0.97 | |
| HC | 50594.00 | N/A | N/A | N/A | |
| HC + DAR1 | 51969.70 | N/A | N/A | N/A | |
| HC + DAR2 | 53345.40 | 53347.68 | 4470.638 | 0.06 | |
| HC + DAR3 | 54721.10 | 54723.9 | 115972.8 | 0.90 | |
| HC + DAR4 | 56096.80 | 56099.37 | 3192.629 | 0.04 | |

It was determined that the light chain of antibody trastuzumab was conjugated with 0 to 1 payload molecule (LC and DAR1 percentages of 3% and 97%, respectively), while the heavy chain was conjugated with 0 to 4 payload molecules (HC, DAR1, DAR2, DAR3, and DAR4 percentages of 0%, 0%, 6%, 90%, and 4%, respectively), and thus the drug-to-antibody coupling ratio (DAR value) of Her2-ADC-04 was calculated to be 7.9. According to the distribution of conjugated payload molecules on the light and heavy chains, q can be 2, 3, 4, 5, 6, 7, 8, 9, or 10.

### Example 31 (4.5): Preparation of reference ADC-01

Reference DL was prepared according to the literature (WO2017151979), and the structure is shown below.

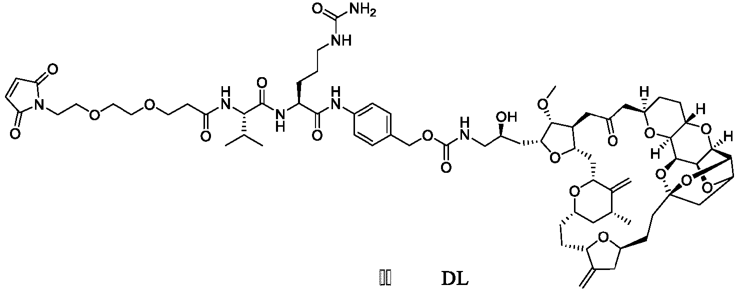

Using the same procedure as in example 4.4 and replacing DL-002 with reference DL, reference ADC-01 was obtained, as shown below. The DAR value was measured to be 7.4.

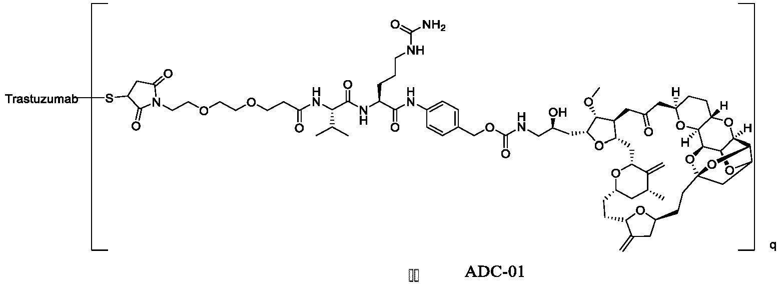

### V. Inhibitory activity assay of ADC on cell viability in vitro

### Example 32 (5.1): Inhibitory activity assay of ADC on cell viability in vitro

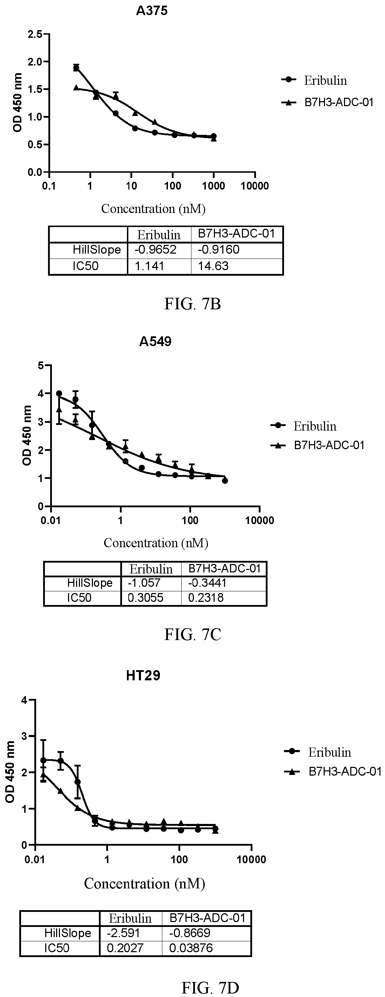

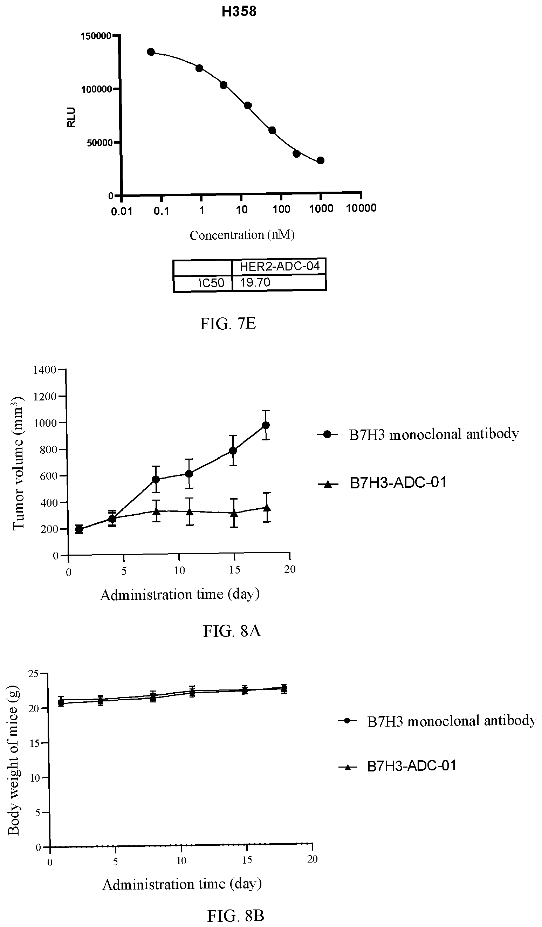

HT29, NCI-H358 (HT29 and NCI-H358 are B7H3 positive tumor cells), SW480, A375, A549, and Calu-3 (Calu-3 is B7H3 negative tumor cells) tumor cells were digested by trypsin using conventional methods. Cells in the tube were collected and counted, then resuspended in the corresponding assay medium (containing 2% FBS), and added to a 96-well plate at 2000 to 5000 cells/well. 100 µL of ADC diluted in 2% FBS medium was added to the 96-well plate, with 3-fold serial dilution at an initial concentration of 150 µg/mL (12 concentration gradients). The plate was incubated at 37°C under 5% CO₂ for 7 days. 20 µL of CCK8 reagent was then added to each well, and the reaction was carried out for 2 to 6 hours. The plate was read on a microplate reader (detection wavelength: 450 nm). The test results are shown in Table 3-1, Table 3-2, and FIGS. 7A to 7E.

The test results show that the ADC molecules of the present disclosure have a killing effect on tumor cells. Meanwhile, the ADC molecules of the present disclosure were tested against B7H3 negative cells Calu-3, and the results showed that the killing effect was weak (IC₅₀ > 10⁷ ng/mL), indicating that for B7H3 positive and negative tumor cells, the ADC molecules of the present disclosure have an excellent selective targeting killing effect *in vitro.*

**Table 3-1. Inhibitory activity assay of B7H3-ADC-01 on cells**

| Drug name | SW480 (IC₅₀, nM) | A375 (IC₅₀, nM) | A549 (IC₅₀, nM) | HT29 (IC₅₀, nM) |
|---|---|---|---|---|
| Eribulin | 1.38 | 1.14 | 0.31 | 0.20 |
| B7H3-ADC-01 | 1.29 | 14.63 | 0.23 | 0.039 |

**Table 3-2. Inhibitory activity assay of Her2-ADC-04 on cells**

| Drug name | H358 (IC₅₀, nM) |
|---|---|
| Her2-ADC-04 | 19.70 |

### Example 33 (5.2): Efficacy assay of ADC on NCI-HT29 xenograft

### 1. Experimental materials

### Test compound: B7H3-ADC-01

### Control compound: B7H3 monoclonal antibody (2E3-02)

Experimental cells: NCI-HT29 cells, purchased from ATCC;
experimental animals: Balb/c nu nude mice, female, 5 to 6 weeks old, purchased from Vital River Laboratory Animal Technology Co., Ltd.

### 2. Experimental scheme

### 2.1. Cell treatment

NCI-HT29 cells were cultured in a 15 cm diameter petri dish with 1640 culture medium containing 10% FBS, digested by trypsin-EDTA when the confluency reached about 80 to 90%, washed twice with PBS, and then centrifuged. The cells were resuspended in pre-cooled PBS, counted by a cell counter, and diluted with PBS to a cell concentration of 5 × 10⁷ /mL.

### 2.2. Tumor cell transplantation

After adapting to the laboratory environment for 2 to 5 days, Balb/c nu mice were subcutaneously inoculated with NCI-HT29 cells in the right rib, with an inoculation amount of 5 × 10⁶ cells/mouse and an inoculation volume of 0.2 mL (containing 50% Matrigel). The experiment was carried out when the tumor grew to about 250 mm³.

### 2.3. Administration of animals and detection

The grouped tumor-bearing nude mice were administered according to the following regimen:

| No. | Group | Number of animals | Dose | Route and cycle of administration |
|---|---|---|---|---|
| 1 | B7H3 monoclonal antibody (2E3-02) | 5 | 3 mg/kg | i.v., QW × 3 |
| 2 | B7H3-ADC-01 | 5 | 3 mg/kg | i.v., QW × 3 |

After the end of the administration cycle, the mice were continually observed for 1 to 2 weeks. After the last measurement of tumor volume, the tumor was isolated, accurately weighed, and photographed for recording.

### 2.4. Determination of tumor volume and body weight:

Tumor volume and body weight were measured on days 1, 4, 8, 11, 15, and 18 of the experiment, and TGI% was calculated.

Tumor volume (V) was calculated according to the formula: V = 1/2 × L_{long} × Lₛₕₒᵣₜ².

TGI was calculated according to the formula: TGI (%) = (1 - T/C) × 100% (T and C are the relative tumor volume at a specific time point in the treatment group and control group, respectively).

### 3. Experimental results

**Table 3-3. NCI-HT29 xenograft model data (tumor volume mm³)**

| Group | Average tumor volume (mm³) D1 | Average tumor volume (mm³) D18 | TGI (%) D18 |
|---|---|---|---|
| B7H3 monoclonal antibody (2E3-02) | 195.4 | 1040 | / |
| B7H3-ADC-01 | 195.6 | 341.4 | 67.17 |

The test results show that the ADC of the present disclosure has a very strong tumor inhibitory effect. There was no significant weight loss and no significant drug toxicity in each group of animals during the administration period. The test results are shown in Table 3-3 and FIGS. 8A to 8B.

### Example 34 (5.3): ADC plasma stability assay

Preparation of solution: The ADC stock solution to be tested was diluted with PBS into a working solution with a concentration of 2 mg/mL.

Incubation of sample: B7H3-ADC-01, B7H3-ADC-03, Her2-ADC-04, reference ADC-01, and human plasma were all added with the bacteriostatic agent ProClin with a final concentration of 0.1%, which was operated in a sterile environment. B7H3-ADC-01, B7H3-ADC-03, Her2-ADC-04, and reference ADC-01 were added to the above sterile plasma at a final concentration of 200 µg/mL, respectively, and incubated in an incubator at 37°C with a shaking speed of 80 rpm. After incubation for 0 and 14 days, ADC samples were removed and 100 µL of Protein A was added to each tube. The sample was adsorbed for 2 hours and eluted to obtain the incubated ADC. The DAR value of the incubated ADC was tested (the test and calculation methods are the same as in example 4.1) to determine the plasma stability of the sample. The test results are shown in Table 3-4.

**Table 3-4. ADC plasma stability assay**

| ADC | Day 0 DAR value | Day 14 DAR value |
|---|---|---|
| B7H3-ADC-01 | 8.0 | 7.9 |
| B7H3-ADC-03 | 4.2 | 4.1 |
| Her2-ADC-04 | 7.9 | 7.5 |
| Reference ADC-01 | 7.4 | 2.3 |

The test results show that the DAR values of B7H3-ADC-01, B7H3-ADC-03, and Her2-ADC-04 were essentially maintained during plasma incubation (i.e., almost no payload linker was detached from the antibody), while the DAR decrease of reference ADC-01 with a maleimide linker was very significant after 14 days of incubation. The experimental results prove that all the ADCs of the present disclosure have better plasma stability compared to reference ADC-01.

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection for the present disclosure is defined by the appended claims.

## Claims

1. A conjugate of antibody-eribulin or a derivative thereof of formula I, 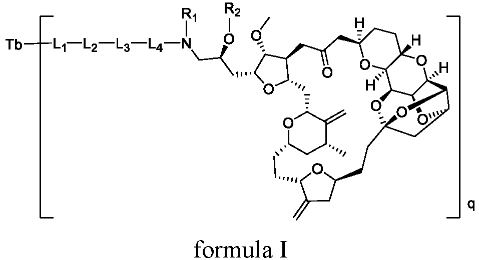
or a stereoisomer of the conjugate, a prodrug thereof, a pharmaceutically acceptable salt thereof, or a pharmaceutically acceptable solvate thereof,
wherein
Tb is an antibody or an antigen-binding fragment thereof that binds to a target, or a targeting moiety;
q is a drug-to-Tb coupling ratio;
R¹ and R² are each independently selected from hydrogen, deuterium, and C₁₋₆ alkyl; or, R¹ and R² together with the nitrogen atom and the oxygen atom to which they are attached form a 5- to 6-membered heterocyclic ring; the 5- to 6-membered heterocyclic ring is optionally substituted by one or more substituents of C₁₋₆ alkyl, C₁₋₆ alkoxy, C₃₋₆ cycloalkyl, and C₃₋₆ heterocyclyl;
L₁ is selected from 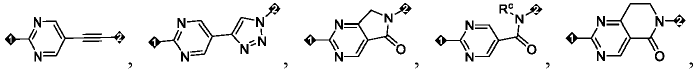 and 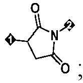 position 1 is attached to Tb via an S atom and position 2 is attached to L₂;
R^{c} is selected from hydrogen and C₁₋₆ alkyl;
L₂ is selected from 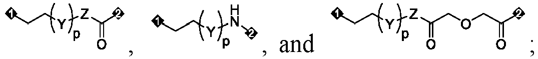
position 1 is attached to L₁ and position 2 is attached to L₃;
p is selected from any integer between 0 and 10;
Y is selected from -CH₂- and -OCH₂CH₂-;
Z is selected from -CR³R⁴- and -NR⁵-;
R³ and R⁴ are each independently selected from hydrogen, C₁₋₄ alkyl, C₂₋₄ alkenyl, C₂₋₄ alkynyl, C₃₋₆ cycloalkyl, and C₃₋₆ heterocyclyl; or, R³ and R⁴ together with the carbon atom to which they are attached form a 3- to 6-membered carbocyclic ring or a 3- to 6-membered heterocyclic ring;
R⁵ is selected from hydrogen, C₁₋₄ alkyl, C₂₋₄ alkenyl, C₂₋₄ alkynyl, C₃₋₆ cycloalkyl, and C₃₋₆ heterocyclyl;
L₃ is selected from a chemical bond, 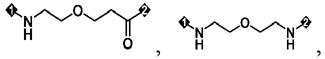
an amino acid residue, or a short peptide consisting of 2 to 10 amino acid residues; the amino acid residue is selected from a natural amino acid residue, an unnatural amino acid residue, or an amino acid residue represented by AA¹ or a stereoisomer thereof;
the amino acid residue represented by AA¹ has a structure as follows, 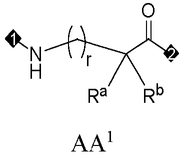
wherein
R^{a} and R^{b} are each independently selected from hydrogen and 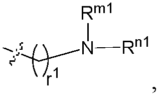
and R^{a} and R^{b} are not both hydrogen at the same time;
or, R^{a} and R^{b} together with the carbon atom to which they are both attached form a 4- to 10-membered heterocyclic ring; the 4- to 10-membered heterocyclic ring is optionally substituted by one or more R⁰;
r and r¹ are each independently selected from any integer between 0 and 20;
R^{m1} and Rⁿ¹ are each independently selected from hydrogen, C₁₋₆ alkyl, and C₃₋₆ cycloalkyl;
or, R^{m1} and Rⁿ¹ together with the nitrogen atom to which they are both attached form a 4-to 10-membered heterocyclic ring; the 4- to 10-membered heterocyclic ring is optionally substituted by one or more R^{0'};
R⁰ and R^{0'} are each independently selected from C₁₋₆ alkyl, C₃₋₆ cycloalkyl, -NR^{m2}Rⁿ², and optionally C₁₋₆ alkyl-substituted 4- to 10-membered heterocyclyl;
R^{m2} and Rⁿ² are each independently selected from hydrogen and C₁₋₆ alkyl;
L₄ is absent or present; when L₄ is present, L₄ is selected from 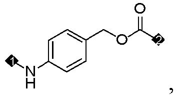 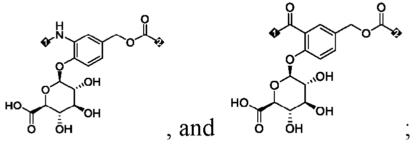
position 1 is attached to L₃ and position 2 is attached to the nitrogen atom of eribulin;
and, when L₁ is 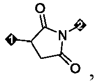
position 1 is attached to Tb via an S atom, and position 2 is attached to L₂:
L₃ is not a chemical bond, Val-Cit, Val-Lys, Gly-Gly-Phe-Gly, or Ala-Ala-Asn; or,
L₄ is present and is not

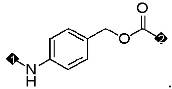

2. The conjugate according to claim 1, or a stereoisomer of the conjugate, a prodrug thereof, a pharmaceutically acceptable salt thereof, or a pharmaceutically acceptable solvate thereof, wherein one or more of the following conditions are satisfied:
(1) Tb is an antibody or an antigen-binding fragment thereof, which satisfies one or more of the following conditions:
(i) the antibody or the antigen-binding fragment thereof comprise Fab, Fab', F(ab')2, Fd, Fv, dAb, complementarity determining region fragment, non-human antibody, humanized antibody, chimeric antibody, fully human antibody, probody, monoclonal antibody, bispecific antibody, or multi-specific antibody;
(ii) Tb is a monoclonal antibody or an antigen-binding fragment;
(iii) Tb is an antibody or an antigen-binding fragment thereof which has endocytic activity;
(iv) Tb is an antibody or an antigen-binding fragment thereof which has the activity of binding to a free antigen in tumor tissues and/or a tumor cell surface antigen;
(v) Tb is selected from an antibody or an antigen-binding fragment thereof that binds to the following targets: growth factor, integrin α5β6, integrin α4β7, 0772P, 5T4, ACTA2, ADGRE1, AGS-16, AIF1, AKR1C1, AKR1C2, ANGPTL4, ApoE, ASLG659, BAFF-R, BMPR1B, BNIP3, Brevican, C1QA, C1QB, CA6, pCAD, P-cadherin, CADM1, CCL5, CCR5, CDl lb, CD1 lc, CD19, CD20, CD21, CD22, CD30, CD33, CD37, CD40, CD45 (PTPRC), CD49D (ITGA4), CD56, CD66e, CD70, CD72, CD74, CD79a, CD80, CD138, CD223, CDCP1, CDH11, Claudin18.2, COL6A3, COL7A1, CRIPTO, CSF1R, CTGF, CTSD, CTSS, CXCL10, CXCL11, CXCR5, DDIT4, DLL3, DLL4, DRS, E16, EFNA4, B7H3, EGFR, EGFRvIII, EGLN, EGLN3, EMR2, Endothelin receptor, ENPP3, EpCAM, EphB2R, ETBR, FGF2, FGFR2, FcRH1, FcRH2, GEDA, GPNMB, GZMB, Her2, Her3, HLA-DOB, HMOX1, IFNG, IFI6, IGF-1R, IGFBP3, IL10RA1, IL20Rα, IL-6, IRTA2, KISS1R, KRT33A, LOX, LRRC15, LUM, LY6E, LY64, LY86, LYPD3, MCPT8, MDP, Mesothelin, c-Met, MFI2, MMP10, MMP14, MMP16, MPF, MSG783, MS4A7, MUC 1, MUC16, NaPi2b, NaPi3b, NCA, Nectin 4, NOG, P2X5, PD-L1, PDK1, PDGFRA, PFKFB3, PGF, PGK1, PIK3AP1, PIK3CD, PLOD2, PSCA, PSCAhlg, PSMA, RNF43, ROR1, Sema5b, SERPINEl, SLC39A6, SLITRK6, SLTRK6, STAT1, STC2, STEAP1, STEAP2, TCF4, TENB2, TGF, TGFBR1, TGFB2, Tissue factor, TNFRSF21, TNFSF9, Trop-2, TrpM4, Tyro7, UPK1B, VEGFA, and WNT5A;
(vi) Tb is an anti-B7H3 antibody or an antigen-binding fragment thereof, an anti-Trop-2 antibody or an antigen-binding fragment thereof, an anti-Her2 antibody or an antigen-binding fragment thereof, an anti-Her3 antibody or an antigen-binding fragment thereof, or an anti-EGFR antibody or an antigen-binding fragment thereof;
(2) the q is selected from any numerical value between 0.1 and 16.0; preferably, q is selected from any numerical value between 1 and 10; more preferably, q is selected from 1, 2, 3, 4, 5, 6, 7, and 8;
(3) the R¹ and R² are hydrogen; or, R¹ and R² together with the nitrogen atom and the oxygen atom to which they are attached form 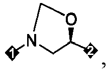 and position 1 is attached to L₄ or L₃;
(4) the L₁ is selected from 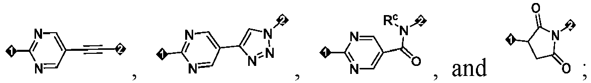 position 1 is attached to Tb via an S atom and position 2 is attached to L₂;
preferably, L₁ is selected from 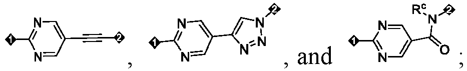 position 1 is attached to Tb via an S atom and position 2 is attached to L₂; more preferably, L₁ is 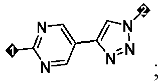 position 1 is attached to Tb via an S atom and position 2 is attached to L₂;
(5) the R^{c} is hydrogen;
(6) the p is selected from 0, 1, 2, 3, 4, 5, 6, 7, and 8;
(7) the Z is -CH₂-, -C(CH₃)₂-, or -NH-;
(8) the R³ and R⁴ are each independently selected from hydrogen and methyl;
(9) the R⁵ is H or methyl;
(10) the L₃ is selected from amino acid residues Val, D-Val, Cit, Phe, Lys, Lys(Ac), Leu, Gly, Ala, Asn, Asp, Arg, and AA¹, or a short peptide consisting of 2 to 10 amino acid residues selected from Val, Cit, Phe, Lys, D-Val, Leu, Gly, Ala, Asn, Asp, and AA¹;
preferably, L₃ is selected from Val, Cit, Phe, Lys, D-Val, Leu, Gly, Ala, Asn, AA¹, Val-Cit, Cit-Val, Cit-Ala, Val-Ala, Lys-Val, Val-Lys(Ac), Phe-Lys, Phe-Lys(Ac), Ala-Ala, Val-AA¹, Ala-AA¹, Gly-AA¹, AA¹-Gly, Ala-Ala-Ala, Ala-Ala-Asn, Ala-Ala-Asp, Val-AA¹-Gly, Ala-AA¹-Gly, Gly-AA¹-Gly, Lys-(Ala)₂-Asn, Lys-(Ala)₂-Asp, Gly-Phe-Gly, (Gly)₂-Phe-Gly, D-Val-Leu-Lys, Gly-Gly-Arg, Ala-Ala-Asn, Gly-Gly-Phe, Val-Lys-Gly, Val-Lys-(Gly)₂, Val-Lys, Lys-Ala-Asn, and (Gly)₂-Phe-AA¹;
more preferably, L₃ is selected from Val-AA¹-Gly, Val-AA¹, Val-Cit, and (Gly)₂-Phe-AA¹; particularly preferably, L₃ is selected from Val-AA¹ and Val-Cit;
(11) one of the R^{a} and R^{b} is hydrogen, and the other is 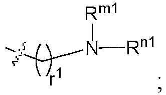 or, R^{a} and R^{b} together with the carbon atom to which they are both attached form a 5- to 6-membered heterocyclic ring substituted by R⁰; preferably, R^{a} and R^{b} together with the carbon atom to which they are both attached form 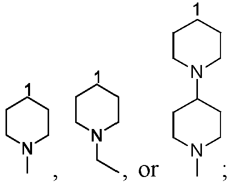 the carbon atom marked with number 1 is the carbon atom to which R^{a} and R^{b} are both attached;
(12) the r and r¹ are each independently selected from 0, 1, 2, 3, 4, and 5; preferably, r and r¹ are each independently selected from 0 and 4; more preferably, r is 0 and r¹ is 4;
(13) the R^{m1} and Rⁿ¹ are each independently selected from C₁₋₆ alkyl; or, R^{m1} and Rⁿ¹ together with the nitrogen atom to which they are both attached form a 5- to 6-membered heterocyclic ring optionally substituted by R^{0'};
preferably, R^{m1} and Rⁿ¹ are each independently selected from methyl, ethyl, n-propyl, and n-butyl; or, R^{m1} and Rⁿ¹ together with the nitrogen atom to which they are both attached form a piperidine ring or a piperazine ring, and the piperidine ring and the piperazine ring are optionally substituted by R^{0'};
(14) the R⁰ and R^{0'} are each independently selected from C₁₋₆ alkyl, -NR^{m2}Rⁿ², and optionally C₁₋₆ alkyl-substituted 5- to 6-membered heterocyclyl;
(15) the R^{m2} and Rⁿ² are methyl; and
(16) the L₂ is selected from 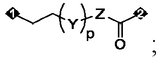 position 1 is attached to L₁ and position 2 is attached to L₃.

3. The conjugate according to claim 1 or 2, or a stereoisomer of the conjugate, a prodrug thereof, a pharmaceutically acceptable salt thereof, or a pharmaceutically acceptable solvate thereof, wherein one or more of the following conditions are satisfied:
(1) L₂ is selected from 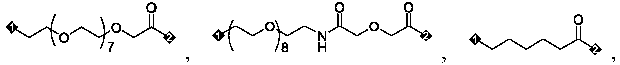 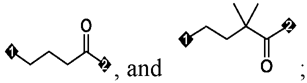 position 1 is attached to L₁ and position 2 is attached to L₃; preferably, L₂ is 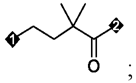 position 1 is attached to L₁ and position 2 is attached to L₃;
(2) L₃ is selected from a chemical bond, 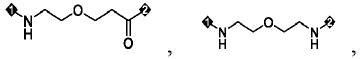 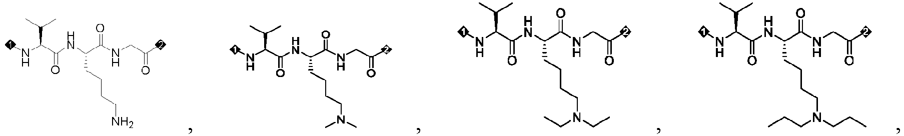 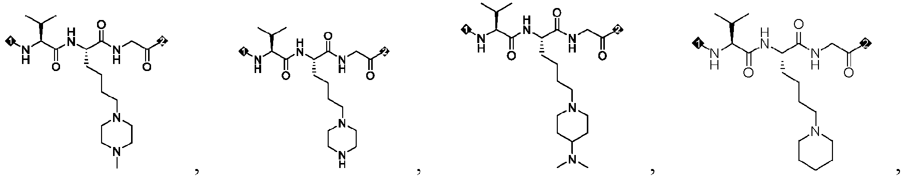 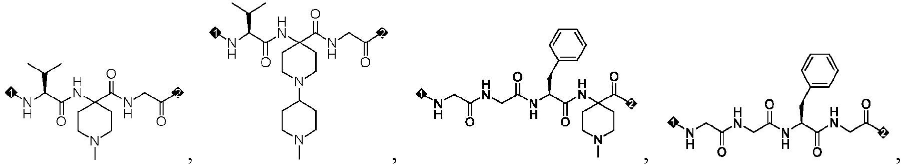 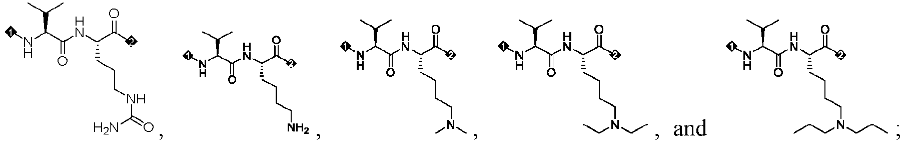 position 1 is attached to L₂ and position 2 is attached to L₄ or the nitrogen atom of eribulin;
preferably, L₃ is selected from 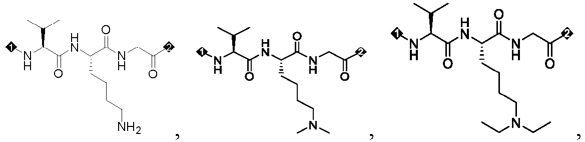 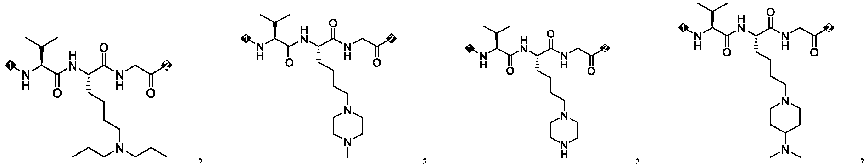 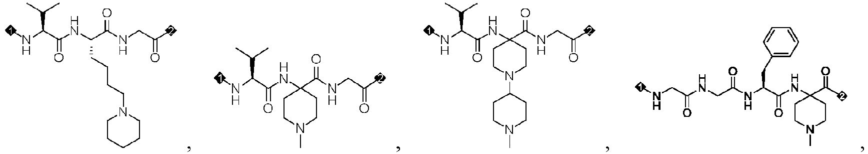 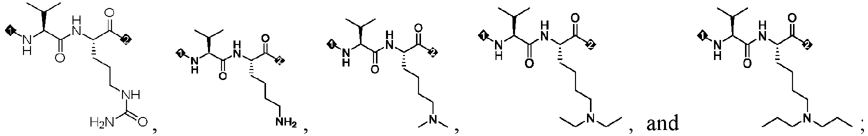 position 1 is attached to L₂ and position 2 is attached to L₄ or the nitrogen atom of eribulin;
more preferably, L₃ is selected from 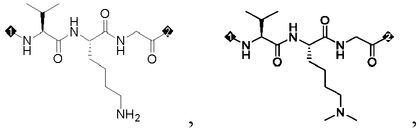 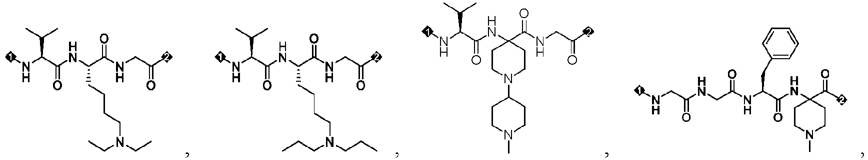 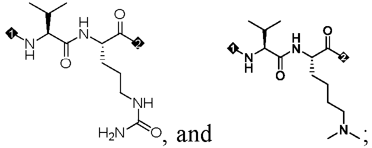
position 1 is attached to L₂ and position 2 is attached to L₄ or the nitrogen atom of eribulin;
particularly preferably, L₃ is selected from 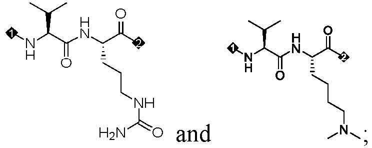
position 1 is attached to L₂ and position 2 is attached to L₄ or the nitrogen atom of eribulin;
(3) the amino acid residue represented by AA¹ is selected from 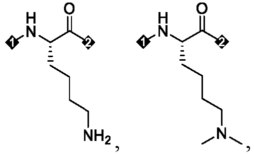 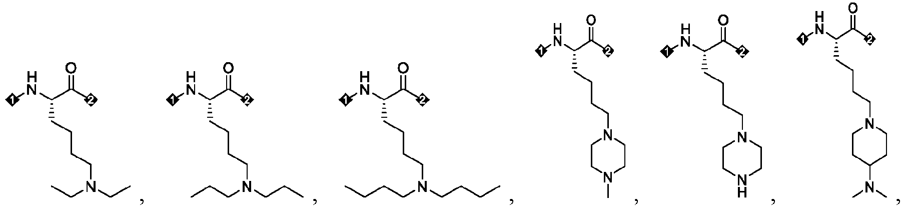 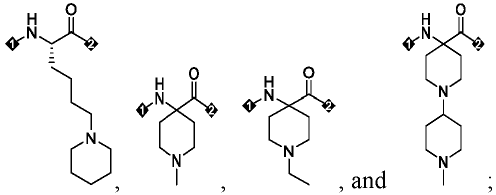
preferably, the amino acid residue represented by AA¹ is selected from 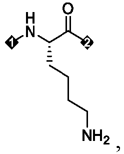 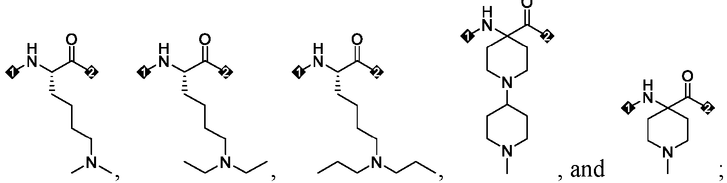
more preferably, the amino acid residue represented by AA¹ is 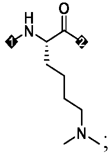 and
(4) L₄ is absent or present; when L₄ is present, L₄ is selected from 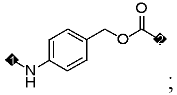 position 1 is attached to L₃ and position 2 is attached to the nitrogen atom of eribulin;
preferably, L₄ is 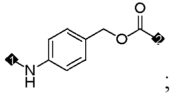 position 1 is attached to L₃ and position 2 is attached to the nitrogen atom of eribulin.

4. The conjugate according to any one of claims 1 to 3, or a stereoisomer of the conjugate, a prodrug thereof, a pharmaceutically acceptable salt thereof, or a pharmaceutically acceptable solvate thereof, wherein the linker 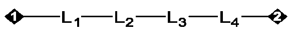 is selected from a linker of the following formulas: 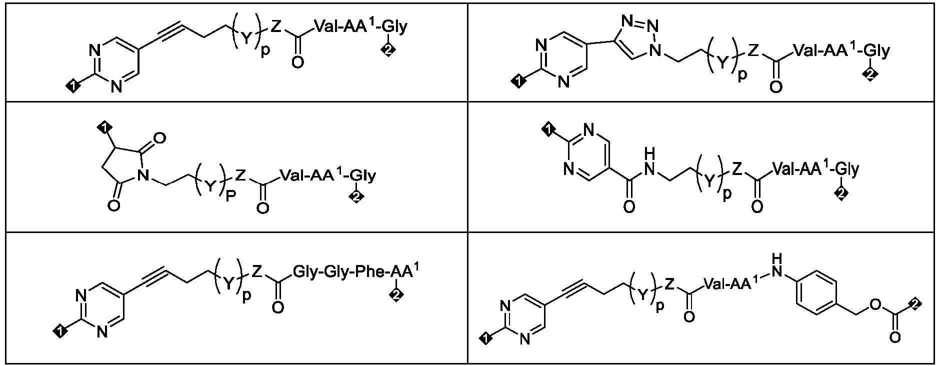 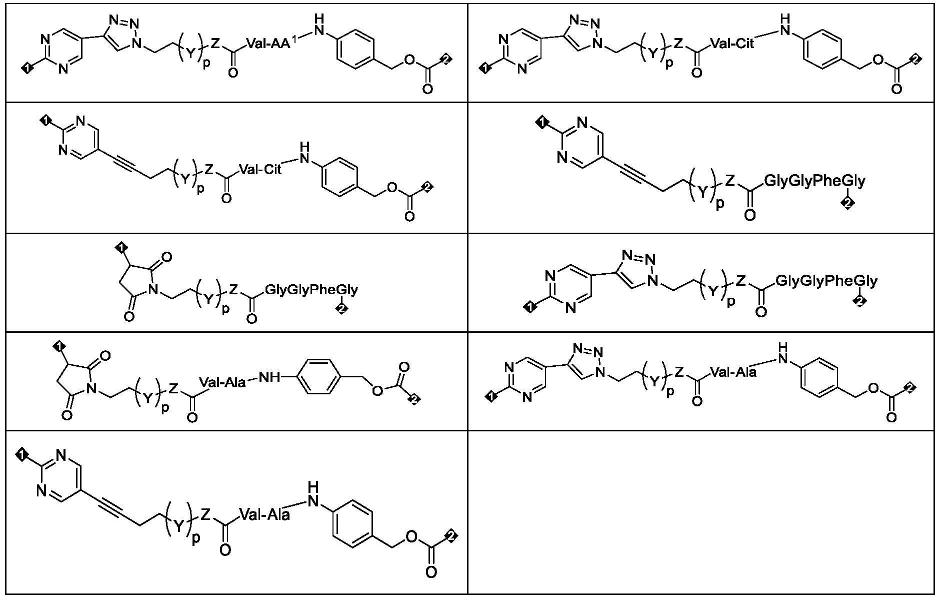
wherein Y, Z, p, and AA¹ are as defined in any one of claims 1 to 3; position 1 is attached to Tb via an S atom and position 2 is attached to the nitrogen atom of eribulin; 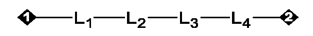
preferably, the linker is selected from a linker of the following formulas: 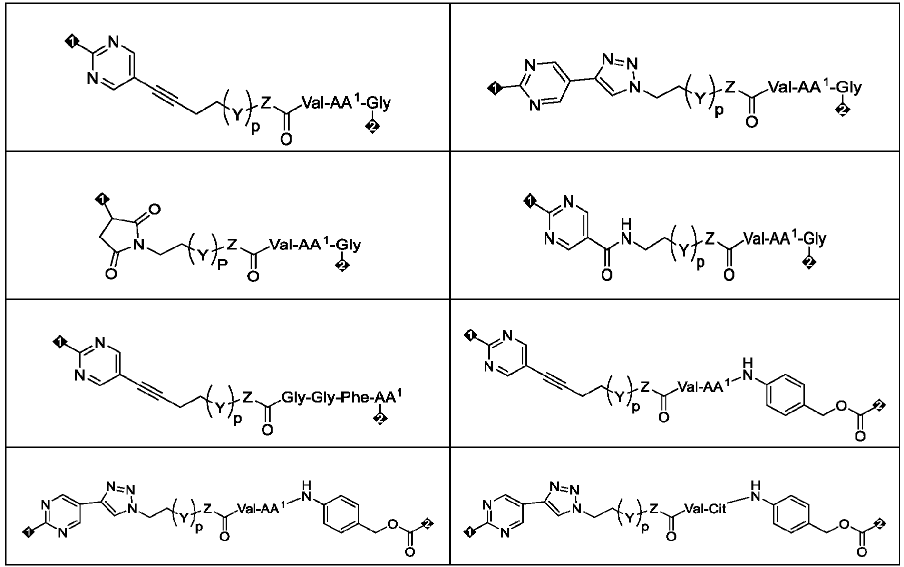
wherein Y, Z, p, and AA¹ are as defined in any one of claims 1 to 3; position 1 is attached to Tb via an S atom and position 2 is attached to the nitrogen atom of eribulin;
more preferably, the linker 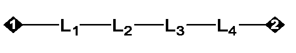 is selected from a linker of the following formulas: 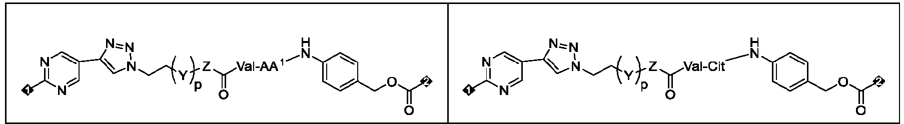
wherein Y, Z, p, and AA¹ are as defined in any one of claims 1 to 3; position 1 is attached to Tb via an S atom and position 2 is attached to the nitrogen atom of eribulin.

5. The conjugate according to any one of claims 1 to 4, or a stereoisomer of the conjugate, a prodrug thereof, a pharmaceutically acceptable salt thereof, or a pharmaceutically acceptable solvate thereof, wherein the linker 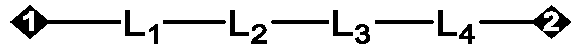 is selected from the following: 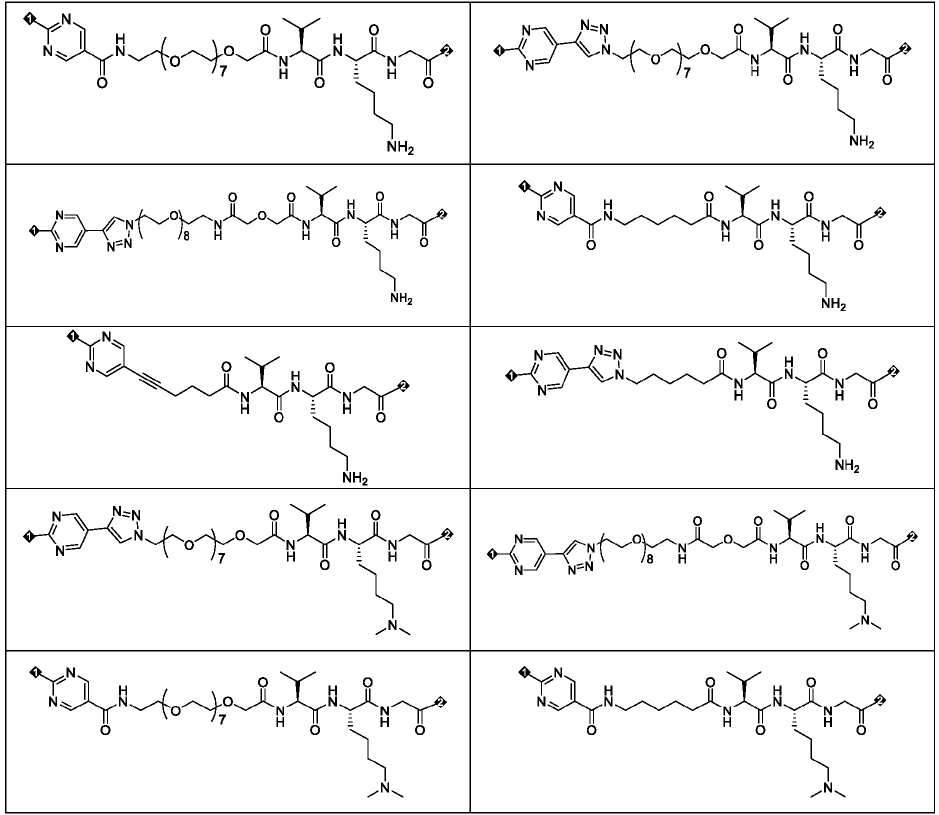 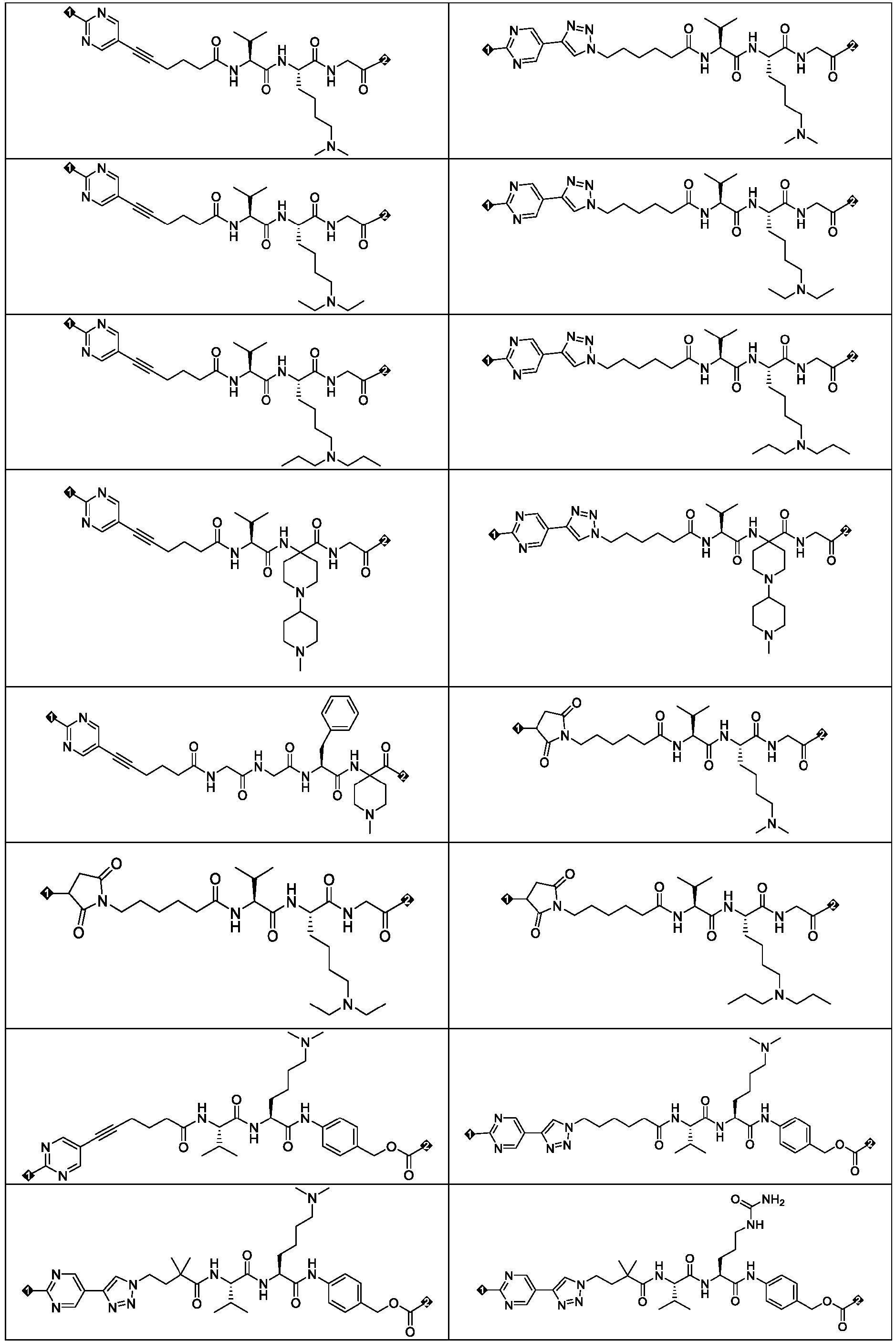 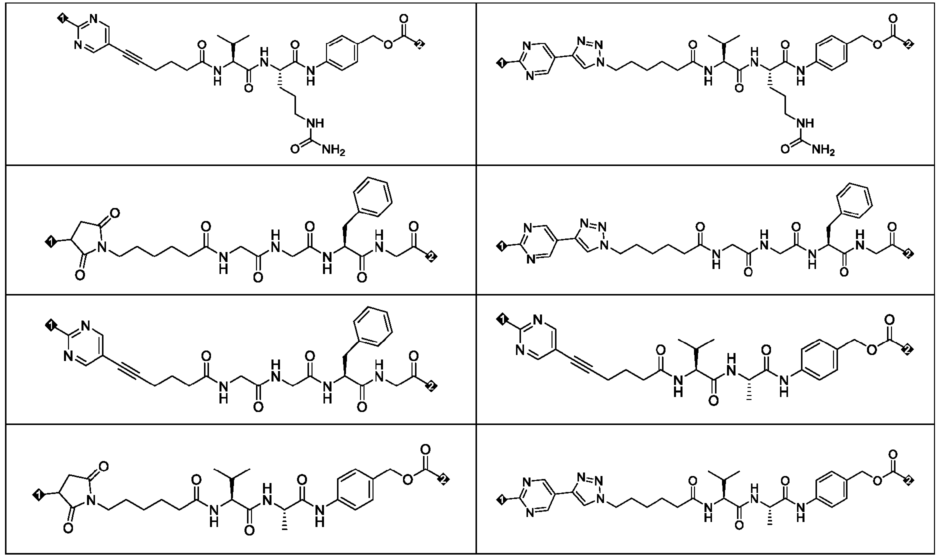
position 1 is attached to Tb via an S atom and position 2 is attached to the nitrogen atom of eribulin;
preferably, the linker 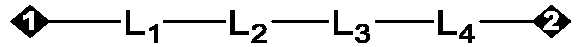
is selected from the following: 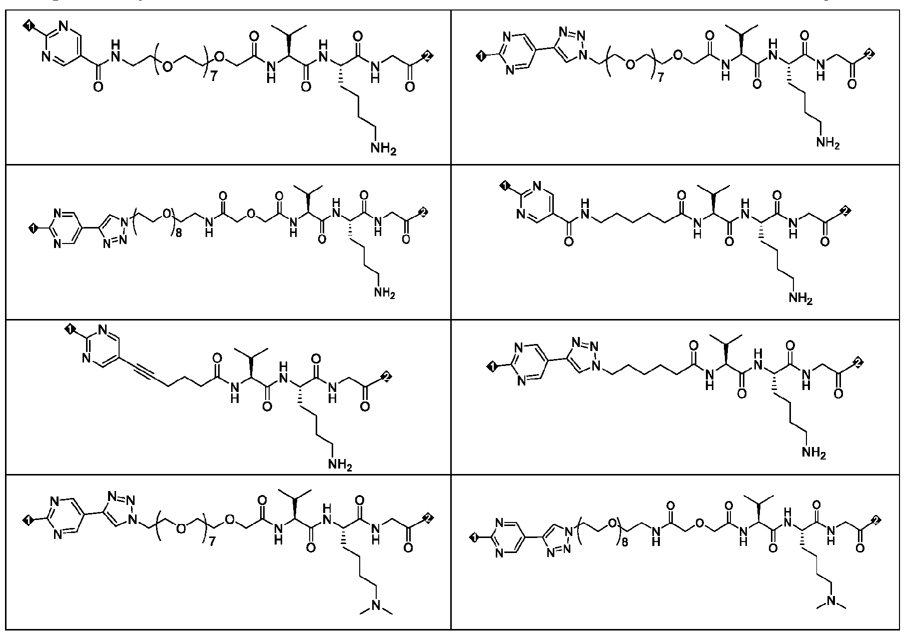 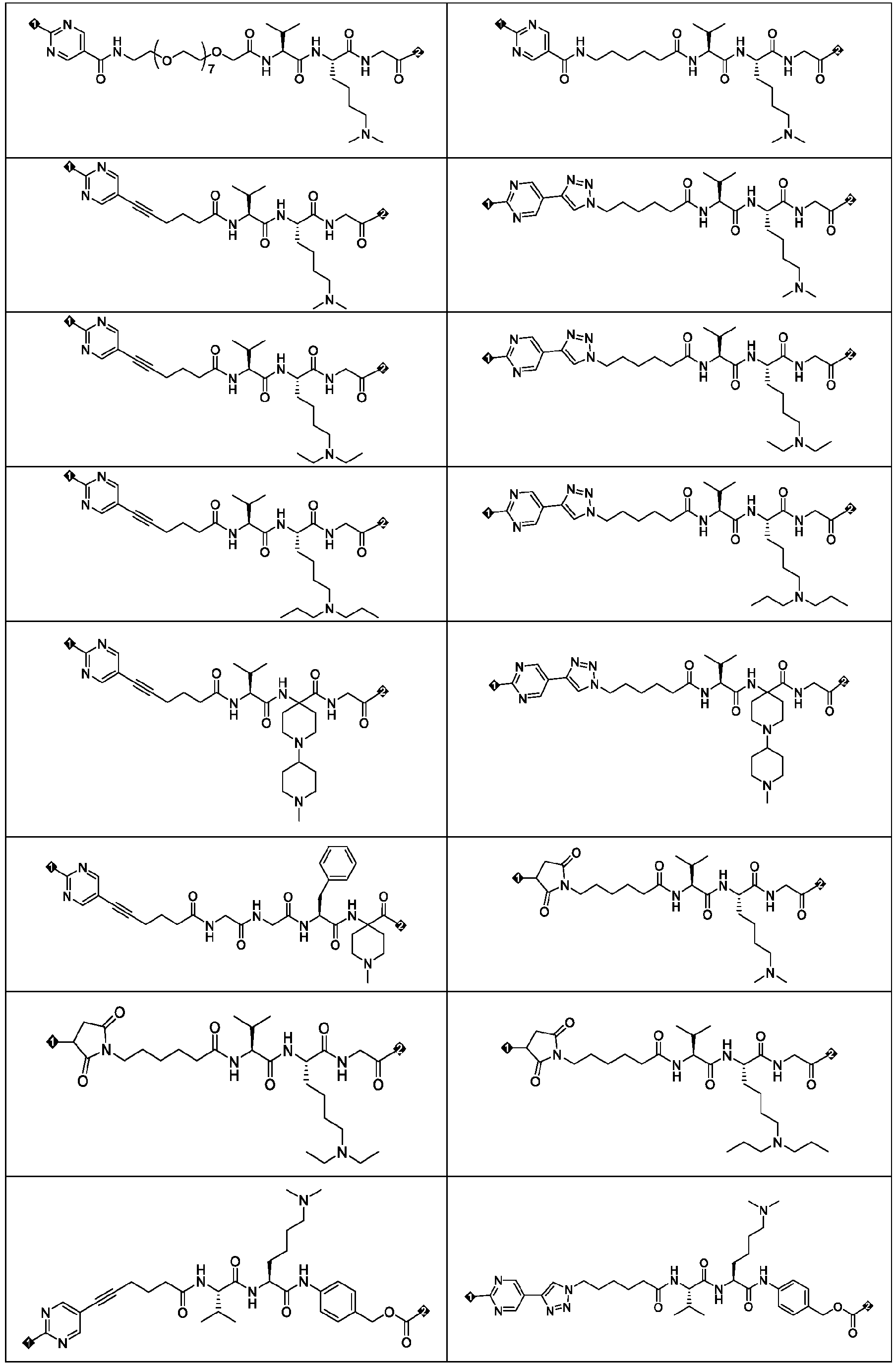 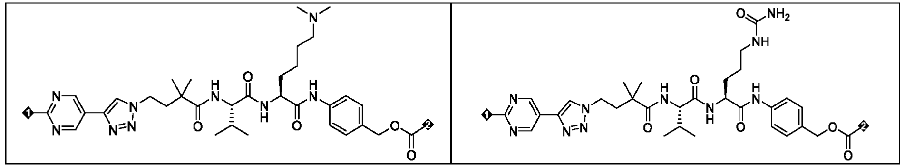
position 1 is attached to Tb via an S atom and position 2 is attached to the nitrogen atom of eribulin;
more preferably, the linker 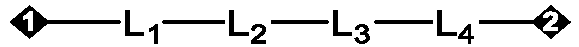
is selected from the following: 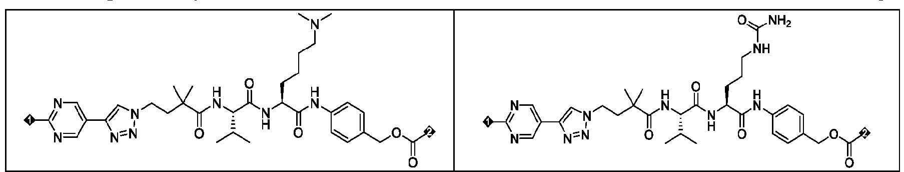
position 1 is attached to Tb via an S atom and position 2 is attached to the nitrogen atom of eribulin.

6. The conjugate according to any one of claims 1 to 5, or a stereoisomer of the conjugate, a prodrug thereof, a pharmaceutically acceptable salt thereof, or a pharmaceutically acceptable solvate thereof, wherein the conjugate of antibody-eribulin or the derivative thereof is selected from: 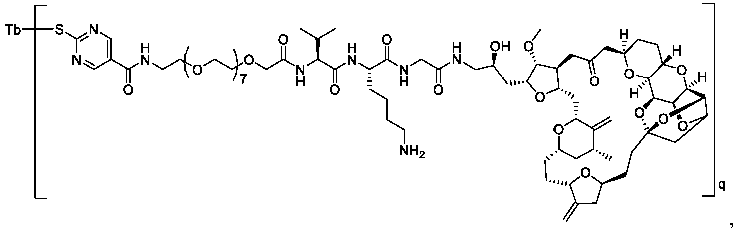 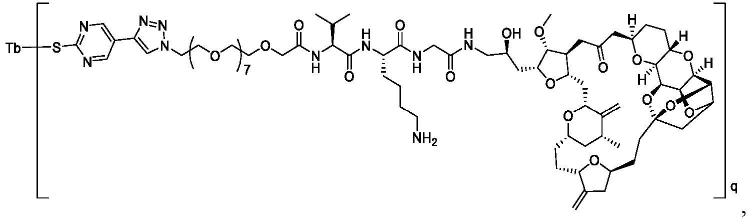 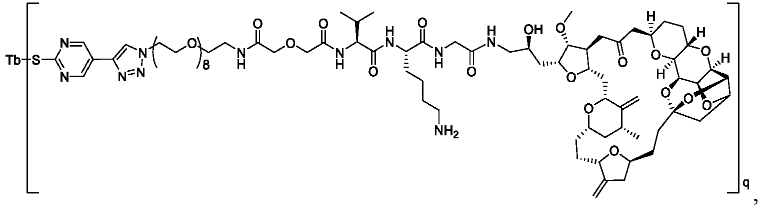 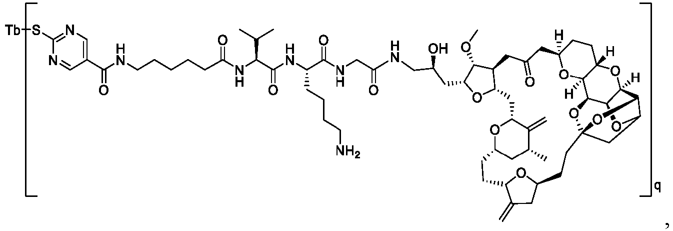 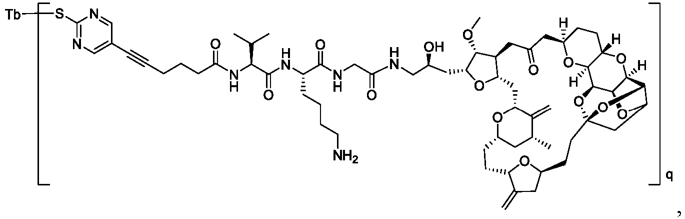 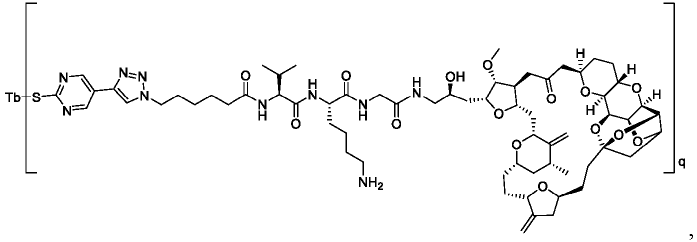 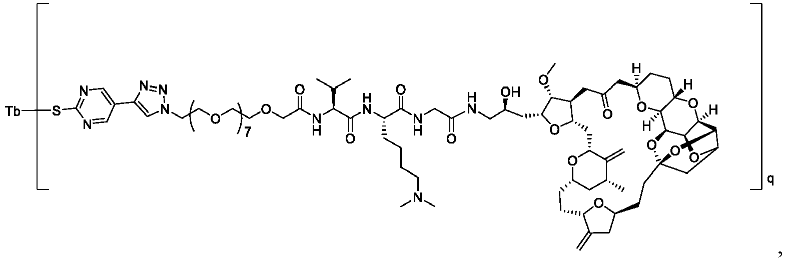 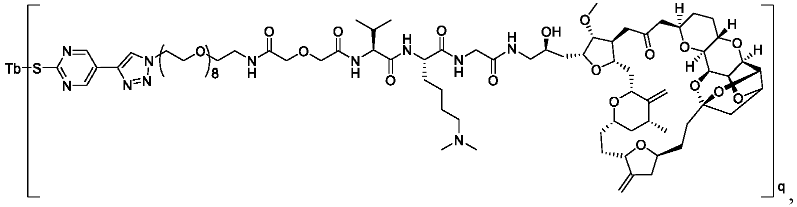 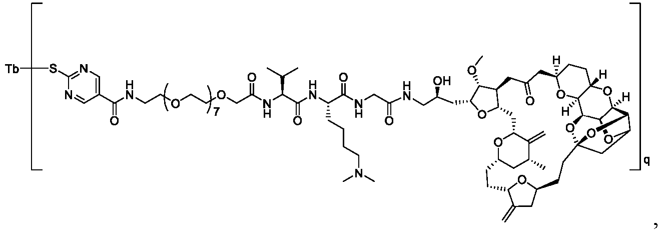 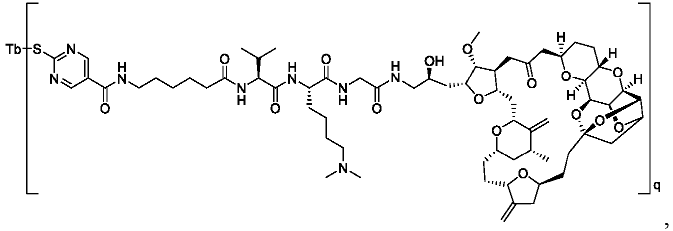 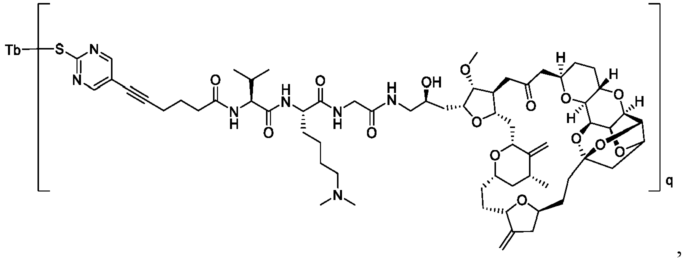 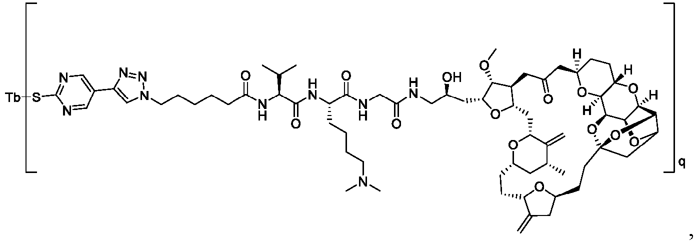 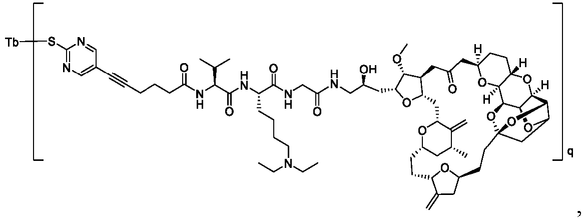 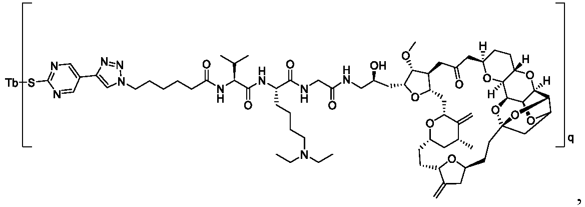 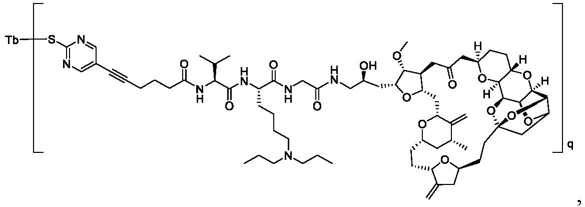 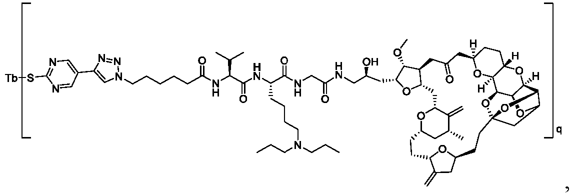 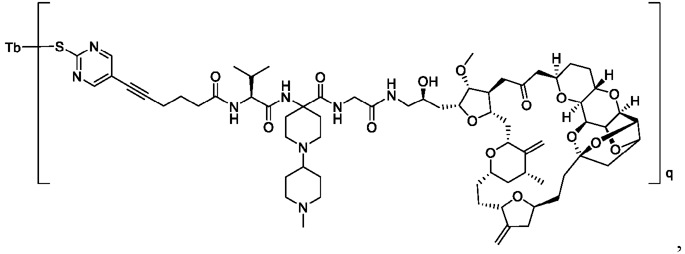 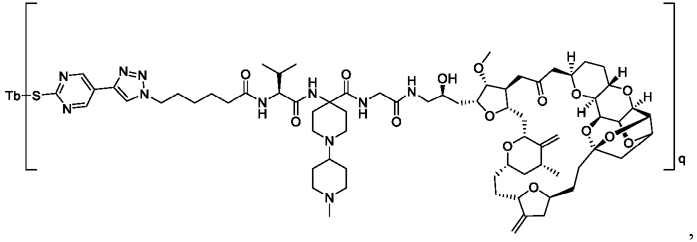 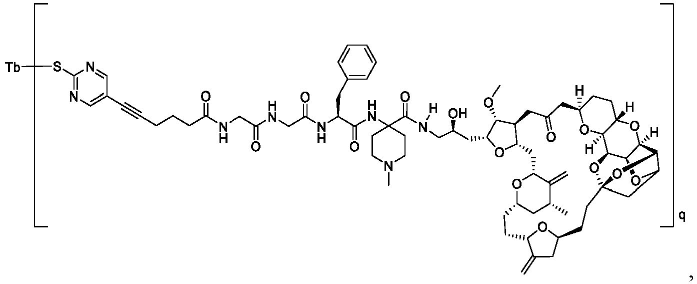 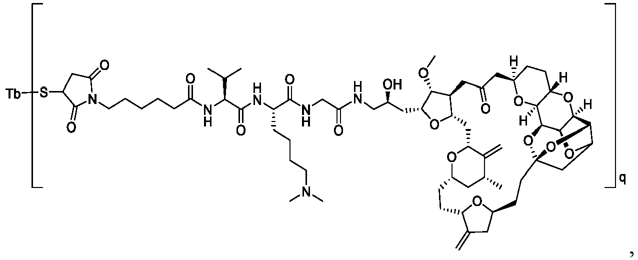 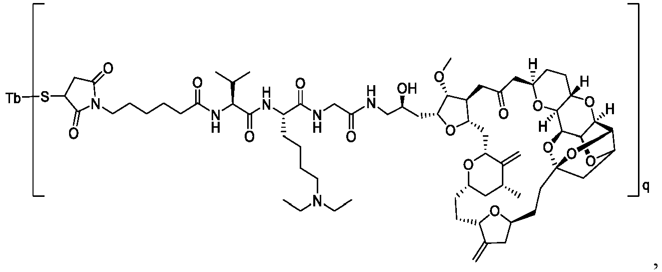 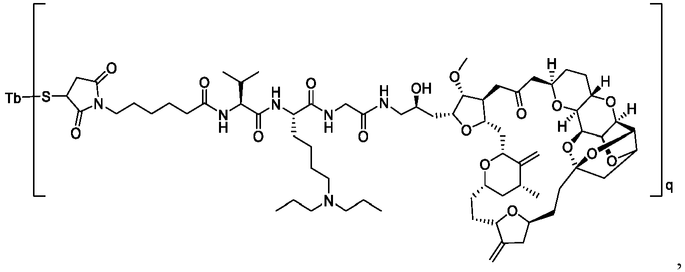 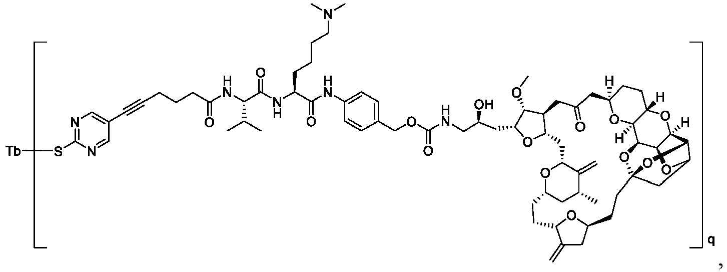 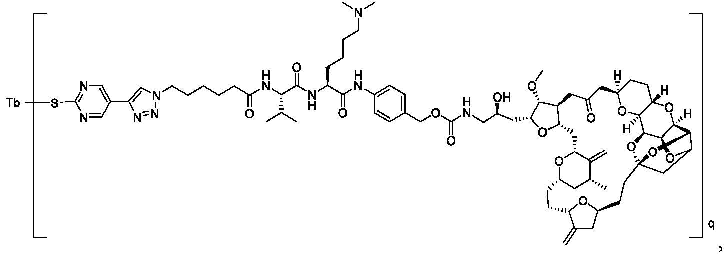 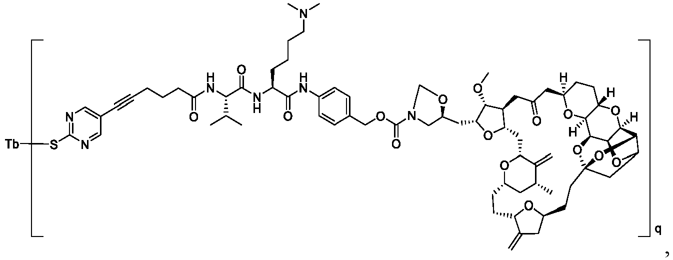 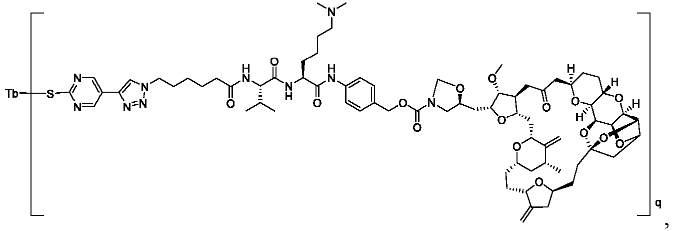 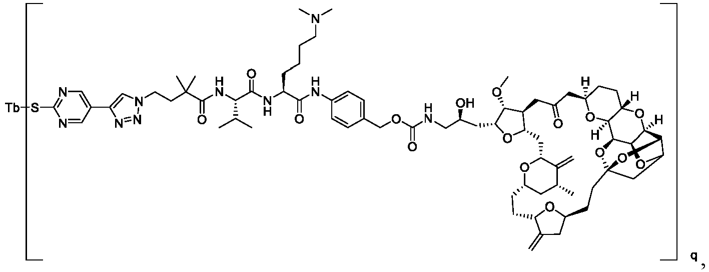 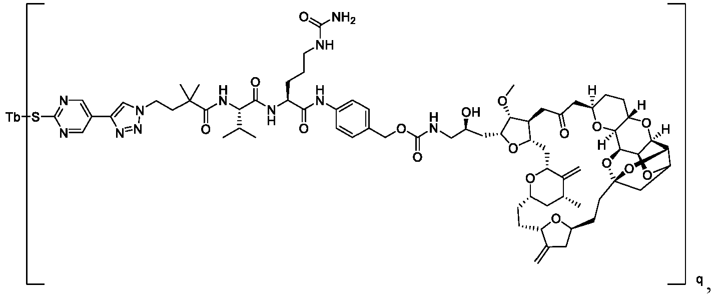 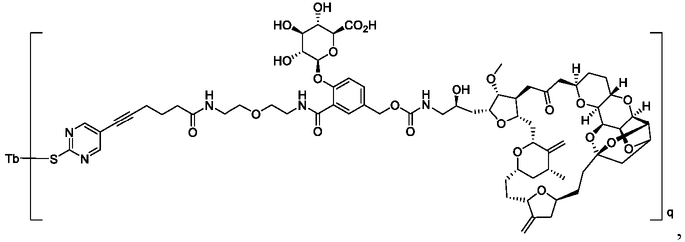 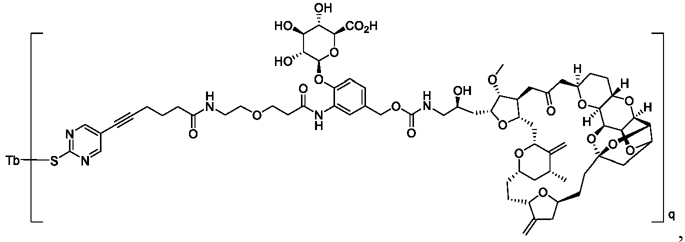 and

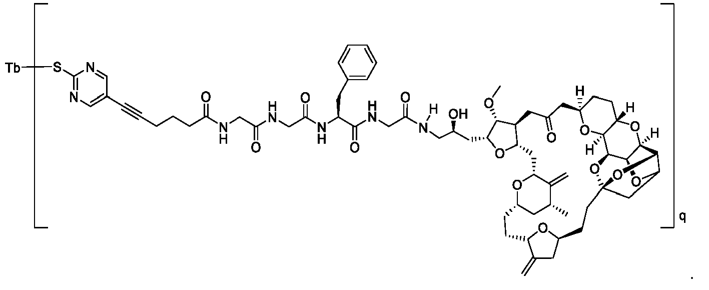

7. The conjugate according to any one of claims 1 to 6, or a stereoisomer of the conjugate, a prodrug thereof, a pharmaceutically acceptable salt thereof, or a pharmaceutically acceptable solvate thereof, wherein Tb is any one of the following schemes:
(i) Tb is an anti-Her2 antibody or an antigen-binding fragment thereof, wherein the anti-Her2 antibody is anbenitamab, coprelotamab, disitamab, gancotamab, margetuximab, pertuzumab, timigutuzumab, zanidatamab, trastuzumab, pertuzumab, or an antigen-binding fragment thereof;
(ii) Tb is an anti-Trop-2 antibody or an antigen-binding fragment thereof, wherein the anti-Trop-2 antibody is datopotamab, sacituzumab, or an antigen-binding fragment thereof;
(iii) Tb is an anti-EGFR antibody or an antigen-binding fragment thereof, wherein the anti-EGFR antibody is demupitamab, depatuxizumab, futuximab, imgatuzumab, laprituximab, losatuxizumab, matuzumab, modotuximab, necitumumab, nimotuzumab, panitumumab, pimurutamab, serclutamab, tomuzotuximab, zalutumumab, cetuximab, or an antigen-binding fragment thereof;
(iv) Tb is an anti-B7H3 antibody or an antigen-binding fragment thereof, wherein the anti-B7H3 antibody is enoblituzumab, mirzotamab, omburtamab, antibody 1D1-01, antibody 2E3-02, or an antigen-binding fragment thereof;
(v) Tb is an anti-B7H3 antibody or an antigen-binding fragment thereof, wherein the anti-B7H3 antibody or the antigen-binding fragment thereof comprises a complementarity determining region (CDR) as follows, wherein the CDR is defined by the Kabat numbering system:
HCDR1 with the sequence of SEQ ID NO: 9, HCDR2 with the sequence of SEQ ID NO: 10, and HCDR3 with the sequence of SEQ ID NO: 11; and/or,
LCDR1 with the sequence of SEQ ID NO: 12, LCDR2 with the sequence of SEQ ID NO: 13, and LCDR3 with the sequence of SEQ ID NO: 14;
(vi) Tb is an anti-B7H3 antibody or an antigen-binding fragment thereof, wherein the anti-B7H3 antibody or the antigen-binding fragment thereof comprises a complementarity determining region (CDR) as follows, wherein the CDR is defined by the IMGT numbering system:
HCDR1 with the sequence of SEQ ID NO: 15, HCDR2 with the sequence of SEQ ID NO: 16, and HCDR3 with the sequence of SEQ ID NO: 17; and/or,
LCDR1 with the sequence of SEQ ID NO: 18, LCDR2 with the sequence of GAS, and LCDR3 with the sequence of SEQ ID NO: 19;
(vii) Tb is an anti-B7H3 antibody or an antigen-binding fragment thereof, wherein the anti-B7H3 antibody or the antigen-binding fragment thereof comprises: a VH set forth in SEQ ID NO: 1 or 5 and/or a VL set forth in SEQ ID NO: 2 or 6;
the anti-B7H3 antibody or the antigen-binding fragment thereof further comprises:
(a) a heavy chain constant region (CH) of a human immunoglobulin or a variant thereof; and/or
(b) a light chain constant region (CL) of a human immunoglobulin or a variant thereof;
(viii) Tb is an anti-B7H3 antibody or an antigen-binding fragment thereof, wherein the anti-B7H3 antibody or the antigen-binding fragment thereof comprises:
a VH set forth in SEQ ID NO: 5 and a CH set forth in SEQ ID NO: 20 or a variant thereof, and/or, a VL set forth in SEQ ID NO: 6 and a CL set forth in SEQ ID NO: 21 or a variant thereof;
preferably, the anti-B7H3 antibody or the antigen-binding fragment thereof comprises:
a heavy chain set forth in SEQ ID NO: 3 and/or a light chain set forth in SEQ ID NO: 4; or
a heavy chain set forth in SEQ ID NO: 7 and/or a light chain set forth in SEQ ID NO: 8.

8. The conjugate according to any one of claims 1 to 7, or a stereoisomer of the conjugate, a prodrug thereof, a pharmaceutically acceptable salt thereof, or a pharmaceutically acceptable solvate thereof, wherein the conjugate is selected from any one of the following: 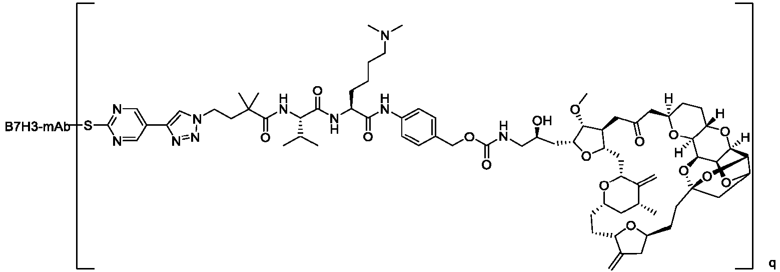
wherein B7H3-mAb is antibody 2E3-02, and q is selected from any integer between 0.1 and 10.0; preferably, q is selected from 1, 2, 3, 4, 5, 6, 7, and 8; 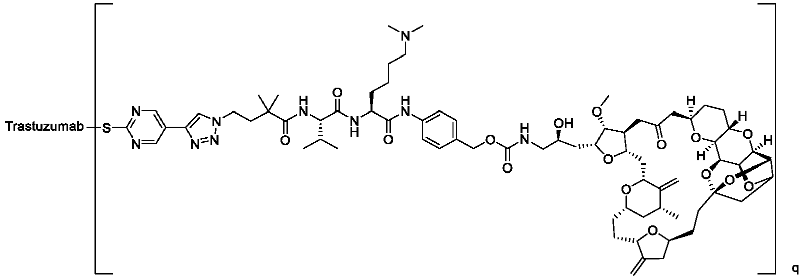
wherein q is selected from any integer between 0.1 and 10.0; preferably, q is selected from 1, 2, 3, 4, 5, 6, 7, and 8; 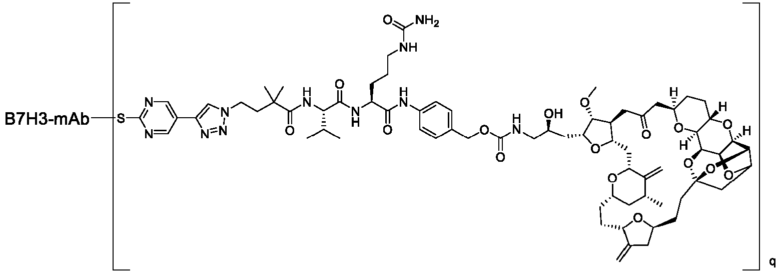
wherein B7H3-mAb is antibody 2E3-02, and q is selected from any integer between 0.1 and 10.0; preferably, q is selected from 1, 2, 3, 4, 5, 6, 7, and 8; or 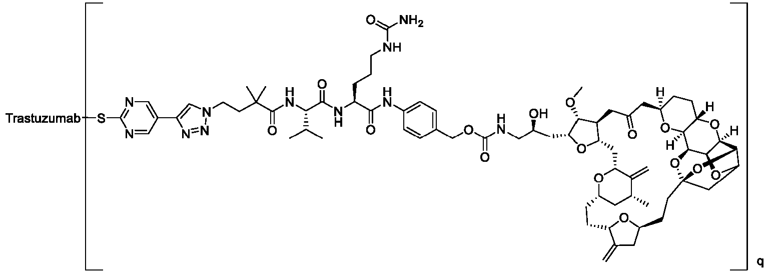
wherein q is selected from any integer between 0.1 and 10.0; preferably, q is selected from 1, 2, 3, 4, 5, 6, 7, and 8.

9. A drug-linker conjugate of formula II, 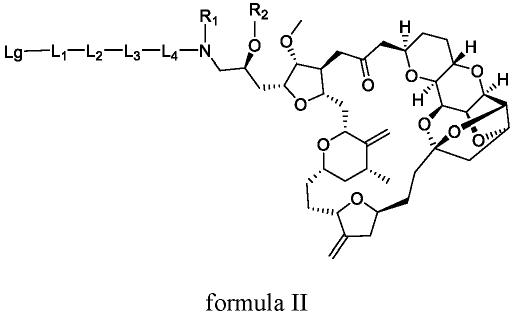
or a stereoisomer of the drug-linker conjugate, a prodrug thereof, a pharmaceutically acceptable salt thereof, or a pharmaceutically acceptable solvate thereof,
wherein
when L₁ is 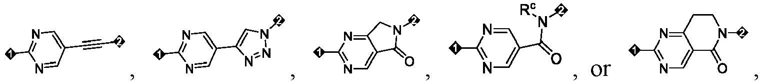 position 1 is attached to Lg and position 2 is attached to L₂;
Lg is a leaving group when reacting with Tb; L₂, L₃, L₄, R^{c}, R¹, and R² are as defined in any one of claims 1 to 6;
when L₁ is 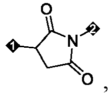
Lg-L₁ is 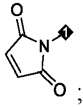
L₂, L₃, L₄, R^{c}, R¹, and R² are as defined in any one of claims 1 to 6.

10. The drug-linker conjugate according to claim 9, or a stereoisomer of the conjugate, a prodrug thereof, a pharmaceutically acceptable salt thereof, or a pharmaceutically acceptable solvate thereof, wherein the Lg is selected from halogen, sulfonyl, a tertiary amine salt group (Me₃N⁺, Et₃N⁺), diazonium, -OMs, MeSO₂-, and CF₃SO₃-; preferably, Lg is selected from F, Cl, and MeSO₂-; more preferably, Lg is selected from F and MeSO₂-.

11. The drug-linker conjugate according to claim 9 or 10, or a stereoisomer of the drug-linker conjugate, a prodrug thereof, a pharmaceutically acceptable salt thereof, or a pharmaceutically acceptable solvate thereof, wherein the drug-linker conjugate is selected from: 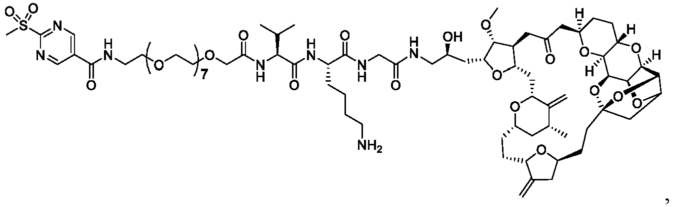 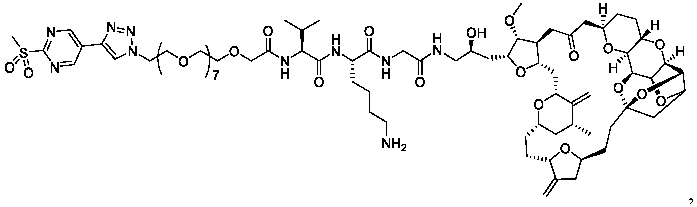 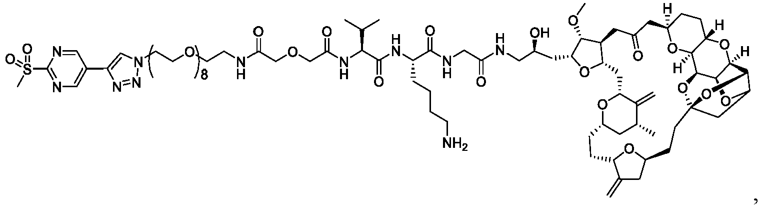 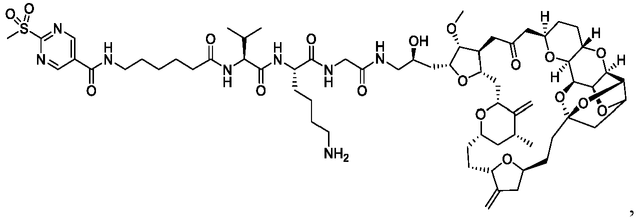 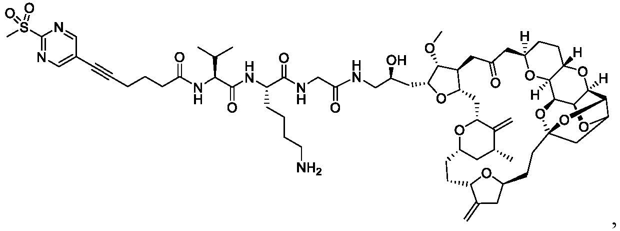 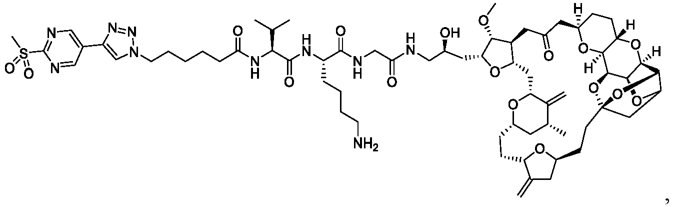 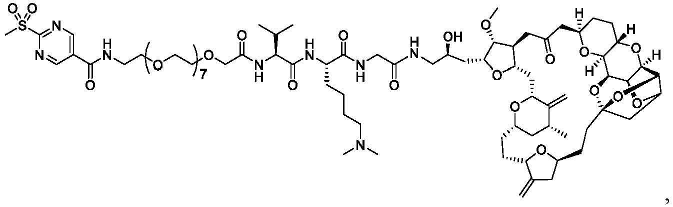 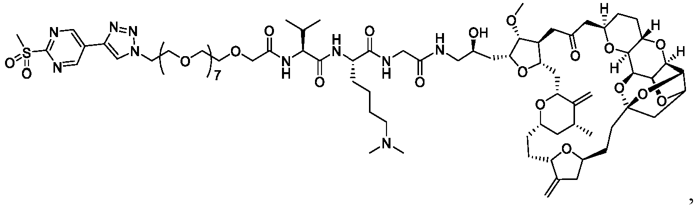 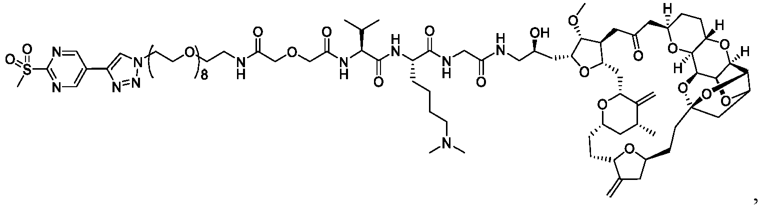 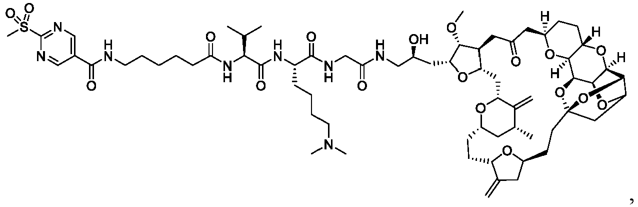 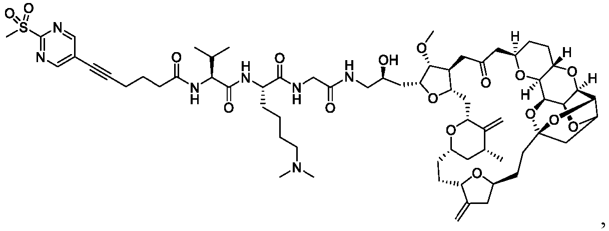 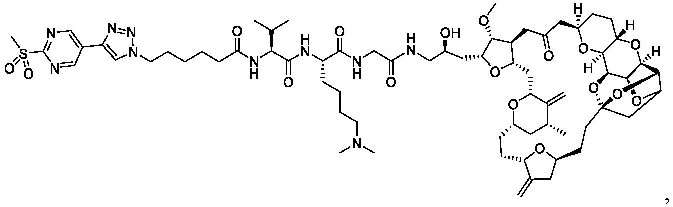 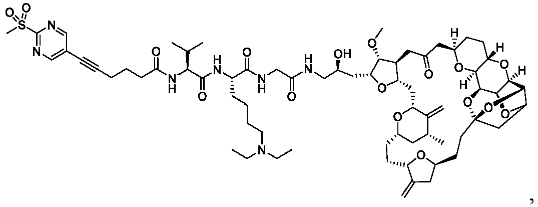 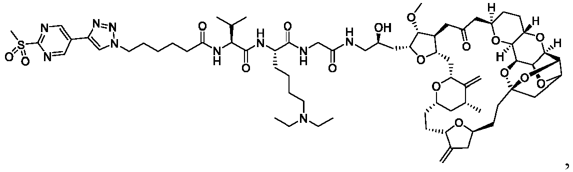 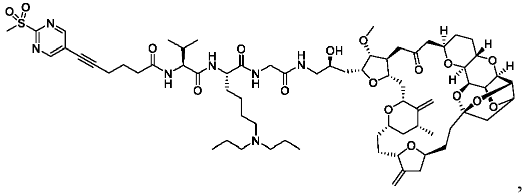 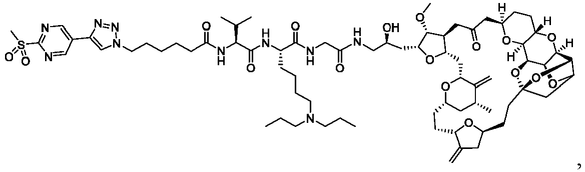 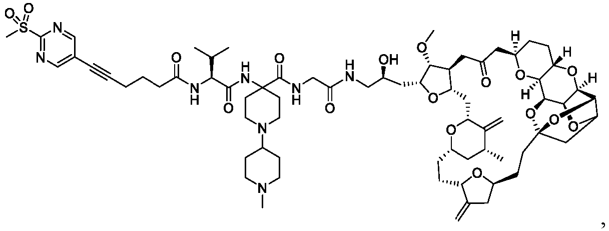 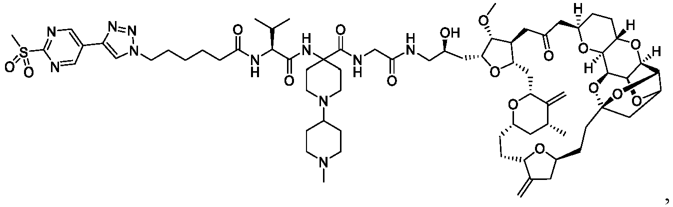 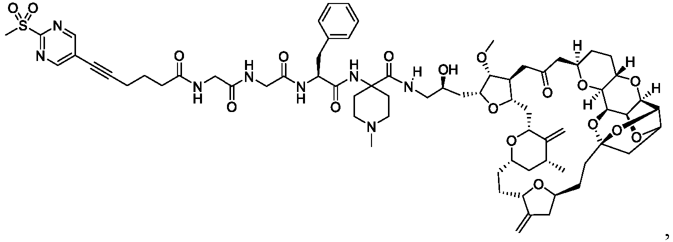 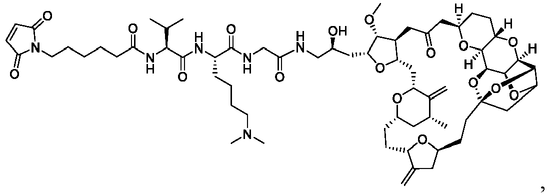 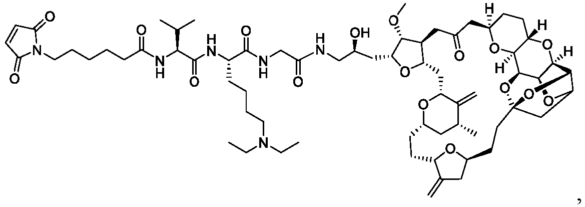 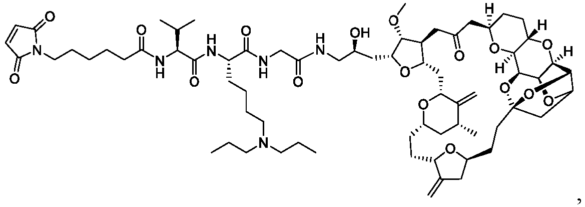 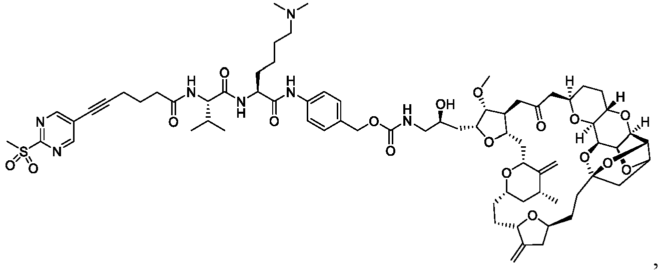 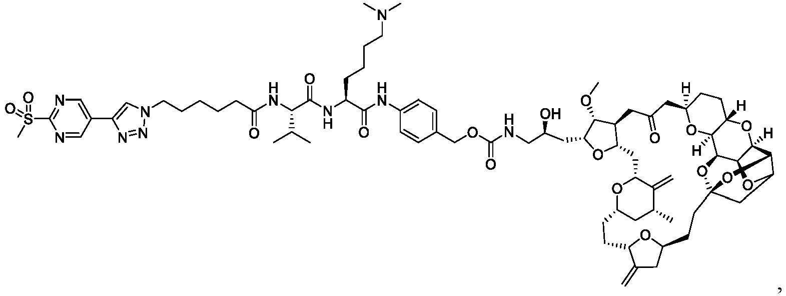 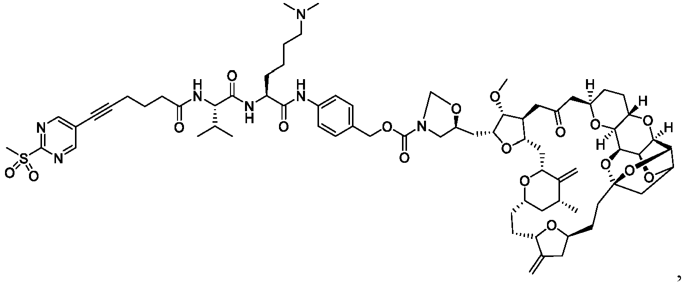 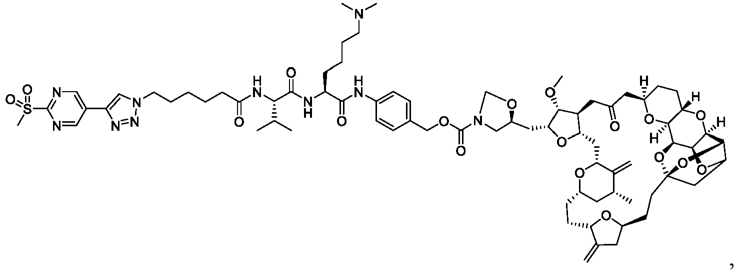 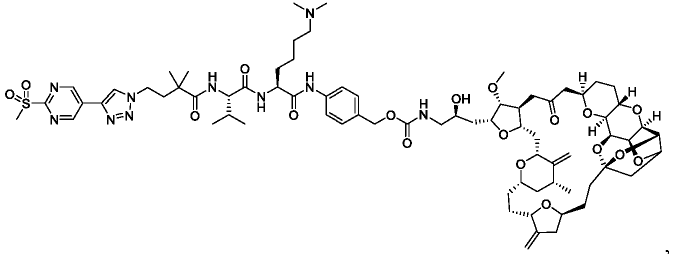 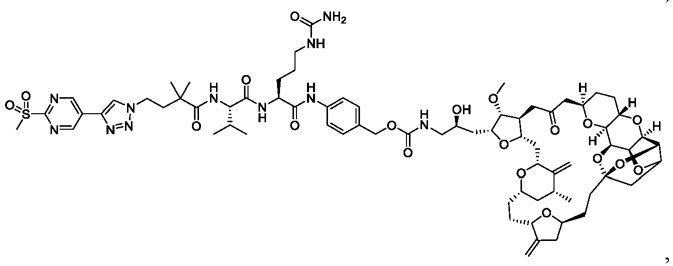 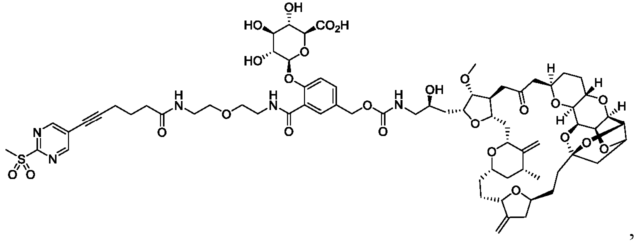 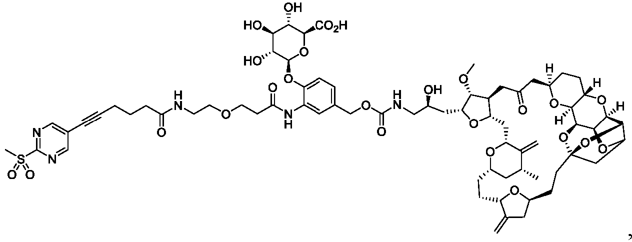 and

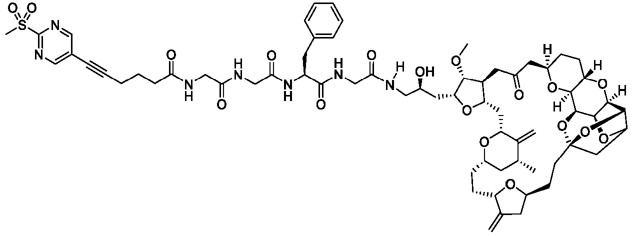

12. A linker of formula III, 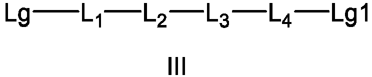 Lg, L₁, L₂, L₃, and L₄ are as defined in any one of claims 1 to 11; Lg1 is a leaving group when reacting with eribulin or a derivative thereof; preferably, Lg1 is -OH , 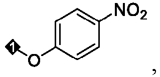 or halogen.

13. A method for preparing a conjugate of formula I, comprising: performing a coupling reaction between a sulfhydryl group on Tb and a drug-linker conjugate of general formula II 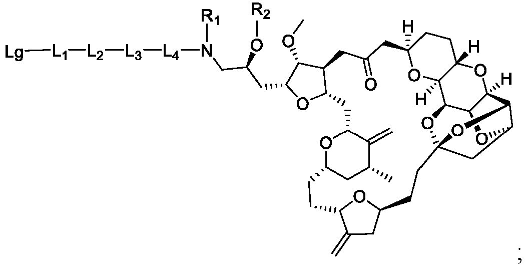 wherein Tb, q, Lg, L₁, L₂, L₃, L₄, R₁, and R₂ are as defined in any one of claims 1 to 11.

14. The method according to claim 13, wherein the method comprises the step of performing a coupling reaction between a sulfhydryl group on Tb and the drug-linker conjugate of general formula II 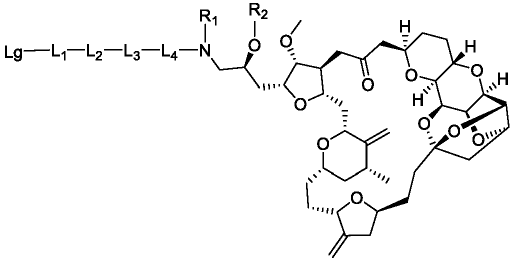 in a suitable solvent and under suitable conditions to form a C-S bond; wherein Tb, q, Lg, L₁, L₂, L₃, L₄, R₁, and R₂ are as defined in any one of claims 1 to 11;
the molar ratio of the sulfhydryl group on Tb to the drug-linker conjugate is 1 :(1-20), such as 1:(2-20), 1:(4-20), 1:(6-20), 1:(8-20), 1:(10-20), 1:(12-20), 1:(14-20), 1:(16-20), or 1:(18-20);
the coupling reaction is preferably carried out in water and/or an organic solvent; the organic solvent is preferably one or more of *N,N*-dimethylformamide, dimethyl sulfoxide, *N-*methylpyrrolidone, a nitrile solvent (e.g., acetonitrile), and an alcohol solvent (*e.g*., methanol, ethanol);
the post-treatment operation of the method is a conventional post-treatment method for coupling reactions in the art, preferably purifying the coupling product by chromatography; the chromatography is typically one or more of ion exchange chromatography, hydrophobic chromatography, reversed-phase chromatography, or affinity chromatography.

15. A conjugate population of antibody-eribulin or a derivative thereof, comprising the conjugate of antibody-eribulin or the derivative thereof according to any one of claims 1 to 8, a stereoisomer thereof, a prodrug thereof, a pharmaceutically acceptable salt thereof, or a pharmaceutically acceptable solvate thereof; in the conjugate population of antibody-eribulin or the derivative thereof, the conjugate of antibody-eribulin or the derivative thereof has two or more q values;
optionally, the drug-to-antibody ratio (DAR) in the conjugate population of antibody-eribulin or the derivative thereof is selected from an integer or decimal between 1 and 10;
preferably, the drug-to-antibody ratio (DAR) in the conjugate population of antibody-eribulin or the derivative thereof is selected from an integer or decimal between 1 and 8;
more preferably, the drug-to-antibody ratio (DAR) in the conjugate population of antibody-eribulin or the derivative thereof is selected from an integer or decimal between 2 and 8;
further more preferably, the drug-to-antibody ratio (DAR) in the conjugate population of antibody-eribulin or the derivative thereof is selected from 2, 2.5, 3, 3.5, 4, 4.2, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, and 9.

16. A pharmaceutical composition comprising the conjugate of antibody-eribulin or the derivative thereof according to any one of claims 1 to 8, a stereoisomer thereof, a prodrug thereof, a pharmaceutically acceptable salt thereof, or a pharmaceutically acceptable solvate thereof, or the drug-linker conjugate of formula II according to any one of claims 9 to 11, a stereoisomer thereof, a prodrug thereof, a pharmaceutically acceptable salt thereof, or a pharmaceutically acceptable solvate thereof, or the conjugate population of antibody-eribulin or the derivative thereof according to claim 15, and optionally one or more pharmaceutically acceptable adjuvants.

17. A use of the conjugate of antibody-eribulin or the derivative thereof according to any one of claims 1 to 8, a stereoisomer thereof, a prodrug thereof, a pharmaceutically acceptable salt thereof, or a pharmaceutically acceptable solvate thereof, or the drug-linker conjugate of formula II according to any one of claims 9 to 11, a stereoisomer thereof, a prodrug thereof, a pharmaceutically acceptable salt thereof, or a pharmaceutically acceptable solvate thereof, or the mixture according to claim 15, or the pharmaceutical composition according to claim 16 in the manufacture of a medicament for the treatment and/or prevention of a disease associated with abnormal cell activity;
optionally, the disease associated with abnormal cell activity is a hematological and/or solid tumor, for example selected from esophageal cancer (*e.g*., esophageal adenocarcinoma and esophageal squamous cell carcinoma), brain tumor, lung cancer (*e.g*., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, urothelial cancer, solid tumor, non-Hodgkin lymphoma, central nervous system tumor (*e.g.*, neuroglioma, glioblastoma multiforme, glioma, or sarcoma), prostate cancer, or thyroid cancer.
